(19) Europäisches Patentamt
European Patent Office
Office européen des brevets

(11) EP 1 783 215 B1

(12) **EUROPEAN PATENT SPECIFICATION**

(45) Date of publication and mention of the grant of the patent:
**20.07.2011 Bulletin 2011/29**

(51) Int Cl.:
***C12N 9/68*** *(2006.01)* ***A61K 38/48*** *(2006.01)*
***A61K 48/00*** *(2006.01)*

(21) Application number: **07003963.1**

(22) Date of filing: **26.04.1995**

(54) **Angiostatin and method of use for inhibition of angiogenesis**

Angiostatin und Verfahren zu seiner Nutzung zur Vermeidung der Angiogenese

Angiostatine et procédé d'utilisation de ladite substance pour inhiber l'angiogenèse

(84) Designated Contracting States:
**AT BE CH DE DK ES FR GB GR IE IT LI LU MC NL PT SE**

(30) Priority: **26.04.1994 US 248629**
**20.10.1994 US 326785**

(43) Date of publication of application:
**09.05.2007 Bulletin 2007/19**

(62) Document number(s) of the earlier application(s) in accordance with Art. 76 EPC:
**95918854.1 / 0 758 390**

(73) Proprietor: **The Children's Medical Center Corporation**
**Boston, Massachusetts 02115 (US)**

(72) Inventors:
- **O'Reilly, Michael S.**
  **Winchester, MA 01890 (US)**
- **Folkman, Judah M.**
  **Brookline, MA 02115 (US)**
- **Sim, Kim Lee**
  **Gaitherburg, MD 20878 (US)**
- **Cao, Yihai**
  **116 32 Stockholm (SE)**

(74) Representative: **Perin, Georges**
**Cabinet Plasseraud**
**52 rue de la Victoire**
**75440 Paris Cedex 09 (FR)**

(56) References cited:
- **O'REILLY M ET AL: "ANGIOSTATIN A NOVEL ANGIOGENESIS INHIBITOR THAT MEDIATES THE SUPPRESSION OF METASTASES BY A LEWIS LUNG CARCINOMA" CELL, vol. 79, 21 October 1994 (1994-10-21), pages 315-328, XP002015254 ISSN: 0092-8674**
- **CAO ET AL: "KRINGLE DOMAINS OF HUMAN ANGIOSTATIN. CHARACTERIZATION OF THE ANTI-PROLIFERATIVE ACTIVITY ON ENDOTHELIAL CELLS" JOURNAL OF BIOLOGICAL CHEMISTRY, vol. 271, no. 46, 15 November 1996 (1996-11-15), pages 29461-29467, XP002086445 ISSN: 0021-9258**
- **O'REILLY M S ET AL: "ANGIOSTATIN: A CIRCULATING ENDOTHELIAL CELL INHIBITOR THAT SUPPRESSES ANGIOGENESIS AND TUMOR GROWTH" COLD SPRING HARBOR SYMPOSIA ON QUANTITATIVE BIOLOGY, vol. 14, 1994, pages 471-482, XP000929844**
- **M. O'REILLY ET AL.: "The suppression of tumor metastases by a primary tumor" SURGICAL FORUM, vol. 44, 1993, pages 474-476, XP009081405**

EP 1 783 215 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of the Invention

The present invention relates to endothelial inhibitors, called angiostatin, which reversibly inhibit proliferation of endothelial cells.

### Background of the Invention

As used herein, the term "angiogenesis" means the generation of new blood vessels into a tissue or organ. Under normal physiological conditions, humans or animals undergo angiogenesis only in very specific restricted situations. For example, angiogenesis is normally observed in wound healing, fetal and embryonal development and formation of the corpus luteum, endometrium and placenta. The term "endothelium" means a thin layer of flat epithelial cells that lines serous cavities, lymph vessels, and blood vessels.

Both controlled and uncontrolled angiogenesis are thought to proceed in a similar manner. Endothelial cells and pericytes, surrounded by a basement membrane, form capillary blood vessels. Angiogenesis begins with the erosion of the basement membrane by enzymes released by endothelial cells and leukocytes. The endothelial cells, which line the lumen of blood vessels, then protrude through the basement membrane. Angiogenic stimulants induce the endothelial cells to migrate through the eroded basement membrane. The migrating cells form a "sprout" off the parent blood vessel, where the endothelial cells undergo mitosis and proliferate. The endothelial sprouts merge with each other to form capillary loops, creating the new blood vessel.

Persistent, unregulated angiogenesis occurs in a multiplicity of disease states, tumor metastasis and abnormal growth by endothelial cells and supports the pathological damage seen in these conditions. The diverse pathological disease states in which unregulated angiogenesis is present have been grouped together as angiogenic dependent or angiogenic associated diseases.

The hypothesis that tumor growth is angiogenesis-dependent was first proposed in 1971. (Folkman J., Tumor angiogenesis: Therapeutic implications., N. Engl. Jour. Med. 285:1182 1186, 1971) In its simplest terms it states: "Once tumor 'take' has occurred, every increase in tumor cell population must be preceded by an increase in new capillaries converging on the tumor." Tumor 'take' is currently understood to indicate a prevascular phase of tumor growth in which a population of tumor cells occupying a few cubic millimeters volume and not exceeding a few million cells, can survive on existing host microvessels. Expansion of tumor volume beyond this phase requires the induction of new capillary blood vessels. For example, pulmonary micrometastases in the early prevascular phase in mice would be undetectable except by high power microscopy on histological sections.

Examples of the indirect evidence which support this concept include:

(1) The growth rate of tumors implanted in subcutaneous transparent chambers in mice is slow and linear before neovascularization, and rapid and nearly exponential after neovascularization. (Algire GH, et al. Vascular reactions of normal and malignant tumors in vivo. I. Vascular reactions of mice to wounds and to normal and neoplastic transplants. J. Natl. Cancer Inst. 6:73-85, 1945)

(2) Tumors grown in isolated perfused organs where blood vessels do not proliferate are limited to 1-2 $mm^3$ but expand rapidly to >1000 times this volume when they are transplanted to mice and become neovascularized. (Folkman J, et al., Tumor behavior in isolated perfused organs: In vitro growth and metastasis of biopsy material in rabbit thyroid and canine intestinal segments. Annals of Surgery 164:491-502, 1966)

(3) Tumor growth in the avascular cornea proceeds slowly and at a linear rate, but switches to exponential growth after neovascularization. (Gimbrone, M.A., Jr. et al., Tumor growth and neovascularization: An experimental model using the rabbit cornea. J. Natl. Cancer Institute 52:41-427, 1974)

(4) Tumors suspended in the aqueous fluid of the anterior chamber of the rabbit eye, remain viable, avascular and limited in size to < 1 $mm^3$. Once they are implanted on the iris vascular bed, they become neovascularized and grow rapidly, reaching 16,000 times their original volume within 2 weeks. (Gimbrone MA Jr., et al., Tumor dormancy in vivo by prevention of neovascularization. J. Exp. Med. 136:261-276)

(5) When tumors are implanted on the chick embryo chorioallantoic membrane, they grow slowly during an avascular phase of >72 hours, but do not exceed a mean diameter of 0.93 + 0.29 mm. Rapid tumor expansion occurs within 24 hours after the onset of neovascularization, and by day 7 these vascularized tumors reach a mean diameter of 8.0 + 2.5 mm. (Knighton D., Avascular and vascular phases of tumor growth in the chick embryo. British J. Cancer, 35:347-356, 1977)

(6) Vascular casts of metastases in the rabbit liver reveal heterogeneity in size of the metastases, but show a relatively uniform cut-off point for the size at which vascularization is present. Tumors are generally avascular up to 1 mm in diameter, but are neovascularized beyond that diameter. (Lien W., et al., The blood supply of experimental liver metastases. II. A microcirculatory study of normal and tumor vessels of the liver with the use of perfused silicone rubber. Surgery 68:334-340, 1970)

(7) In transgenic mice which develop carcinomas in the beta cells of the pancreatic islets, pre-vascular hyperplastic islets are limited in size to < 1 mm. At 6-7 weeks of age, 4-10% of the islets become neovascularized, and from these islets arise large vascularized tumors of more than 1000 times the volume of the pre-vascular islets. (Folkman J, et al., Induction of angiogenesis during the transition from hyperplasia to neoplasia. Nature 339:58-61, 1989)

(8) A specific antibody against VEGF (vascular endothelial growth factor) reduces microvessel density and causes "significant or dramatic" inhibition of growth of three human tumors which rely on VEGF as their sole mediator of angiogenesis (in nude mice). The antibody does not inhibit growth of the tumor cells *in vitro.* (Kim K J, et al., Inhibition of vascular endothelial growth factor-induced angiogenesis suppresses tumor growth in vivo. Nature 362:841-844, 1993)

(9) Anti-bFGF monoclonal antibody causes 70% inhibition of growth of a mouse tumor which is dependent upon secretion of bFGF as its only mediator of angiogenesis. The antibody does not inhibit growth of the tumor cells *in vitro.* (Hori A, et al., Suppression of solid tumor growth by immunoneutralizing monoclonal antibody against human basic fibroblast growth factor. Cancer Research, 51:6180-6184, 1991)

(10) Intraperitoneal injection of bFGF enhances growth of a primary tumor and its metastases by stimulating growth of capillary endothelial cells in the tumor. The tumor cells themselves lack receptors for bFGF, and bFGF is not a mitogen for the tumors cells *in vitro.* (Gross JL, et al. Modulation of solid tumor growth in vivo by bFGF. Proc. Amer. Assoc. Canc. Res. 31:79, 1990)

(11) A specific angiogenesis inhibitor (AGM-1470) inhibits tumor growth and metastases *in vivo,* but is much less active in inhibiting tumor cell proliferation *in vitro.* It inhibits vascular endothelial cell proliferation half-maximally at 4 logs lower concentration than it inhibits tumor cell proliferation. (Ingber D, et al., Angioinhibins: Synthetic analogues of fumagillin which inhibit angiogenesis and suppress tumor growth. Nature, 48:555-557, 1990). There is also indirect clinical evidence that tumor growth is angiogenesis dependent.

(12) Human retinoblastomas that are metastatic to the vitreous develop into avascular spheroids which are restricted to less than 1 $mm^3$ despite the fact that they are viable and incorporate $^3$H-thymidine (when removed from an enucleated eye and analyzed *in vitro*).

(13) Carcinoma of the ovary metastasizes to the peritoneal membrane as tiny avascular white seeds (1-3 $mm^3$). These implants rarely grow larger until one or more of them becomes neovascularized.

(14) Intensity of neovascularization in breast cancer (Weidner N, et al., Tumor angiogenesis correlates with metastasis in invasive breast carcinoma. N. Engl. J. Med. 324:1-8, 1991, and Weidner N, et al., Tumor angiogenesis: A new significant and independent prognostic indicator in early-stage breast carcinoma, J Natl. Cancer Inst. 84: 1875-1887, 1992) and in prostate cancer (Weidner N, Carroll PR, Flax J, Blumenfeld W, Folkman J. Tumor angiogenesis correlates with metastasis in invasive prostate carcinoma. American Journal of Pathology, 143(2):401-409, 1993) correlates highly with risk of future metastasis.

(15) Metastasis from human cutaneous melanoma is rare prior to neovascularization. The onset of neovascularization leads to increased thickness of the lesion and an increasing risk of metastasis. (Srivastava A, et al., The prognostic significance of tumor vascularity in intermediate thickness (0.76-4.0 mm thick) skin melanoma. Amer. J. Pathol. 133: 419-423, 1988)

(16) In bladder cancer, the urinary level of an angiogenic peptide, bFGF, is a more sensitive indicator of status and extent of disease than is cytology. (Nguyen M, et al., Elevated levels of an angiogenic peptide, basic fibroblast growth factor, in urine of bladder cancer patients. J. Natl. Cancer Inst. 85:241-242, 1993)

Thus, it is clear that angiogenesis plays a major role in the metastasis of a cancer. If this angiogenic activity could be repressed or eliminated, then the tumor, although present, would not grow. In the disease state, prevention of angiogenesis could avert the damage caused by the invasion of the new microvascular system. Therapies directed at control of the angiogenic processes could lead to the abrogation or mitigation of these diseases.

What is needed therefore is a composition and method which can inhibit the unwanted growth of blood vessels, especially into tumors. Also needed is a method for detecting, measuring, and localizing the composition. The composition should be able to overcome the activity of endogenous growth factors in premetastatic tumors and prevent the formation of the capillaries in the tumors thereby inhibiting the growth of the tumors. The composition, fragments of the composition, and antibodies specific to the composition, should also be able to modulate the formation of capillaries in other angiogenic processes, such as wound healing and reproduction. The composition and method for inhibiting angiogenesis should preferably be non-toxic and produce few side effects. Also needed is a method for detecting, measuring, and localizing the binding sites for the composition as well as sites of biosynthesis of the composition. The composition and fragments of the composition should be capable of being conjugated to other molecules for both radioactive and nonradioactive labeling purposes

**Summary of the Invention**

The invention is defined by the claims. Compositions and methods are provided that are effective for modulating angiogenesis, and inhibiting unwanted angiogenesis, especially angiogenesis related to tumor growth. It is disclosed a protein, which has been named "angiostatin", defined by its ability to overcome the angiogenic activity of endogenous growth factors such as bFGF, *in vitro,* and by it amino acid sequence homology and structural similarity to an internal portion of plasminogen beginning at approximately plasminogen amino acid 98. Angiostatin comprises a protein having a molecular weight of between approximately 38 kilodaltons and 45 kilodaltons as determined by reducing polyacrylamide gel electrophoresis and having an amino acid sequence substantially similar to that of a fragment of murine plasminogen beginning at amino acid number 98 of an intact murine plasminogen molecule (SEQ ID NO:2).

The amino acid sequence of angiostatin varies slightly between species. For example, in human angiostatin the amino acid sequence is substantially similar to the sequence of the above described murine plasminogen fragment, although an active human angiostatin sequence may start at either amino acid number 97 or 99 of an intact human plasminogen amino acid sequence. Further, fragments of human plasminogen has similar anti-angiogenic activity as shown in a mouse tumor model. It is to be understood that the number of amino acids in the active angiostatin molecule may vary and all amino acid sequences that have endothelial inhibiting activity are contemplated as being included in the present invention.

It is disclosed methods and compositions for treating diseases and processes mediated by undesired and uncontrolled angiogenesis by administering to a human or animal a composition comprising a substantially purified angiostatin or angiostatin derivative in a dosage sufficient to inhibit angiogenesis. The present invention is particularly useful for treating, or for repressing the growth of, tumors. Administration of angiostatin to a human or animal with prevascularized metastasized tumors will prevent the growth or expansion of those tumors.

The present invention also encompasses DNA sequences encoding angiostatin, expression vectors containing DNA sequences encoding angiostatin, and cells containing one or more expression vectors containing DNA sequences encoding angiostatin. The present invention further encompasses gene therapy methods whereby DNA sequences encoding angiostatin are introduced into a patient to modify *in vivo* angiostatin levels.

It is another object of the present invention to provide a composition for treating diseases and processes that are mediated by angiogenesis.

It is yet another object of the present invention to provide a composition for treating diseases and processes that are mediated by angiogenesis including, but not limited to, hemangioma, solid tumors, blood borne tumors, leukemia, metastasis, telangiectasia, psoriasis, scleroderma, pyogenic granuloma, myocardial angiogenesis, Crohn's disease, plaque neovascularization, coronary collaterals, cerebral collaterals, arteriovenous malformations, ischemic limb angiogenesis, corneal diseases, rubeosis, neovascular glaucoma, diabetic retinopathy, retrolental fibroplasia, arthritis, diabetic neovascularization, macular degeneration, wound healing, peptic ulcer, *Helicobacter* related diseases, fractures, keloids, vasculogenesis, hematopoiesis, ovulation, menstruation, placentation, and cat scratch fever.

It is another object of the present invention to provide a composition for treating or repressing the growth of a cancer.

It is yet another object of the present invention to provide a therapy for cancer that has minimal side effects.

Another object of the present invention is to provide a method for targeted delivery of angiostatin-related compositions to specific locations.

Yet another object of the invention is to provide compositions useful for gene therapy for the modulation of angiogenic processes.

These and other objects, features and advantages of the present invention will become apparent after a review of the following detailed description of the disclosed embodiments and the appended claims.

**Brief Description of the Figures**

Figure 1 shows SEQ ID NO: 1, the amino acid sequence of the whole murine plasminogen.

Figure 2 shows the beginning sequence of the angiostatin for murine (SEQ ID NO:2) and compares the murine sequence with corresponding human (SEQ ID NO:3), Rhesus monkey (SEQ ID NO:4), porcine (SEQ ID NO:5) and bovine (SEQ ID NO:6) plasminogen peptide fragments. The mouse sequence is listed first, followed by human, Rhesus, porcine and bovine.

Figure 3 shows BrdU labeling index of tumor cells in the lung in the presence or absence of a primary tumor.

Figure 4 shows Matrigel analysis of the influence of a Lewis lung primary tumor on bFGF driven angiogenesis *in vivo.*

Figure 5 shows dose response curve for serum derived from mice bearing Lewis lung carcinoma (LLC-Low) versus serum from normal mice. Bovine capillary endothelial cells were assayed in a bFGF-driven 72-hour proliferation assay.

Figure 6 shows that both low and high metastatic tumors contain endothelial mitogenic activity in their ascites, but only the low metastatic tumor line has endothelial inhibitory activity in the serum.

Figure 7 shows a C4 Reverse Phase Chromatographic profile of partially purified serum or urine from tumor-bearing animals.

Figure 8 shows surface lung metastases after the 13 day treatment of mice with intact plasminogen molecule, active fraction from a lysine binding site I preparation of human plasminogen, concentrated urine from tumor bearing mice and concentrated urine from normal mice.

Figure 9 shows lung weight after the 13 day treatment of mice with intact plasminogen molecule of human plasminogen, active fraction from lysine binding site I preparation, concentrated urine from tumor bearing mice and concentrated urine from normal mice.

Figure 10 is a schematic representation of the pTrcHis vector.

Figure 11 depicts an immunoblot of E.coli expressed human angiostatin from a 10L scaled-up fermentation, probed with monoclonal antibody against human plasminogen kringle region 1-3. Arrow shows recombinant human angiostatin. A) shows recombinant angiostatin eluted with 0.2 M amino caproic acid; B) shows the last wash with 1 X PBS of the lysine column; and C) shows clarified lysate from cracked cells.

Figure 12. Is a graph depicting percent inhibition of growing bovine capillary endothelial cells as a function of dilution of stock; A1, A2, B1, B2, and E are recombinant clones that express human angiostatin anit-angiogenesis activity; C1, C2, D1 and D2 controls are negative controls clones containing vector only without the human DNA sequence coding for angiostatin.

Figure 13 shows the inhibitory effect on proliferation of recombinant human angiostatin on bovine capillary endothelial cells *in vitro.*

Figure 14 shows the growth proliferation index and apoptotic index after removal of the primary tumor and treatment with saline or a fumagillin analogue with anti-angiogenic activity

Figure 15 shows the inhibition of growth of a T241 primary tumor in mice by treatment with human angiostatin *in vivo* with a single injection of 40 mg/kg/day.

Figure 16 shows the inhibition of growth of a LLC-LM primary tumor in mice by treatment with human angiostatin *in vivo* at two doses of 40 mg/kg per dose (80 mg/kg/day).

Figure 17 shows the effect of the removal of a Lewis lung carcinoma primary tumor on the growth of its lung metastases.

Figure 18 shows the growth proliferation and apoptotic index after tumor resection

Figure 19 shows the effect of administration of angiostatin protein to mice having implated T241 fibrosarcoma cells on total tumor volume as a function of time.

Figure 20 shows the effect of administration of angiostatin protein to mice having implated Lewis lung carcinoma (LM) cells on total tumor volume as a function of time.

Figure 21 shows the effect of administration of angiostatin protein to mice having implated reticulum cell sarcoma cells on total tumor volume as a function of time.

Figure 22 shows the effect of administration of angiostatin protein to immunodeficient SCID mice having implated human prostate carcinoma PC-3 cells on total tumor volume as a function of time over a 24 day period.

Figure 23 shows the effect of administration of angiostatin protein to immunodeficient SCID mice having implated human breast carcinoma MDA-MB cells on total tumor volume as a function of time over a 24 day period.

Figure 24 is a schematic representation of cloning of the mouse DNA sequence coding for mouse angiostatin protein derived from mouse plasminogen cDNA. The mouse angiostatin encompasses mouse plasminogen kringle regions 1-4. PCR means polymerase chain reaction; P1 is the 5'-end oligonucleotide primre for PCR; P2 is the 3'-end oligonucleotide primre for PCR; SS designates the signal sequence; ATG is the translation initiation codon; TAA is the translation stop codon; HA represents the hemagglutinin epitope tag (YPYDVPDYASL); K1, K2, K3 and K4 represent mouse plasminogen kringle regions 1, 2, 3 and 4 respectively. CMV is the cytomegalovirus promoter; T7 is the bacteria phage promoter; PA represents pre-activation peptides; and SP6 is the Sp 6 promoter.

Figure 25 depicts the number of cells as a function of days for non-transfected cells (mock); cells transfected with the vector alone, without the DNA sequence coding for angiostatin (Vector 5), and two angiostatin expressing clones (AST 31 and AST 37). Panel (a) represents the results of transfection of T241 cells. Panel (b) represents the results of LL2 cells.

Figure 26 shows the results of culture medium derived from E. coli cells containing the angiostatin clone on cell number. Non-transfected cells (mock); cells transfected with the vector alone, without the DNA sequence coding for angiostatin (Vector 5), and three angiostatin expressing clones (AST 25, AST 31 and AST 37). Panel (a) represents the results of incubation of culture medium from control (mock) and all angiostatin clones (expressing and non-expressing) on cell number. Panel (b) represents the results of incubation of culture medium from control (mock), vector alone (vector 6) and angiostatin clones expressing mouse angiostatin on cell number. Panel (c) represents the results of incubation of purified culture medium from control (mock) and angiostatin clones expressing mouse angiostatin on cell number, wherein the culture medium was purified over a lysine-sepharose column to yield lysine binding components.

Figure 27 shows the effect on total tumor volume as a function of time of implanting T241 fibrosarcoma cells in mice, where the fibrosarcoma cells have been transfected with a vector containing a DNA sequence coding for angiostatin protein, and where the vector is capable of expressing angiostatin protein. "Non-transfected" represents unaltered T241 fibrosarcoma cells implanted in mice. "Vector 6" represents T241 fibrosarcoma cells transfected with the vector only, which does not contain the DNA sequence coding for angiostatin protein, implanted in mice. "Clone 25, Clone 31 and Clone 37" represent three angiostatin-producing clones of T241 fibrosarcoma cells transfected with a vector containg the DNA sequence coding for angiostation protein implanted in mice.

**Detailed Description**

The present invention includes compositions for the treatment of diseases and processes that are mediated by or associated with angiogenesis. Angiostatin can be isolated from body fluids including, but not limited to, serum, urine and ascites, or synthesized by chemical or biological methods (e.g. cell culture, recombinant gene expression, peptide synthesis, and *in vitro* enzymatic catalysis of plasminogen or plasmin to yield active angiostatin). Recombinant techniques include gene amplification from DNA sources using the polymerase chain reaction (PCR), and gene amplification from RNA sources using reverse transcriptase/PCR. Angiostatin inhibits the growth of blood vessels into tissues such as unvascularized or vascularized tumors.

The present invention also encompasses a composition comprising, a vector containing a DNA sequence encoding angiostatin, wherein the vector is capable of expressing angiostatin when present in a cell, a composition comprising a cell containing a vector, wherein the vector contains a DNA sequence encoding angiostatin or fragments or analogs thereof, and wherein the vector is capable of expressing angiostatin when present in the cell, and a method comprising, implanting into a human or non-human animal a cell containing a vector, wherein the vector contains a DNA sequence encoding angiostatin, and wherein the vector is capable of expressing angiostatin when present in the cell.

More particularly, it is described a protein designated angiostatin that has a molecular weight of approximately 38 to 45 kilodaltons (kD) that is capable of overcoming the angiogenic activity of endogenous growth factors such as bFGF, *in vitro.* Angiostatin is a protein having a molecular weight of between approximately 38 kilodaltons and 45 kilodaltons as determined by reducing polyacrylamide gel electrophoresis and having an amino acid sequence substantially similar to that of a murine plasminogen fragment beginning at amino acid number 98 of an intact murine plasminogen molecule. The term "substantially similar," when used in reference to angiostatin amino acid sequences, means an amino acid sequence having anti-angiogenic activity and having a molecular weight of approximately 38 kD to 45 kD, which also has a high degree of sequence homology to the peptide fragment of mouse plasminogen beginning approximately at amino acid number 98 in mouse plasminogen and weighing 38 kD to 45 kD. A high degree of homology means at least approximately 60% amino acid homology, desirably at least approximately 70% amino acid homology, and more desirably at least approximately 80% amino acid homology. The term "endothelial inhibiting activity" as used herein means the capability of a molecule to inhibit angiogenesis in general and, for example, to inhibit the growth of bovine capillary endothelial cells in culture in the presence of fibroblast growth factor.

The amino acid sequence of the complete murine plasminogen molecule is shown in Figure 1 and in SEQ ID NO:1, The sequence for angiostatin begins approximately at amino acid 98. Active human angiostatin may start at either amino acid 97 or 99 of the intact human plasminogen molecule. The amino acid sequence of the first 339 amino acids of angiostatin from mouse is shown in Figure 2, (SEQ ID NO:2), and is compared with the sequences of corresponding plasminogen peptide fragments from human (SEQ ID NO:3, Rhesus monkey (SEQ ID NO:4), porcine (SEQ ID NO:5) and bovine (SEQ ID NO:6) plasminogen. Given that these sequences are identical in well over 50% of their amino acids, it is to be understood that the amino acid sequence of the angiostatin is substantially similar among species. The total number of amino acids in angiostatin is not known precisely but is defined by the molecular weight of the active molecule. The amino acid sequence of the angiostatin of the present invention may vary depending upon from which species the plasminogen molecule is derived. Thus, although the angiostatin of the present invention that is derived from human plasminogen has a slightly different sequence than angiostatin derived from mouse, it has anti-angiogenic activity as shown in a mouse tumor model.

Angiostatin has been shown to be capable of inhibiting the growth of endothelial cells *in vitro.* Angiostatin does not inhibit the growth of cell lines derived from other cell types. Specifically, angiostatin has no effect on Lewis lung carcinoma cell lines, mink lung epithelium, 3T3 fibroblasts, bovine aortic smooth muscle cells, bovine retinal pigment epithelium, MDCk cells (canine renal epithelium), WI38 cells (human fetal lung fibroblasts) EFN cells (murine fetal fibroblasts) and LM cells (murine connective tissue). Endogenous angiostatin in a tumor bering mouse is effective at inhibiting metastases at a systemic concentration of approximately 10 mg angiostatin/kg body weight.

Angiostatin has a specific three dimensional conformation that is defined by the kringle region of the plasminogen molecule. (Robbins, K.C., "The plasminogen-plasmin enzyme system" Hemostasis and Thrombosis. Basic Principles and Practice, 2nd Edition, ed. by Colman, R.W. et al. J.B. Lippincott Company, pp. 340-357, 1987) There are five such kringle regions, which are conformationally related motifs and have substantial sequence homology, in the $NH_2$ terminal portion of the plasminogen molecule. The three dimensional conformation of angiostatin is believed to encompass plasminogen kringle regions 1 through 3 and a part of kringle region 4. Each kringle region of the plasminogen molecule contains approximately 80 amino acids and contains 3 disulfide bonds. This cysteine motif is known to exist in other biologically active proteins. These proteins include, but are not limited to, prothrombin, hepatocyte growth factor, scatter factor and macrophage stimulating protein. (Yoshimura, T, et al., "Cloning, sequencing, and expression of human macrophage stimulating protein (MSP, MST1) confirms MSP as a member of the family of kringle proteins and locates the MSP gene on Chromosome 3" J. Biol. Chem., Vol. 268, No. 21, pp. 15461-15468, 1993). It is contemplated that any isolated protein or peptide having a three dimensional kringle-like conformation or cysteine motif that has anti-angiogenic activity *in vivo,* is part of the present invention.

The present invention also encompasses gene therapy whereby the gene encoding angiostatin is regulated in a patient. Various methods of transferring or delivering DNA to cells for expression of the gene product protein, otherwise referred to as gene therapy, are disclosed in Gene Transfer into Mammalian Somatic Cells *in vivo,* N. Yang, Crit. Rev. Biotechn. 12(4): 335-356 (1992). Gene therapy encompasses incorporation of DNA sequences into somatic cells or germ line cells for use in either *ex vivo* or *in vivo* therapy. Gene therapy functions to replace genes, augment normal or abnormal gene function, and to combat infectious diseases and other pathologies.

Strategies for treating these medical problems with gene therapy include therapeutic strategies such as identifying the defective gene and then adding a functional gene to either replace the function of the defective gene or to augment a slightly functional gene; or prophylactic strategies, such as adding a gene for the product protein that will treat the condition or that will make the tissue or organ more susceptible to a treatment regimen. As an example of a prophylactic strategy, a gene such as angiostatin may be placed in a patient and thus prevent occurrence of angiogenesis; or a gene that makes tumor cells more susceptible to radiation could be inserted and then radiation of the tumor would cause increased killing of the tumor cells.

Many protocols for transfer of angiostatin DNA or angiostatin regulatory sequences are envisioned in this invention. Transfection of promoter sequences, other than one normally found specifically associated with angiostatin, or other sequences which would increase production of angiostatin protein are also envisioned as methods of gene therapy. An example of this technology is found in Transkaryotic Therapies, Inc., of Cambridge, Massachusetts, using homologous recombination to insert a "genetic switch" that turns on an erythropoietin gene in cells. See Genetic Engineering News, April 15, 1994. Such "genetic switches" could be used to activate angiostatin (or the angiostatin receptor) in cells not normally expressing angiostatin (or the angiostatin receptor).

Gene transfer methods for gene therapy fall into three broad categories-physical (e.g., electroporation, direct gene transfer and particle bombardment), chemical (lipid-based carriers, or other non-viral vectors) and biological (virus-derived vector and receptor uptake). For example, non-viral vectors may be used which include liposomes coated with DNA. Such liposome/DNA complexes may be directly injected intravenously into the patient. It is believed that the liposome/DNA complexes are concentrated in the liver where they deliver the DNA to macrophages and Kupffer cells. These cells are long lived and thus provide long term expression of the delivered DNA. Additionally, vectors or the "naked" DNA of the gene may be directly injected into the desired organ, tissue or tumor for targeted delivery of the therapeutic DNA.

Gene therapy methodologies can also be described by delivery site. Fundamental ways to deliver genes include *ex vivo* gene transfer, *in vivo* gene transfer, and *in vitro* gene transfer. In *ex vivo* gene transfer, cells are taken from the patient and grown in cell culture. The DNA is transfected into the cells, the transfected cells are expanded in number and then reimplanted in the patient. In *in vitro* gene transfer, the transformed cells are cells growing in culture, such as tissue culture cells, and not particular cells from a particular patient. These "laboratory cells" are transfected, the transfected cells are selected and expanded for either implantation into a patient or for other uses.

*In vivo* gene transfer involves introducing the DNA into the cells of the patient when the cells are within the patient. Methods include using virally mediated gene transfer using a noninfectious virus to deliver the gene in the patient or injecting naked DNA into a site in the patient and the DNA is taken up by a percentage of cells in which the gene product protein is expressed. Additionally, the other methods described herein, such as use of a "gene gun," may be used for *in vitro* insertion of angiostatin DNA or angiostatin regulatory sequences.

Chemical methods of gene therapy may involve a lipid based compound, not necessarily a liposome, to ferry the DNA across the cell membrane. Lipofectins or cytofectins, lipid-based positive ions that bind to negatively charged DNA, make a complex that can cross the cell membrane and provide the DNA into the interior of the cell. Another chemical method uses receptor-based endocytosis, which involves binding a specific ligand to a cell surface receptor and enveloping and transporting it across the cell membrane. The ligand binds to the DNA and the whole complex is transported into the cell. The ligand gene complex is injected into the blood stream and then target cells that have the receptor will specifically bind the ligand and transport the ligand-DNA complex into the cell.

Many gene therapy methodologies employ viral vectors to insert genes into cells. For example, altered retrovirus vectors have been used in *ex vivo* methods to introduce genes into peripheral and tumor-infiltrating lymphocytes, hepatocytes, epidermal cells, myocytes, or other somatic cells. These altered cells are then introduced into the patient to provide the gene product from the inserted DNA.

Viral vectors have also been used to insert genes into cells using *in vivo* protocols. To direct tissue-specific expression of foreign genes, cis-acting regulatory elements or promoters that are known to be tissue specific can be used. Alternatively, this can be achieved using *in situ* delivery of DNA or viral vectors to specific anatomical sites *in vivo.* For example, gene transfer to blood vessels *in vivo* was achieved by implanting *in vitro* transduced endothelial cells in chosen sites on arterial walls. The virus infected surrounding cells which also expressed the gene product. A viral vector can be delivered directly to the *in vivo* site, by a catheter for example, thus allowing only certain areas to be infected by the virus, and providing long-term, site specific gene expression. *In vivo* gene transfer using retrovirus vectors has also been demonstrated in mammary tissue and hepatic tissue by injection of the altered virus into blood vessels leading to the organs.

Viral vectors that have been used for gene therapy protocols include but are not limited to, retroviruses, other RNA viruses such as poliovirus or Sindbis virus , adenovirus, adeno-associated virus, herpes viruses, SV 40, vaccinia and other DNA viruses. Replication-defective murine retroviral vectors are the most widely utilized gene transfer vectors. Murine leukemia retroviruses are composed of a single strand RNA complexed with a nuclear core protein and polymerase (pol) enzymes, encased by a protein core (gag) and surrounded by a glycoprotein envelope (env) that determines host range. The genomic structure of retroviruses include the gag, pol, and env genes enclosed at by the 5' and 3' long terminal repeats (LTR). Retroviral vector systems exploit the fact that a minimal vector containing the 5' and 3' LTRs and the packaging signal are sufficient to allow vector packaging, infection and integration into target cells providing that the viral structural proteins are supplied *in trans* in the packaging cell line. Fundamental advantages of retroviral vectors for gene transfer include efficient infection and gene expression in most cell types, precise single copy vector integration into target cell chromosomal DNA, and ease of manipulation of the retroviral genome.

The adenovirus is composed of linear, double stranded DNA complexed with core proteins and surrounded with capsid proteins. Advances in molecular virology have led to the ability to exploit the biology of these organisms to create vectors capable of transducing novel genetic sequences into target cells *in vivo.* Adenoviral-based vectors will express gene product peptides at high levels. Adenoviral vectors have high efficiencies of infectivity, even with low titers of virus. Additionally, the virus is fully infective as a cell free virion so injection of producer cell lines are not necessary. Another potential advantage to adenoviral vectors is the ability to achieve long term expression of heterologous genes *in vivo.*

Mechanical methods of DNA delivery include fusogenic lipid vesicles such as liposomes or other vesicles for membrane fusion, lipid particles of DNA incorporating cationic lipid such as lipofectin, polylysine-mediated transfer of DNA, direct injection of DNA, such as microinjection of DNA into germ or somatic cells, pneumatically delivered DNA-coated particles, such as the gold particles used in a "gene gun," and inorganic chemical approaches such as calcium phosphate transfection. Another method, ligand-mediated gene therapy, involves complexing the DNA with specific ligands to form ligand-DNA conjugates, to direct the DNA to a specific cell or tissue.

It has been found that injecting plasmid DNA into muscle cells yields high percentage of the cells which are transfected and have sustained expression of marker genes. The DNA of the plasmid may or may not integrate into the genome of the cells. Non-integration of the transfected DNA would allow the transfection and expression of gene product proteins in terminally differentiated, non-proliferative tissues for a prolonged period of time without fear of mutational insertions, deletions, or alterations in the cellular or mitochondrial genome. Long-term, but not necessarily permanent, transfer of therapeutic genes into specific cells may provide treatments for genetic diseases or for prophylactic use. The DNA could be reinjected periodically to maintain the gene product level without mutations occurring in the genomes of the recipient cells. Non-integration of exogenous DNAs may allow for the presence of several different exogenous DNA constructs within one cell with all of the constructs expressing various gene products.

Particle-mediated gene transfer methods were first used in transforming plant tissue. With a particle bombardment device, or "gene gun," a motive force is generated to accelerate DNA-coated high density particles (such as gold or tungsten) to a high velocity that allows penetration of the target organs, tissues or cells. Particle bombardment can be used in *in vitro* systems, or with *ex vivo* or *in vivo* techniques to introduce DNA into cells, tissues or organs.

Electroporation for gene transfer uses an electrical current to make cells or tissues susceptible to electroporation-mediated gene transfer. A brief electric impulse with a given field strength is used to increase the permeability of a membrane in such a way that DNA molecules can penetrate into the cells. This technique can be used in *in vitro* systems, or with *ex vivo* or *in vivo* techniques to introduce DNA into cells, tissues or organs.

Carrier mediated gene transfer *in vivo* can be used to transfect foreign DNA into cells. The carrier-DNA complex can be conveniently introduced into body fluids or the bloodstream and then site specifically directed to the target organ or tissue in the body. Both liposomes and polycations, such as polylysine, lipofectins or cytofectins, can be used. Liposomes can be developed which are cell specific or organ specific and thus the foreign DNA carried by the liposome will be taken up by target cells. Injection of immunoliposomes that are targeted to a specific receptor on certain cells can be used as a convenient method of inserting the DNA into the cells bearing the receptor. Another carrier system that has been used is the asialoglycoportein/polylysine conjugate system for carrying DNA to hepatocytes for *in vivo* gene transfer.

The transfected DNA may also be complexed with other kinds of carriers so that the DNA is carried to the recipient cell and then resides in the cytoplasm or in the nucleoplasm. DNA can be coupled to carrier nuclear proteins in specifically engineered vesicle complexes and carried directly into the nucleus.

Gene regulation of angiostatin may be accomplished by administering compounds that bind to the angiostatin gene, or control regions associated with the angiostatin gene, or its corresponding RNA transcript to modify the rate of transcription or translation. Additionally, cells transfected with a DNA sequence encoding angiostatin may be administered to a patient to provide an *in vivo* source of angiostatin. For example, cells may be transfected with a vector containing a nucleic acid sequence encoding angiostatin. The term "vector" as used herein means a carrier that can contain or associate with specific nucleic acid sequences, which functions to transport the specific nucleic acid sequences into a cell. Examples of vectors include plasmids and infective microorganisms such as viruses, or non-viral vectors such as ligand-DNA conjugates, liposomes, lipid-DNA complexes. It may be desirable that a recombinant DNA molecule comprising an angiostatin DNA sequence is operatively linked to an expression control sequence to form an expression vector capable of expressing angiostatin. The transfected cells may be cells derived from the patient's normal tissue, the patient's diseased tissue, or may be non-patient cells.

For example, tumor cells removed from a patient can be transfected with a vector capable of expressing the angiostatin protein of the present invention, and re-introduced into the patient. The transfected tumor cells produce angiostatin levels in the patient that inhibit the growth of the tumor. Patients may be human or non-human animals. Cells may also be transfected by non-vector, or physical or chemical methods known in the art such as electroporation, ionoporation, or via a "gene gun." Additionally, angiostatin DNA may be directly injected, without the aid of a carrier, into a patient. In particular, angiostatin DNA may be injected into skin, muscle or blood.

The gene therapy protocol for transfecting angiostatin into a patient may either be through integration of the angiostatin DNA into the genome of the cells, into minichromosomes or as a separate replicating or non-replicating DNA construct in the cytoplasm or nucleoplasm of the cell. Angiostatin expression may continue for a long-period of time or may be reinjected periodically to maintain a desired level of the angiostatin protein in the cell, the tissue or organ or a determined blood level.

Angiostatin can be isolated on an HPLC C4 column (see Table 3). The angiostatin protein is eluted at 30 to 35% in an acetonitrile gradient. On a sodium dodecyl sulfate polyacrylamide gel electrophoresis (PAGE) gel under reducing conditions, the protein band with activity eluted as a single peak at approximately 38 kilodaltons.

The inventors have shown that a growing primary tumor is associated with the release into the blood stream of specific inhibitor(s) of endothelial cell proliferation, including angiostatin which can suppress angiogenesis within a metastasis and thereby inhibit the growth of the metastasis itself. The source of the angiostatin associated with the primary tumor is not known. The compound may be produced by degradation of plasminogen by a specific protease, or angiostatin could be produced by expression of a specific gene coding for angiostatin.

The angiogenic phenotype of a primary tumor depends on production of angiogenic peptides in excess of endothelial cell inhibitors which are elaborated by normal cells, but are believed to be down-regulated during transformation to neoplasia. While production of angiostatin may be down-regulated in an individual tumor cell relative to production by its parent cell type, the total amount of inhibitor elaborated by the whole tumor may be sufficient to enter the circulation and suppress endothelial growth at remote sites of micrometastases. Angiostatin remains in the circulation for a significantly longer time than the angiogenic peptide(s) released by a primary tumor. Thus, the angiogenic peptides appear to act locally, whereas angiostatin acts globally and circulates in the blood with a relatively long half-life. The half-life of the angiostatin is approximately 12 hours to 5 days.

Although not wanting to be bound by the following hypothesis, it is believed that when a tumor becomes angiogenic it releases one or more angiogenic peptides (e.g., aFGF, bFGF, VEGF, IL-8, GM-CSF, etc.), which act locally, target endothelium in the neighborhood of a primary tumor from an extravascular direction, and do not circulate (or circulate with a short half-life). These angiogenic peptides must be produced in an amount sufficient to overcome the action of endothelial cell inhibitor (inhibitors of angiogenesis) for a primary tumor to continue to expand its population. Once such a primary tumor is growing well, it continues to release endothelial cell inhibitors into the circulation. According to this hypothesis, these inhibitors act remotely at a distance from the primary tumor, target capillary endothelium of a metastasis from an intravascular direction, and continue to circulate. Thus, just at the time when a remote metastasis might begin to initiate angiogenesis, the capillary endothelium in its neighborhood could be inhibited by incoming angiostatin.

Once a primary tumor has reached sufficient size to cause angiostatin to be released continuously into the circulation, it is difficult for a second tumor implant (or a micrometastasis) to initiate or increase its own angiogenesis. If a second tumor implant (e.g., into the subcutaneous space, or into the cornea, or intravenously to the lung) occurs shortly after the primary tumor is implanted, the primary tumor will not be able to suppress the secondary tumor (because angiogenesis in the secondary tumor will already be well underway). If two tumors are implanted simultaneously (e.g., in opposite flanks), the inhibitors may have an equivalent inhibiting effect on each other.

The angiostatin can be:

(i) Administered to tumor-bearing humans or animals as anti-angiogenic therapy;
(ii) Monitored in human or animal serum, urine, or tissues as prognostic markers; and
(iii) Used as the basis to analyze serum and urine of cancer patients for similar angiostatic molecules.

It is contemplated that angiostatin can be isolated from a body fluid such as blood or urine of patients or the angiostatin can be produced by recombinant DNA methods or synthetic peptide chemical methods that are well known to those of ordinary skill in the art. Protein purification methods are well known in the art and a specific example of a method for purifying angiostatin, and assaying for inhibitor activity is provided in the examples below. Isolation of human endogenous angiostatin is accomplished using similar techniques.

One example of a method of producing angiostatin using recombinant DNA techniques entails the steps of (1) identifying and purifying angiostatin as discussed above, and as more fully described below, (2) determining the N-terminal amino acid sequence of the purified inhibitor, (3) synthetically generating 5' and 3' DNA oligonucleotide primers for the angiostatin sequence, (4) amplifying the angiostatin gene sequence using polymerase, (5) inserting the amplified sequence into an appropriate vector such as an expression vector, (6) inserting the gene containing vector into a microorganism or other expression system capable of expressing the inhibitor gene, and (7) isolating the recombinantly produced inhibitor. Appropriate vectors include viral, bacterial and eukaryotic (such as yeast) expression vectors. The above techniques are more fully described in laboratory manuals such as "Molecular Cloning: A Laboratory Manual" Second Edition by Sambrook et al., Cold Spring Harbor Press, 1989. The DNA sequence of human plasminogen has been published (Browne, M.J., et al., "Expression of recombinant human plasminogen and aglycoplasminogen in HeLa cells" Fibrinolysis Vol.5 (4). 257-260, 1991).

The gene for angiostatin may also be isolated from cells or tissue (such as tumor cells) that express high levels of angiostatin by (1) isolating messenger RNA from the tissue, (2) using reverse transcriptase to generate the corresponding DNA sequence and then (3) using the polymerase chain reaction (PCR) with the appropriate primers to amplify the DNA sequence coding for the active angiostatin amino acid sequence.

Yet another method of producing angiostatin, or biologically active fragments thereof, is by peptide synthesis. Once a biologically active fragment of an angiostatin is found using the assay system described more fully below, it can be sequenced, for example by automated peptide sequencing methods. Alternatively, once the gene or DNA sequence which codes for angiostatin is isolated, for example by the methods described above, the DNA sequence can be determined using manual or automated sequencing methods well know in the art. The nucleic acid sequence in turn provides information regarding the amino acid sequence. Thus, if the biologically active fragment is generated by specific methods, such as tryptic digests, or if the fragment is N-terminal sequenced, the remaining amino acid sequence can be determined from the corresponding DNA sequence.

Once the amino acid sequence of the peptide is known, the fragment can be synthesized by techniques well known in the art, as exemplified by "Solid Phase Peptide Synthesis: A Practical Approach" E. Atherton and R.C. Sheppard, IRL Press, Oxford, England. Similarly, multiple fragments can be synthesized which are subsequently linked together to form larger fragments. These synthetic peptide fragments can also be made with amino acid substitutions at specific locations to test for agonistic and antagonistic activity *in vitro* and *in vivo.* Peptide fragments that possess high affinity binding to tissues can be used to isolate the angiostatin receptor on affinity columns. Isolation and purification of the angiostatin receptor is a fundamental step towards elucidating the mechanism of action of angiostatin. Isolation of an angiostatin receptor and identification of angiostatin agonists and antagonists will facilitate development of drugs to modulate the activity of the angiostatin receptor, the final pathway to biological activity. Isolation of the receptor enables the construction of nucleotide probes to monitor the location and synthesis of the receptor, using *in situ* and solution hybridization technology. Further, the gene for the angiostatin receptor can be isolated, incorporated into an expression vector and transfected into cells, such as patient tumor cells to increase the ability of a cell type, tissue or tumor to bind angiostatin and inhibit local angiogenesis.

Angiostatin is effective in treating diseases or processes that are mediated by, or involve, angiogenesis. It is described a method of treating an angiogenesis mediated disease with an effective amount of angiostatin, or a biologically active fragment thereof, or combinations of angiostatin fragmetns that collectively possess anti-angiogenic activity, or angiostatin agonists and antagonists. The angiogenesis mediated diseases include, but are not limited to, solid tumors; blood born tumors such as leukemias; tumor metastasis; benign tumors, for example hemangiomas, acoustic neuromas, neurofibromas, trachomas, and pyogenic granulomas; rheumatoid arthritis; psoriasis; ocular angiogenic diseases, for example, diabetic retinopathy, retinopathy of prematurity, macular degeneration, corneal graft rejection, neovascular glaucoma, retrolental fibroplasia, rubeosis; Osler-Webber Syndrome; myocardial angiogenesis; plaque neovascularization; telangiectasia; hemophiliac joints; angiofibroma; and wound granulation. Angiostatin is useful in the treatment of disease of excessive or abnormal stimulation of endothelial cells. These diseases include, but are not limited to, intestinal adhesions, Crohn's disease, atherosclerosis, scleroderma, and hypertrophic scars, i.e., keloids. Angiostatin can be used as a birth control agent by preventing vascularization required for embryo implantation. Angiostatin is useful in the treatment of diseases that have angiogenesis as a pathologic consequence such as cat scratch disease (*Rochele minalia quintosa*) and ulcers (*Helicobacter pylori*).

The synthetic peptide fragments of angiostatin have a variety of uses. The peptide that binds to the angiostatin receptor with high specificity and avidity is radiolabeled and employed for visualization and quantitation of binding sites using autoradiographic and membrane binding techniques. This application provides important diagnostic and research tools. Knowledge of the binding properties of the angiostatin receptor facilitates investigation of the transduction mechanisms linked to the receptor.

In addition, labeling angiostatin peptides with short lived isotopes enables visualization of receptor binding sites *in vivo* using positron emission tomography or other modem radiographic techniques to locate tumors with angiostatin binding sites.

Systematic substitution of amino acids within these synthesized peptides yields high affinity peptide agonists and antagonists to the angiostatin receptor that enhance or diminish angiostatin binding to its receptor. Such agonists are used to suppress the growth of micrometastases, thereby limiting the spread of cancer. Antagonists to angiostatin are applied in situations of inadequate vascularization, to block the inhibitory effects of angiostatin and promote angiogenesis. For example, this treatment may have therapeutic effects to promote wound healing in diabetics.

Angiostatin peptides are employed to develop affinity columns for isolation of the angiostatin receptor from cultured tumor cells. Isolation and purification of the angiostatin receptor is followed by amino acid sequencing. Using this information the gene or genes coding for the angiostatin receptor can be identified and isolated. Next, cloned nucleic acid sequences are developed for insertion into vectors capable of expressing the receptor. These techniques are well known to those skilled in the art. Transfection of the nucleic acid sequence(s) coding for angiostatin receptor into tumor cells, and expression of the receptor by the transfected tumor cells enhances the responsiveness of these cells to endogenous or exogenous angiostatin and thereby decreasing the rate of metastatic growth.

Cytotoxic agents such as ricin, are linked to angiostatin, and high affinity angiostatin peptide fragments, thereby providing a tool for destruction of cells that bind angiostatin. These cells may be found in many locations, including but not limited to, micrometastases and primary tumors. Peptides linked to cytotoxic agents are infused in a manner designed to maximize delivery to the desired location. For example, ricin-linked high affinity angiostatin fragments are delivered through a cannula into vessels supplying the target site or directly into the target. Such agents are also delivered in a controlled manner through osmotic pumps coupled to infusion cannulae. A combination of angiostatin antagonists may be co-applied with stimulators of angiogenesis to increase vascularization of tissue. This therapeutic regimen provides an effective means of destroying metastatic cancer.

Angiostatin may be used in combination with other compositions and procedures for the treatment of diseases. For example, a tumor may be treated conventionally with surgery, radiation or chemotherapy combined with angiostatin and then angiostatin may be subsequently administered to the patient to extend the dormancy of micrometastases and to stabilize and inhibit the growth of any residual primary tumor. Additionally, angiostatin, angiostatin fragments, angiostatin antisera, angiostatin receptor agonists, angiostatin receptor antagonists, or combinations thereof, are combined with pharmaceutically acceptable excipients, and optionally sustained-release matrix, such as biodegradable polymers, to form therapeutic compositions.

A sustained-release matrix, as used herein, is a matrix made of materials, usually polymers, which are degradable by enzymatic or acid/base hydrolysis or by dissolution. Once inserted into the body, the matrix is acted upon by enzymes and body fluids. The sustained-release matrix desirably is chosen from biocompatible materials such as liposomes, polylactides (polylactic acid), polyglycolide (polymer of glycolic acid), polylactide co-glycolide (co-polymers of lactic acid and glycolic acid) polyanhydrides, poly(ortho)esters, polypeptides, hyaluronic acid, collagen, chondroitin sulfate, carboxylic acids, fatty acids, phospholipids, polysaccharides, nucleic acids, polyamino acids, amino acids such as phenylalanine, tyrosine, isoleucine, polynucleotides, polyvinyl propylene, polyvinylpyrrolidone and silicone. A preferred biodegradable matrix is a matrix of one of either polylactide, polyglycolide, or polylactide co-glycolide (co-polymers of lactic acid and glycolic acid).

The angiogenesis-modulating therapeutic composition of the present invention may be a solid, liquid or aerosol and may be administered by any known route of administration. Examples of solid therapeutic compositions include pills, creams, and implantable dosage units. The pills may be administered orally, the therapeutic creams may be administered topically. The implantable dosage unitst may be administered locally, for example at a tumor site, or which may be implanted for systemic release of the therapeutic angiogenesis-modulating composition, for example subcutaneously. Examples of liquid composition include formulations adapted for injection subcutaneously, intravenously, intraarterially, and formulations for topical and intraocular administration. Examples of aersol formulation include inhaler formulation for administration to the lungs.

**Example 1**

*Choice of an animal-tumor system in which growth of metastasis is inhibited by the primary tumor and is accelerated after removal of the primary tumor.*

By screening a variety of murine tumors capable of inhibiting their own metastases, a Lewis lung carcinoma was selected in which the primary tumor most efficiently inhibited lung metastasis. Syngeneic C57Bl6/J six-week-old male mice were injected (subcutaneous dorsum) with 1 x $10^6$ tumor cells. Visible tumors first appeared after 3-4 days. When tumors were approximately 1500 $mm^3$ in size, mice were randomized into two groups. The primary tumor was completely excised in the first group and left intact in the second group after a sham operation. Although tumors from 500 $mm^3$ to 3000 $mm^3$ inhibited growth of metastases, 1500 $mm^3$ was the largest primary tumor that could be safely resected with high survival and no local recurrence.

After 21 days, all mice were sacrificed and autopsied. In mice with an intact primary tumor, there were four +2 visible metastases, compared to fifty +5 metastases in the mice in which the tumor had been removed ($p<0.0001$). These data were confirmed by lung weight, which correlates closely with tumor burden, as has been previously demonstrated. There was a 400% increase in wet lung weight in the mice that had their tumors removed compared to mice in which the tumor remained intact ($p<0.0001$).

This experimental model gave reproducible data and the experiment described is reproducible. This tumor is labeled "Lewis lung carcinoma - low metastatic" (LLC-Low). The tumor also suppressed metastases in a nearly identical pattern in SCID mice, which are deficient in both B and T lymphocytes.

**Example 2**

*Isolation of a variant of Lewis lung carcinoma tumor that is highly metastatic, whether or not the primary tumor is removed.*

A highly metastatic variant of Lewis lung carcinoma arose spontaneously from the LLC-Low cell line of Example 1 in one group of mice and has been isolated according to the methods described in Example 1 and repeatedly transplanted. This tumor (LLC-High) forms more than 30 visible lung metastases whether or not the primary tumor is present.

**Example 3**

*Size of metastases and proliferation rate of tumor cells within them. Effect of the primary tumor that inhibits metastases (LLC-Low).*

C57Bl6/J mice were used in all experiments. Mice were inoculated subcutaneously with LLC-Low cells, and 14 days later the primary tumor was removed in half of the mice. At 5, 10 and 15 days after the tumor had been removed, mice were sacrificed. Histological sections of lung metastases were obtained. Mice with an intact primary tumor had micrometastases in the lung which were not neovascularized. These metastases were restricted to a diameter of 12-15 cell layers and did not show a significant size increase even 15 days after tumor removal. In contrast, animals from which the primary tumor was removed, revealed large vascularized metastases as early as 5 days after operation. These metastases underwent a further 4-fold increase in volume by the 15th day after the tumor was removed (as reflected by lung weight and histology). Approximately 50% of the animals who had a primary tumor removed died of lung metastases before the end of the experiment. All animals with an intact primary tumor survived to the end of the experiment.

Replication rate of tumor cells within metastases was determined by counting nuclei stained with BrdU which had been previously injected into the mice. The high percentage of tumor cells incorporating BrdU in small, avascular metastases of animals with an intact primary tumor was equivalent to the BrdU incorporation of tumor cells in the large vascularized metastases of mice from which the primary tumor had been removed (Figure 3). This finding suggests that the presence of a primary tumor has no direct effect on the replication rate of tumor cells within a metastasis.

In Figure 3, the left panel shows BrdU labeling index of tumor cells in the lung in the presence or absence of a primary tumor. Before immunohistochemical staining, sections were permeabilized with 0.2 M HCl for 10 minutes and digested with 1 $\mu$g/ml proteinase K (Boehringer Mannheim GmbH, Mannheim, Germany) in 0.2 M Tris-HCl, 2 mM $CaCl_2$ at 37° C for 15 minutes. Labeling index was estimated by counting percentage of positive nuclei at 250 power. The right panel of Figure 3 depicts an analysis of total lung weight of tumors with primary tumors intact or removed 5, 10 and 15 days after operation. Animals were sacrificed 6 hours after intraperitoneal injection of BrdU (0.75 mg/mouse).

**Example 4**

*Inhibition of angiogenesis in lung metastases in the presence of an intact primary tumor.*

To measure the degree of vascularization in lung metastases, tissues were stained with antibodies against von Willebrand factor (an endothelial specific marker, available from Dako Inc., Carpenteria, CA). Metastases from animals with intact tumors formed a thin cuff (8-12 tumor cell layers) around existing pulmonary vessels. Except for the endothelial cells of the vessel lining, no or few cells were positive for von Willebrand factor. In contrast, lung metastases of animals 5 days after removal of the primary tumor were not only larger but were also infiltrated with capillary sprouts containing endothelial cells which stained strongly for von Willebrand factor.

In immunohistochemical analysis of the presence of endothelial cells in lung metastases, a lung metastasis with the primary lung tumor intact 19 days after inoculation, had a cuff of tumor cells around a pre-existing microvessel in the lung. The metastasis was limited to 8 to 12 cell layers. There was no evidence of neovascularization around the microvessel, and it did not contain any new microvessels. This was typical of the maximum size of an avascular pre-angiogenic metastasis.

In an immunohistochemical analysis of tissue collected five days after the primary tumor was resected (19 days after inoculation of the primary tumor), the metastasis surrounded a pre-existing vessel in the lung. In contrast, in the sample where the primary tumor was not resected, the tumor was neovascularized. Thus, an intact primary tumor inhibits formation of new capillary blood vessels in metastases, but proliferation of tumor cells within a metastasis are not affected by the primary tumor.

**Example 5**

*A primary tumor inhibits angiogenesis of a second tumor implanted in the mouse cornea. Growth of this second tumor is inhibited.*

A 0.25 to 0.5 $mm^2$ Lewis lung tumor (LLC-Low) was implanted in the mouse cornea on day 0. (Muthukkaruppan Vr., et al., Angiogenesis in the mouse cornea. Science 205:1416-1418, 1979) A primary tumor was formed by inoculating 1 x $10^6$ LLC-Low cells subcutaneously in the dorsum, either 4 or 7 days before the corneal implant; or on the day of the corneal implant; or 4 or 7 days after the corneal implant. Control mice received the corneal implant but not the subcutaneous tumor. Other control mice received the corneal implant and an inoculation of LLC-High tumor cells in the dorsum 4 days before the corneal implant. The corneas were evaluated daily by slit-lamp stereomicroscopy for the growth of the corneal tumor (measured by an ocular micrometer) and for the growth of new capillary vessels from the edge of the corneal limbus.

In control mice not bearing a primary subcutaneous tumor, a majority of corneas (6/8) developed neovascularization starting at day 6 to 7 days after corneal implantation and continuing to day 10. By day 10, the vascularized corneal tumors had reached approximately a quarter of the volume of the whole eye. In the presence of the primary subcutaneous LLC-Low tumor, the corneal implants did not become vascularized if the primary tumor was in place by at least 4 days or more before the corneal implant (Table 1). In the absence of neovascularization, corneal tumors grew slowly as thin, white, avascular discs within the cornea.

However, if the primary tumor was not implanted until 4 days after the corneal implant, corneas became vascularized and 3/3 corneal tumors grew at similar rates as the non-tumor bearing controls. In the presence of the primary subcutaneous LLC-High tumor, the majority of corneas (2/3) developed neovascularization starting at day 7 after corneal implantation and continuing to day 10. By day 10, the vascularized corneal tumors again had reached approximately a quarter of the volume of the whole eye.

**Table 1.**

| Inhibition of tumor angiogenesis in the cornea by a primary subcutaneous tumor. [All primary tumors are LLC-Low except (*) which is LLC-High]. | | | | | | | |
|---|---|---|---|---|---|---|---|
| Day of eye implant | 0 | 0 | 0 | 0 | 0 | 0 | 0 |
| Day of primary tumor implant | -7 | -4 | -4* | 0 | none | +4 | +7 |

(continued)

| Inhibition of tumor angiogenesis in the cornea by a primary subcutaneous tumor. [All primary tumors are LLC-Low except (*) which is LLC-High]. | | | | | | | |
|---|---|---|---|---|---|---|---|
| Number of mice with new corneal vessels at day 10 | 2/10 | 0/9 | 2/3 | 2/3 | 6/8 | 3/3 | 2/3 |

It would be expected that 0/10 corneas would show neovascularization when the primary LLC-Low subcutaneous tumor was implanted 7 days before the eye tumor implant (i.e.-7). However, 2 of the tumors (2/10) had become necrotic because they were too large (> 3 $cm^3$).

**Example 6**

*Primary intact tumor inhibits angiogenesis induced by a secondary subcutaneous implant of basic fibroblast growth factor (bFGF.).*

Although the experiments described in Examples V and VI show that a primary tumor inhibits angiogenesis in a secondary metastasis, these studies do not reveal whether the primary tumor: (i) inhibits endothelial proliferation (or angiogenesis) directly, or (ii) indirectly by down-regulating the angiogenic activity of the metastatic tumor cells. To distinguish between these two possibilities, a focus of subcutaneous angiogenesis was induced by an implant of matrigel containing basic fibroblast growth factor (bFGF). (Passaniti A, et al., A simple, quantitative method for assessing angiogenesis and anti-angiogenic agents using reconstituted basement membrane, heparin and fibroblast growth factor. Lab. Invest. 67:519, 1992)

Matrigel (an extract of basement membrane proteins), containing either 25 or 50 ng/ml bFGF in the presence of heparin, was injected subcutaneously on the ventral surface of normal and tumor-bearing mice (LLC-Low). Mice were sacrificed 4 days later and hemoglobin concentration in the gel was measured to quantify blood vessel formation. It has previously been shown that the number of new vessels which enter the matrigel is correlated with hemoglobin concentration. (Folkman J., Angiogenesis and its inhibitors in "Important Advances in Oncology 1985", VT DeVita, S. Hellman and S. Rosenberg, editors, J.B. Lippincott, Philadelphia 1985) Some gels were also prepared for histological examination. In normal mice, matrigel pellets which contained 50 ng/ml bFGF were completely red. They were heavily invaded by new capillary vessels, and contained 2.4 g/dl hemoglobin. Matrigel which lacked bFGF was translucent and gray and contained only 0.4 g/dl hemoglobin (a 6-fold difference). In contrast, matrigel from mice with a primary tumor contained only 0.5 g/dl (Figure 4).

The near complete inhibition of angiogenesis in this experiment suggests that the presence of a Lewis lung primary tumor can inhibit bFGF-induced angiogenesis directly.

**Example 7**

*Transfer of serum from a tumor-bearing animal to an animal from which the primary tumor has been removed suppresses metastases.*

Mice were implanted with Lewis lung carcinoma as described above. After 15 days, when tumors were approximately 1500 $mm^3$, the mice were randomized into four groups. Three groups underwent complete surgical resection of the primary tumor; in one group the tumors were left in place (after a sham surgical procedure). The mice in the three resection groups then received daily intraperitoneal injections of saline, serum from normal nontumor bearing mice, or serum from mice with 1500 $mm^3$ Lewis lung carcinomas. The group of mice with the tumors left intact received intraperitoneal saline injections. All mice were treated for 21 days, after which the animals were euthanized and lung metastases were counted (Table 2).

**Table 2**

| | Primary Tumor Removed | | | Primary Intact |
|---|---|---|---|---|
| Treatment (Intraperitoneal Injections) | Saline | Serum from normal mice | Serum from tumor-bearing mice | Saline Injections |
| Number of Lung Metastases: | 55±5 | 50±4 | 7±2 | 3±1 |

These results were confirmed by lung weight. p = < 0.0001 for the difference between the two groups [(55 & 50) vs. (7 & 3)]. Similar results have been obtained using angiostatin from the urine of tumor-bearing animals.

**Example 8**

*Bovine capillary endothelial (BCE) cell assay*

BCE cells are used between passages 9 and 14 only. At day 0, BCE cells are plated onto gelatinized (1.5 % gelatin in PBS at 37°, 10% $CO_2$ for 24 hours and then rinsed with 0.5 ml PBS) 24 well plates at a concentration of 12,500 cells/well. Cell counts are performed using a hemocytometer. Cells are plated in 500μl DMEM with 10% heat-inactivated (56°C for 20 minutes) bovine calf serum and 1% glutamine-pen-strep (GPS).

BCE cells are challenged as follows: Media is removed and replaced with 250 μl of DMEM/5% BCS/1%GPS. The sample to be tested is then added to wells. (The amount varies depending on the sample being tested) Plates are placed at 37*C/10% $CO_2$ for approximately 10 minutes. 250 μl of DMEM/5% BCS/1% GPS with 2ng/ml bFGF is added to each well. The final media is 500μl of DMEM/5% BCS/1%GPS/ with 1 ng/ml bFGF. The plate is returned to 37°C/ 10% $CO_2$ incubator for 72 hours.

At day 4, cells are counted by removing the medium and then trypsinizing all wells (0.5 ml trypsin/EDTA) for 2 to 3 minutes. The suspended cells are then transferred to scintillation vials with 9.5 ml Hemetall and counted using a Coulter counter. A unit of activity is that amount of serum containing angiostatin that is capable of producing half-maximal inhibition of capillary endothelial proliferation when endothelial cells are incubated in bFGF 1 ng/ml for 72 hours.

**Example 9**

*Serum from mice bearing the low metastatic Lewis lung tumor (LLC-Low) inhibits capillary endothelial cell proliferation in vitro.*

Bovine capillary endothelial cells were stimulated by basic fibroblast growth factor (bFGF 1 ng/ml), in a 72-hour proliferation assay. The serum of tumor-bearing mice added to these cultures inhibited endothelial cell proliferation in a dose-dependent and reversible manner. Normal serum was not inhibitory (Figure 5). Endothelial cell proliferation was inhibited in a similar manner (relative to controls) by serum obtained from tumor-bearing nu/nu mice and SCID mice. After the primary tumor was removed, angiostatin activity disappeared from the serum by 3-5 days.

Tumor-bearing serum also inhibited bovine aortic endothelial cells and endothelial cells derived from a spontaneous mouse hemangioendothelioma, (Obeso, et al., "Methods in Laboratory Investigation, A Hemangioendothelioma-derived cell line; Its use as a Model for the Study of Endothelial Cell Biology," Lab Invest., 63(2), pgs 259-269, 1990) but did not inhibit Lewis lung tumor cells, 3T3 fibroblasts, aortic smooth muscle cells, mink lung epithelium, or W138 human fetal lung fibroblasts.

**Example 10**

*Serum from mice bearing the Lewis lung tumor (LLC-High) that does not inhibit metastases, does not inhibit capillary endothelial cell proliferation in vitro.*

Serum from mice bearing a primary tumor of the LLC-High did not significantly inhibit proliferation of bFGF-stimulated bovine capillary endothelial cells relative to controls. Also, when this serum was subjected to the first two steps of purification (heparin-Sepharose chromatography and gel filtration), angiostatin activity was not found in any fractions.

**Example 11**

*Ascites from Lewis lung carcinoma (low metastatic), also generates angiostatin serum.*

Mice received intraperitoneal injections of either LLC-Low or LLC-High tumor cells ($10^6$), and one week later, 1-2 ml of bloody ascites was obtained from each of 10-20 mice. Mesenteric tumor seeding was seen. The mice were then euthanized. Serum was obtained by cardiac puncture. Serum was also obtained from normal, non-tumor-bearing mice as a control. Serum and ascites were centrifuged to remove cells, and the supernate was assayed on bovine capillary endothelial cells stimulated by bFGF (1 ng/ml) (see Example IX). Ascites originating from both tumor types stimulated significant proliferation of capillary endothelial cells (e.g., 100% proliferation) over controls after 72 hours (Figure 6). In contrast, serum from the low metastatic mice inhibited endothelial cell proliferation (inhibition to 79% of controls). The serum from the high metastatic line was stimulatory by 200%.

These data show that the ascites of the low metastatic line contains a predominance of endothelial growth stimulator over angiostatin. This condition is analogous to a solid primary tumor. Furthermore, angiostatin activity appears in the serum, as though it were unopposed by stimulatory activity. This pattern is similar to the solid primary tumor (LLC-Low). The ascites from the high metastatic tumor (LLC-High) also appears to contain a predominance of endothelial cell stimulator, but angiostatin cannot be identified in the serum.

**Example 12**

*Fractionation of angiostatin from serum by column chromatography and analysis of growth-inhibitory fractions by SDS-PAGE.*

To purify the angiostatin(s), serum was pooled from tumor-bearing mice. The inhibitory activity, assayed according the above-described *in vitro* inhibitor activity assay, was sequentially chromatographed using heparin-Sepharose, Biogel AO.5mm agarose, and several cycles of C4-reverse phase high performance liquid chromatography (HPLC). SDS-PAGE of the HPLC fraction which contained endothelial inhibitory activity, revealed a discrete band of apparent reduced $M_r$ of 38,000 Daltons, which was purified approximately 1 million-fold (see Table 3) to a specific activity of approximately $2x10^7$. At different stages of the purification, pooled fractions were tested with specific antibodies for the presence of known endothelial inhibitors. Platelet factor-4, thrombospondin, or transforming growth factor beta, were not found in the partially purified or purified fractions.

**Table 3.**

| | Specific activity (units*/mg) | Fold purification |
|---|---|---|
| Serum | 1.69 | 1 |
| Heparin Sepharose | 14.92 | 8.8 |
| Bio-gel AO.5m | 69.96 | 41.4 |
| HPLC/C4 | $2x10^7$ | $1.2x10^6$ |

*A unit of activity is that amount of serum containing angiostatin that is capable of producing half maximal inhibition of capillary endothelial proliferation when endothelial cells are incubated in bFGF 1 ng/ml for 72 hours.

**Example 13**

*Fractionation of angiostatin from urine by column chromatography and analysis of growth-inhibitory fractions by SDS-PAGE.*

Purification of the endothelial cell inhibitor(s) from serum is hampered by the small volume of serum that can be obtained from each mouse and by the large amount of protein in the serum.

Urine from tumor bearing mice was analyzed and found that it contains an inhibitor of endothelial cell proliferation that is absent from the urine of non-tumor bearing mice and from mice with LLC-high tumors. Purification of the endothelial cell inhibitory activity was carried out by the same strategy that was employed for purification of serum (described above) (Figure 7).

Figure 7 shows C4 reverse phase chromatography of partially purified serum or urine from tumor-bearing animals. All fractions were assayed on bovine capillary endothelial cells with bFGF in a 72-hour proliferation assay as described in Example IX. A discrete peak of inhibition was seen in both cases eluting at 30 - 35 % acetonitrile in fraction 23. SDS-polyacrylamide gel electrophoresis of inhibitory fraction from the third cycle of C4 reverse phase chromatography of serum from tumor-bearing animals showed a single band at about 38,000 Daltons.

**Example 14**

*Characterization of circulating angiostatin.*

Endothelial inhibition was assayed according to the procedure described in Example 9. Angiostatin was isolated on a Synchropak HPLC C4 column. (Synchrom, Inc. Lafayette, IN) The inhibitor was eluted at 30 to 35% acetonitrile gradient. On a sodium dodecyl sulfate polyacrylamide gel electrophoresis (PAGE) gel under reducing conditions (β-mercaptoethanol(5% v/v), the protein band with activity eluted at 38 kilodaltons. Under non-reducing conditions, the protein with activity eluted at 28 kilodaltons. The activity is found at similar points whether the initial sample was isolated from urine or from serum. Activity was not detected with any other bands.

Activity associated with the bands was lost when heated (100°C for 10 minutes) or treated with trypsin. When the band with activity was extracted with a water/chloroform mixture (1:1), the activity was found in the aqueous phase only.

**Example 15**

*Purification of inhibitory fragments from human plasminogen:*

Plasminogen lysine binding site I was obtained from Sigma Chemical Company. The preparation is purified human plasminogen after digestion with elastase. Lysine binding site I obtained in this manner is a population of peptides that contain, in aggregate, at least the first three triple-loop structures (numbers 1 through 3) in the plasmin A-chain (Kringle 1 +2+3). (Sotrrup-Jensen, L., et al. in Progress in Chemical Fibrinolysis and Thrombolysis, Vol. 3, 191, Davidson, J.F., et al. eds. Raven Press, New York 1978 and Wiman, B., et al., Biochemica et Biophysica Acta, 579, 142 (1979)). Plasminogen lysine binding site I (Sigma Chemical Company, St. Louis, MO) was resuspended in water and applied to a C4-reversed phase column that had been equilibrated with HPLC-grade water/0.1% TFA. The column was eluted with a gradient of water/0.1% TFA to acetonitrile/0.1% TFA and fractions were collected into polypropylene tubes. An aliquot of each was evaporated in a speed vac, resuspended with water, and applied to BCEs in a proliferation assay. This procedure was repeated two times for the inhibitory fractions using a similar gradient for elution. The inhibitory activity eluted at 30-35% acetonitrile in the final run of the C4 column. SDS-PAGE of the inhibitory fraction revealed 3 discrete bands of apparent reduced molecular mass of 40, 42.5, and 45 kd. SDS-PAGE under non-reducing conditions revealed three bands of molecular mass 30, 32.5, and 35 kd respectively.

**Example 16**

*Extraction of inhibitory activity from SDS-PAGE*

Purified inhibitory fractions from human plasminogen based purifications were resolved by SDS-PAGE under non-denaturing conditions. Areas of the gel corresponding to bands seen in neighboring lanes loaded with the same samples by silver staining were cut from the gel and incubated in 1 ml of phosphate buffered saline at 4°C for 12 hours in polypropylene tubes. The supernatant was removed and dialyzed twice against saline for 6 hours (MWCO = 6-8000) and twice against distilled water for 6 hours. The dialysate was evaporated by vacuum centrifugation. The product was resuspended in saline and applied to bovine capillary endothelial cells stimulated by 1 ng/ml basic fibroblast growth factor in a 72 hour assay. Protein extracted from each of the three bands inhibited the capillary endothelial cells.

**Example 17**

*Plasminogen Fragment Treatment Studies*

Mice were implanted with Lewis lung carcinomas and underwent resections when the tumors were 1500-2000 $mm^3$. On the day of operation, mice were randomized into 6 groups of 6 mice each. The mice received daily intraperitoneal injections with the three purified inhibitory fragments of human plasminogen, whole human plasminogen, urine from tumor-bearing animals, urine from normal mice, or saline. One group of tumor-bearing animals that had only a sham procedure was treated with saline injections. Immediately after removal of the primary tumor, the mice receive an intraperitoneal injection of 24 μg (1.2 mg/kg/day/mouse) of the inhibitory plasminogen fragments as a loading dose.

They then receive a daily intraperitoneal injections of 12 μg of the inhibitory fragment (0.6 mg/kg/day/mouse) for the duration of the experiment. Control mice receive the same dose of the whole plasminogen molecule after tumor removal. For the urine treatments, the urine of normal or tumor bearing mice is filtered, dialyzed extensively, lyophilized, and then resuspended in sterile water to obtain a 250 fold concentration. The mice are given 0.8 ml of the dialyzed urine concentrate, either from tumor bearing mice or normal mice, in two intraperitoneal injections on the day of removal of the primary tumor as a loading dose. They then receive daily intraperitoneal injections of 0.4 ml of the dialyzed and concentrated urine for the course of the experiment. Treatments were continued for 13 days at which point all mice were sacrificed and autopsied.

The results of the experiment are shown in Figures 8 and 9. Figure 8 shows surface lung metastases after the 13 day treatment. Surface lung metastases refers to the number of metastases seen in the lungs of the mice at autopsy. A stereomicroscope was used to count the metastases. Figure 8 shows the mean number of surface lung metastases that was counted and the standard error of the mean. As shown, the group of mice with the primary tumor present showed no metastases. The mice in which the primary tumor was resected and were treated with saline showed extensive metastases. The mice treated with the human derived plasminogen fragment showed no metastases. The mice treated with whole plasminogen showed extensive metastases indicating that the whole plasminogen molecule has no endothelial inhibitory activity. Those mice treated with dialyzed and concentrated urine from tumor bearing mice showed no metastases. Mice treated with concentrated urine from normal mice showed extensive metastases. When the weight of the lung was measured, similar results were obtained (Figure 9).

**Example 18**

*Amino acid sequence of murine and human angiostatin.*

The amino acid sequence of angiostatin isolated from mouse urine and angiostatin isolated from the human lysine binding site I fragment preparation was determined on an Applied Biosystem Model 477A protein sequencer. Phenylthiohydantoin amino acid fractions were identified with an on-line ABI Model 120A HPLC. The amino acid sequence determined from the N-terminal sequence and the tryptic digests of the murine and human angiostatin indicate that the sequence of the angiostatin is similar to the sequence beginning at amino acid number 98 of murine plasminogen. Thus, the amino acid sequence of the angiostatin is a molecule comprising a protein having a molecular weight of between approximately 38 kilodaltons and 45 kilodaltons as determined by reducing polyacrylamide gel electrophoresis and having an amino acid sequence substantially similar to that of a murine plasminogen fragment beginning at amino acid number 98 of an intact murine plasminogen molecule. The beginning amino acid sequence of the murine angiostatin (SEQ ID NO:2) is shown in Figure 1. The length of the amino acid sequence may be slightly longer or shorter than that shown in the Figure 1.

N terminal amino acid analysis and tryptic digests of the active fraction of human lysine binding site I (See Example 15) show that the sequence of the fraction begins at approximately amino acid 97 or 99 of human plasminogen and the human angiostatin is homologous with the murine angiostatin. The beginning amino acid sequence of the human angiostatin (starting at amino acid 98) is shown in Figure 2, (SEQ ID NO:3). The amino acid sequence of murine and human angiostatin is compared in Figure 2 to corresponding internal amino acid sequences from plasminogen of other species including porcine, bovine, and Rhesus monkey plasminogen, indicating the presence of angiostatin in those species.

**Example 19**

*Expression of human angiostatin in E. coli.*

The pTrcHisA vector (Invitrogen) (Fig. 10) was used to obtain high-level, regulated transcription from the trc promoter for enhanced translation efficiency of eukaryotic genes in *E. coli.* Angiostatin is expressed fused to an N-terminal nickel-binding poly-histidine tail for one-step purification using metal affinity resins. The enterokinase cleavage recognition site in the fusion peptide allows for subsequent removal of the N-terminal histidine fusion peptide from the purified recombinant protein. The recombinant human angioststin protein was found to bind lysine; is cross-reactive with monoclonal antibodies specific for kringle regions 1, 2 and 3, and inhibits bFGF-driven endothelial cell proliferation *in vitro.*

To construct the insert, the gene fragment encoding human angiostatin is obtained from human liver mRNA which is reverse transcribed and amplified using the polymerase chain reaction (PCR) and specific primers. The product of 1131 base pairs encodes amino acids 93 to 470 of human plasminogen. The amplified fragment was cloned into the XhoI/KpnI site of pTrcHisA, and the resultant construct transformed into XL-1B (available from Stratagene) E. coli host cells. A control clone containing the plasmid vector pTrcHisA alone was transformed inot XL-1B E. coli host cells as well. This clone is referred to as the vector control clone. Both clones were purified identically as described below.

Expressing colonies were selected in the following manner. Colony lifts of *E. coli* transformed with the gene encoding angiostatin were grown on IPTG impregnated nitrocellulose filters and overlaid on an LB agar plate. Following IPTG induction of expression, colonies were lysed on nitrocellulose filters. The nitrocellulose lifts were blocked, rinsed and probed with two separate monoclonal antibodies (mAbs Dcd and Vap; gift of S.G. McCance and F.J. Castellino, University of Notre Dame) which recognize specific conformations of angiostatin. Strongly expressing colonies recognized by the mAbs were selected.

To identify the optimal time for maximal expression, cells were collected at various times before and after IPTG induction and exposed to repeated freeze-thaw cycles, followed by analysis with SDS-PAGE, immunoblotting and probing with mAbs Dcd and Vap.

From these, clone pTrcHisA/HAsH4 was selected. Induction with IPTG was for 4 hours after which the cell pellet was collected and resuspended in 50 mM Tris pH 8.0, 2 mM EDTA, 5% glycerol and 200 mg/ml lysozyme and stirred for 30 min. at 4°C. The slurry was centrifuged at 14,000 rpm for 25 min. and the pellet resuspended in 50 mM Tris pH 8.0, 2 mM EDTA, 5% glycerol and 0.1% DOC. This suspension was stirred for 1 hr. at 4°C, and then centrifuged at 14,000 rpm for 25 min. The supernatant fraction at this step contains expressed angiostatin. The E. coli expressed human angiostatin was found to possess the physical property of native angiostatin, that is the ability to bind lysine. The E. coli expressed angiostatin was thus purified over a lysine-sepharose (Pharmacia or Sigma) column in a single step. Elution of angiostatin from the column was with 0.2M epsilon-amino-n-caproic acid pH7.5.

Subsequent to these experiments, scale-up 10 L fermentation batches of clone pTrcHisA/HAsH4 was performed. The cells obtained from this scaled-up induction were pelleted and resuspended in50 mM Tris pH7.5, cracked at 10,000 psi thrice chilling at 10 °C in-between passes. The lysate obtained was clarified by centrifugation at 10,000 rpm for 30 min at 4°C, and expressed angiostatin isolated over lysine-sepharose (Fig. 11).

Purified E. coli expressed human angiostatin was dialysed exhaustively against water and lyophilized. The expressed human angiostatin was resuspended in media (DMEM, 5% BCS, 1% Gentamycin/ penicillin/streptomycin) to an estimated concentration of 3 ug/ml, and used in bovine capillary endothelial (BCE) cell assays *in vitro,* as described in EXAMPLE 8, pg.39. Similarly, the control clone containing the vector alone was treated in the identical fashion as the clone pTrcHisA/HAsH4. It was induced with IPTG identically, and the bacterial lysate used to bind lysine, eluted with 0.2 M amino caproic acid, dialysed exhaustively and lyophilized. This control preparation was resuspended in media also at an estimated concentration of 3 ug/ml. The samples of recombinant angiostatin, and controls were obtained from different induction and fermentation batches as well as seperate purification runs, and were all coded at EntreMed, Maryland. BCE assays were performed with these coded samples in a blinded fashion at Children's Hospital, Boston.

The results of BCE assays of recombinant human angiostatin showed that human angiostatin expressed in E.coli inhibited the proliferation of BCE cells due to bFGF (used at 1 ng/ml) (Fig. 12). The stock recombinant angiostatin in media (at about 3 ug/ml) was used at a 1:5, 1:10 and 1:20 dilution. Percent inhibition was calculated as follows:

$$1 - \frac{\text{number of cells with angiostatin - number of cells at day 0}}{\text{number of cells with bFGF alone - number of cells at day 0}}$$

The percent inhibition of BCE cell proliferation was comparable or higher to that of plasminogen derived angiostatin at similar concentrations. The results from a repeat run of the BCE assay are depicted in Fig.13, where at a 1:5 dilution of the stock, recombinant angiostatin gave similar percent inhibitions to those obtained with plasminogen derived angiostatin. Figure 13 shows the surprising result that human recombinant angiostatin protein inhibits over 60%, and as much as over 75% of BCE proliferation in culture.

## Example 20

*Angiostatin maintains dormancy of micrometastases by increasing the rate of apoptosis.*

Following subcutaneous inoculation of C57 BL6/J mice with Lewis lung carcinoma cells (1 x$10^6$), primary tumors of approximately 1.5 $cm^3$ developed. Animals were subject to either surgical removal of the primary tumor or sham surgery. At 5, 10 and 15 days after surgery, mice were sacrificed and their lungs prepared for histological examination. Animals with resected primary tumors showed massive proliferation of micrometastases compared to sham operated controls (Fig. 14). These changes were accompanied by a significant increase in lung weight.

Analysis of tumor cell proliferation, as measured by uptake of bromo-deoxyuridine (BrdU) showed no differences between animals with intact primary tumors or resected tumors at 5, 9 and 13 days, indicating that the increase in tumor mass could not be explained by increased proliferation (Fig. 15). Accordingly, cell death was examined in these animals. Apoptosis, a process of cell death that is dependent on changes in gene expression and accounts for elimination of cells during development and in rapidly proliferating tissues such as the small intestine, was examined by immunohistochemically labeling fragmented DNA with the terminal deoxynucleotidyl transferase (TdT) technique. The apoptotic index was determined at each time of sacrifice. The removal of primary tumors caused a statistically significant increase (approximately 3 to 4 fold) in the apoptotic index at all times examined (Fig. 15).

Supporting evidence was obtained by treating mice with removed primary tumors with an exogenous suppressor of angiogenesis. This substance, TNP-1470 (O-chloroacetylcarbamoyl fumagillol, previously named AGM-1470), is an analogue of fumagillin with reported anti-angiogenic activity. Subcutaneous injection of TNP-1470 (30 mg/kg every two days) produced results that were strikingly similar to those described above for animals that had intact primary tumors. These animals displayed a lower lung weight, equivalent proliferative index and increased apoptotic index compared to saline-injected controls (Fig. 16).

These data indicate that metastases remain dormant when tumor cell proliferation is balanced by an equivalent rate of cell death. The removal of the primary tumor causes a rapid increase in the growth of metastases, probably due to the removal of angiogenesis inhibitors (angiostatin) which control metastatic growth by increasing apoptosis in tumor cells. These effects are similar to those seen following removal of primary tumors and administration of an exogenous inhibitor of angiogenesis. Taken together, these data suggest that the primary tumor releases angiostatin which maintains dormancy of micrometastases.

**Example 21**

*Treatment of primary tumors with angiostatin in vivo.*

Angiostatin was purified from human plasminogen by limited elastase digestion as described in Example 15 above. Angiostatin was resuspended in phosphate-buffered saline for administration into six week old male C57BI6/J mice. Animals were implanted subcutaneously with 1 X $10^6$ tumor cells of either the Lewis lung carcinoma or T241 fibrosarcoma. Treatment with angiostatin is begun after four days when tumors are 80-160 $mm^3$ in size. Mice received angiostatin injections in either a single injection of 40 mg/kg or two 80 mg/kg injections via intraperitoneal (ip) or subcutaneous (sc) routes. Animals were sacrificed at various times after treatment extending to 19 days.

Angiostatin, administered at a daily dose of 40 mg/kg ip, produced a highly significant inhibition of the growth of T241 primary tumors (Fig. 17). This inhibitory effect on growth was visibly evident within 2 days and increased in magnitude throughout the time course of the study. By day 18, angiostatin-treated mice had tumors that were approximately 38% of the volume of the saline injected controls. This difference was statistically significant ($p<0.001$, Students t-test).

Angiostatin treatment (total dose of 80 mg/kg/day, administered twice daily at 40 mg/kg ip or sc) also significantly reduced the growth rate of LLC-LM primary tumors (Fig. 17). This inhibitory effect was evident at 4 days and increased in magnitude at all subsequent times examined. On the last day of the experiment (day 19), angiostatin-treated mice possessed a mean tumor volume that was only 20% of the saline-injected controls which was significantly different ($p<0.001$ Students t-test).

In another series of experiments angiostatin was administered (50 mg/kg q12h) to mice implanted with T241 fibrosarcoma, Lewis lung carcinoma (LM) or reticulum cell sarcoma cells. For each tumor cell type, the mice receiving angiostatin had substantially reduced tumor size. Figure 19 demonstrates that for T241 fibrosarcoma, the angiostatin treated mice had mean tumor volumes that were only 15% of the untreated mice at day 24. Figure 20 demonstrates that for Lewis lung carcinoma (LM), the angiostatin treated mice had mean tumor volumes that were only 13% of the untreated mice at day 24. Figure 21 demonstrates that for reticulum sacroma, the angiostatin treated mice had mean tumor volumes that were only 19% of the untreated mice at day 24. The data represent the average of 4 mice at each time point.

These results demonstrate that angiostatin is an extremely potent inhibitor of the growth of three different primary tumors *in vivo.*

**Example 22**

*Treatment of human cell-derived primary tumors in mice with angiostatin in vivo.*

The effect of angiostatin on two human tumor cell lines, human prostate carcinoma PC-3 and human breast carcinoma MDA-MB, was studied. Immunodeficient SCID mice were implanted with human tumor cells, and the mice treated with 50 mg/kg angiostatin every 12 hours essentially as described in Example 21. The results demonstrate that the angiostatin protein of the present invention is a potent inhibitor of human tumor cell growth. Figure 22 shows that for human prostate carcinoma PC-3, the angiostatin treated mice had only 2% of the mean tumor volume compared to the untreated control mice at day 24. Figure 23 shows that for human breast carcinoma MDA-MB, he angiostatin treated mice had only 8% of the mean tumor volume compared to the untreated control mice at day 24.

**Example 23**

*Gene Therapy - Effect of transfection of the angiostatin gene on tumor volume.*

A 1380 base pair DNA sequence for angiostatin derived from mouse plasminogen cDNA (obtained from American Type Culture Collection (ATCC)), coding for mouse plasminogen amino acids 1 - 460, was generated using PCR and inserted into an expression vector. The expression vector was transfected into T241 fibrosarcoma cells and the transfected cells were implanted into mice. Control mice received either non-transfected T241 cells, or T241 cells transfected with the vector only (i.e. non-angiostatin expressing transfected cells). Three angiostatin-expressing transfected cell clones were used in the experiment. Mean tumor volume determined over time. The results show the surprising and dramatic reduction in mean tumor volume in mice for the angiostatin-expressing cells clones as compared with the non-transfected and non-expressing control cells.
The mouse DNA sequence coding for mouse angiostatin protein is derived from mouse plasminogen cDNA. The mouse angiostatin encompasses mouse plasminogen kringle regions 1-4. The schematic for constructing this clone is shown in Figure 24.
The mouse angiostatin protein clones were transfected into T241 fibrosarcoma cells using the LIPOFECTIN™ transfection system (available from Life Technologies, Gaithersburg, MD). The LIPOFECTIN™ reagent is a 1:1 (w/w) liposome formulation of the cationic lipid N-[1-(2,3-dioleyloxy)propyl]-n,n,n-trimethylammonium chloride (DOTMA), and diolecoyl phosphotidylethanolamine (DOPE) in membrane filtered water.
The procedure for transient transfection of cells is as follows:

1. T241 cells are grown in 60 $cm^2$ tissue culture dishes, seed ≈1-2 x $10^5$ cells in 2 ml of the appropriate growth medium supplemented with serum.

2. Incubate the cells at 37°C in a $CO_2$ incubator until the cells are 40-70% confluent. will usually take 18-24 h, but the time will vary among cell types. The T241 tumor cells confluency was approximately 70%.

3. Prepare the following solutions in 12 x 75 mm sterile tubes:

   Solution A: For each transfection, dilute 5 μg of DNA in 100 μl of serum-free OPTI-MEM I Reduced Serum Medium (available from Life Technologies) (tissue culture grade deionized water can also be used).
   Solution B: For each transfection, dilute 30 μg of LIPOFECTIN in 100 μl OPTI-MEM medium.

4. Combine the two solutions, mix gently, and incubate at room temperature for 10-15 min.

5. Wash cells twice with serum-free medium.

6. For each transfection, add 0.8 ml serum-free medium to each tube containing the LIPOFECTIN™ reagent-DNA complexes. Mix gently and overlay the complex onto cells.

7. Incubate the cells for approximately 12 h at 37°C in a $CO_2$ incubator.

8. Replace the DNA containing medium with 1 mg/ml selection medium containing serum and incubate cells at 37°C in a $CO_2$ incubator for a total of 48-72 h.

9. Assay cell extracts for gene activity at 48-72 h post transfection.

Transfected cells can be assayed for expression of angiostatin protein using angiostatin-specific antibodies. Alternatively, after about 10-14 days, G418 resistant colonies appeared in the CMV-angiostatin transfected T241 cells. Also, a number of clones were seen in the vector alone transfected clones but not in the untransfected clones. The G418 resistant clones were selected for their expression of angiostatin, using a immunofluorence method.
Interestingly, the *in vitro* cell growth T241 cells and Lewis lung cells transfected with angiostatin was not inhibited or otherwise adversely affected, as shown in Figures 25 and 26.

Figure 27 depicts the results of the transfection experiment. All three of the angiostatin-expressing T241 transfected clones produced mean tumor volumes in mice that were substantially reduced relative to the tumor volume in contol mice. The mean tumor volume of the mice implanted with Clone 37 was only 13% of the control, while Clone 31 and Clone 25 tumor volumes were only 21% and 34% of the control tumor volumes, respectively. These results demonstrate that the DNA sequences coding for angiostatin can be transfected into cells, that the transfected DNA sequences are capable of expressing angiostatin protein by implanted cells, and that the expressed angiostatin fucntions *in vivo* to reduce tumor growth.

**Example 24**

*Localization of in vivo site of angiostatin expression*

To localize the *in vivo* site of expression of angiostatin protein, total RNA from various cell types, Lewis lung carcinoma cells (mouse), T241 fibrosarcoma (mouse), and Burkitt's lymphoma cells (human), both from fresh tumor or cell culture after several passages were analysed to determine the presence of angiostatin transcripts. Northern analysis of samples showed an absence of any signal hybridizing with thn sequence from all samples except that of normal mouse liver RNA showing a single signal of approximately 2.4 kb corresponding to mouse plasminogen. Northern analysis of human samles show an absence of any signal hybridizing with human angiostatin sequence from all samples except that of normal human liver RNA showing a single signal of approximately 2.4 kb corresponding to human plasminogen.

Reverse transcription polymerase chain reaction (RT-PCR) analysis showed an absence of any product from all samples probed with mouse angiostatin sequences except that of the normal mouse liver. RT-PCR analysis showed an absence of any product from all human samples probed with human angiostatin sequences except that of the normal human liver (expected size of 1050 bp for mouse and 1134 bp for human).

Thus it appears that mouse angiostatin transcripts (assuming identity with amino acids 97 to 450 of mouse plasminogen) are not produced by all the above mouse samples and human angiostatin transcripts (assuming identity with amino acids 93 to 470 of human plasminogen) are not produced by the above human samples. The positive signals obtained in normal mouse/human liver is from hybridization with plasminogen.

**Example 25**

*Expression of Angiostatin in Yeast*

The gene fragment encoding amino acids 93 to 470 of human plasminogen was cloned into the XhoI/EcoRI site of pHIL-SI(Invitrogen) which allows the secreted expression of proteins using the PHO1 secretion signal in the yeast *Pichia pastoris.* Similarly, the gene fragment encoding amino acids 93 to 470 of human plasminogen was cloned into the SnaBI/EcoRI site of pPIC9 (Invitrogen) which allows the secreted expression of proteins using the $\alpha$-factor secretion signal in the yeast *Pichia pastoris.* The expressed human angiostatin proteins in these systems will have many advantages over those expressed in E. coli such as protein processing, protein folding and posttranslational modification inclusive of glycosylation.

Expression of gene in *P. pastoris:* is described in) Sreekrishna, K. et al. (1988) High level expression of heterologous proteins in methylotropic yeast Pichia pastoris. J. Basic Microbiol. 29 (4): 265-278, and Clare, J.J. et al. (1991) Production of epidermal growth factor in yeast: High-level secretion using Pichia pastoris strains containing multiple gene copies, Gene 105:205-212.

**Example 26**

*Expression of angiostatin proteins in transgenic animals and plants*

Transgenic animals such as of the bovine or procine family are created which express the angiostatin gene transcript. The transgenic animal express angiostatin protein for example in the milk of these animals. Additionally edible transgenic plants which express the angiostatin gene transcript are constructed.

Constructing transgenic animals that express foreign DNA is described in Smith H. Phytochrome transgenics: functional, ecological and biotechnical applications, Semin. Cell. Biol. 1994 5(5):315-325.

## Claims

1. A composition comprising a vector comprising a nucleotide sequence encoding a protein termed angiostatin, the protein having a molecular weight of between approximately 38 kilodaltons and 45 kilodaltons as determined by reducing polyacrylamide gel electrophoresis and having an amino acid sequence beginning at approximately amino acid number 98 of plasminogen, or having an amino acid sequence of plasminogen kringle regions 1 through 3 and part of kringle 4, wherein the protein has anti-angiogenic activity, wherein the vector is a viral vector or a non viral vector and is capable of expressing angiostatin when present in a cell.

2. The composition as claimed in claim 1 wherein the encoded angiostatin having anti-angiogenic activity has at least 70% homology to the sequence of SEQ ID NO: 2.

3. The composition according to Claim 1 or 2, wherein the vector is a plasmid.

4. The composition according to any of Claims 1 to 3, wherein the protein is a fragment of human plasminogen, murine plasminogen, bovine plasminogen, Rhesus plasminogen or porcine plasminogen.

5. The composition according to any of Claims 1 to 4, wherein the protein has the first 339 amino acids of its amino acid sequence selected from a group consisting of SEQ ID NO:2, SEQ ID NO:3, SEQ ID NO:4, SEQ ID NO:5, and SEQ ID NO:6.

6. A composition comprising a viral vector or a non-viral vector comprising a nucleotide sequence encoding a plasminogen fragment having anti-angiogenic activity that contains at least plasminogen kringle regions 1 through 3, wherein the plasminogen fragment is a fragment of human plasminogen obtainable by digestion of plasminogen with elastase.

7. The composition according to any of Claim 1 or 2 or Claims 4 to 6, wherein the vector is a viral vector selected from a group consisting of a retrovirus vector, a poliovirus vector, a Sindbis virus vector, an adenovirus vector, an adeno-associated virus vector, a herpes virus vector, an SV 40 vector and a vaccinia virus vector.

8. The composition according to Claim 7, wherein the vector is a retrovirus vector or an adenovirus vector.

9. The composition according to any of Claims 1 to 8, wherein the composition is for therapeutic use.

10. The composition as claimed in Claim 6 for therapeutic use.

11. The composition as claimed in Claim 6 and a pharmaceutically acceptable excipient.

12. The composition according to any of Claims 1 to 11, wherein the composition is for use in a method for inhibiting angiogenesis in a human or an animal.

13. The composition according to any of Claims 1 to 12, wherein the composition is for use in a method for treating a process or a disease that is mediated by angiogenesis.

14. The composition according to Claim 13, wherein the process or the disease is selected from the group consisting of hemangioma, solid tumors, blood borne tumors, leukemia, metastasis, telangiectasia, psoriasis, scleroderma, pyogenic granuloma, myocardial angiogenesis, Crohn's disease, plaque neovascularization, coronary collaterals, cerebral collaterals, arteriovenous malformations, ischemic limb angiogenesis, corneal diseases, rubeosis, neovascular glaucoma, diabetic retinopathy, retrolental fibroplasia, arthritis, diabetic neovascularization, macular degeneration, wound healing, peptic ulcer, *Helicobacter* related diseases, fractures, keloids, vasculogenesis, hematopoiesis, ovulation, menstruation, placentation, and cat scratch fever.

15. The composition according to any of Claims 1 to 14, wherein the composition is for use in a method for treating or for repressing growth of tumors in a human or an animal.

**Patentansprüche**

1. Zusammensetzung, die einen Vektor umfasst, der eine Nukleotidsequenz unfasst, die für ein als Angiostatin bezeichnetes Protein codiert, wobei das Protein ein Molekulargewicht zwischen ungefähr 38 Kilodalton und 45 Kilodalton hat, bestimmt mit reduzierender Polyacrylamid-Gelelektrophorese, und wobei das Protein eine Aminosäuresequenz, die ungefähr bei der Aminosäure Nummer 98 des Plasminogens anfängt, oder eine Aminosäuresequenz der Plasminogen Kringelregionen 1 bis 3 und eines Teils der Kringelregion 4 hat, wobei das Protein anti-angiogene Aktivität hat, wobei der Vektor ein viraler Vektor oder ein nicht-viraler Vektor ist und im Stande ist, Angiostatin zu exprimieren, wenn er in einer Zelle vorhanden ist.

2. Zusammensetzung wie in Anspruch 1 beansprucht, wobei das codierte Angiostatin, das anti-angiogene Aktivität hat, mindestens 70% Homologie mit der Sequenz der SEQ ID NO: 2 hat.

3. Zusammensetzung gemäß Anspruch 1 oder 2, wobei der Vektor ein Plasmid ist.

4. Zusammensetzung gemäß einem der Ansprüche 1 bis 3, wobei das Protein ein Fragment des Menschen Plasminogens, des murinen Plasminogens, des Rinder Plasminogens, des Rhesus Plasminogens oder des Schweine Plasminogens ist.

5. Zusammensetzung gemäß einem der Ansprüche 1 bis 4, wobei die ersten 339 Aminosäuren der Aminosäuresequenz des Proteins aus einer Gruppe bestehend aus SEQ ID NO: 2, SEQ ID NO: 3, SEQ ID NO: 4, SEQ ID NO: 5 und SEQ ID NO: 6 ausgewählt sind.

6. Zusammensetzung, die einen viralen Vektor oder einen nicht-viralen Vektor umfasst, der eine Nukleotidsequenz umfasst, die ein Plasminogenfragment mit antiangiogener Aktivität codiert, das mindestens die Plasminogen Kringelregionen 1 bis 3 enthält, wobei das Plasminogenfragment ein Fragment des Menschen Plasminogens ist, das durch Verdau von Plasminogen mit Elastase erhältlich ist.

7. Zusammensetzung gemäß einem der Ansprüche 1 oder 2 oder der Ansprüche 4 bis 6, wobei der Vektor ein viraler Vektor ist, der aus der Gruppe bestehend aus einem Retrovirus Vektor, einem Poliovirus Vektor, einem Sindbis Virus Vektor, einem Adenovirus Vektor, einem adeno-assoziierten Virus Vektor, einem Herpes Virus Vektor, einem SV40 Virus Vektor und einem Vakzinia Virus Vektor ausgewählt ist.

8. Zusammensetzung gemäß Anspruch 7, wobei der Vektor ein Retrovirus Vektor oder ein Adenovirus Vektor ist.

9. Zusammensetzung gemäß einem der Ansprüche 1 bis 8 zur therapeutischen Verwendung.

10. Zusammensetzung, wie in Anspruch 6 beansprucht, zur therapeutischen Verwendung.

11. Zusammensetzung, wie in Anspruch 6 beansprucht, und ein pharmazeutisch akzeptabler Exzipient.

12. Zusammensetzung gemäß einem der Ansprüche 1 bis 11, wobei die Zusammensetzung zur Verwendung in einer Methode zur Hemmung der Angiogenese in einem Menschen oder einem Tier ist.

13. Zusammensetzung gemäß einem der Ansprüche 1 bis 12, wobei die Zusammensetzung zur Verwendung in einer Methode zur Behandlung eines durch Angiogenese vermittelten Prozesses oder einer durch Angiogenese vermittelten Krankheit ist.

14. Zusammensetzung gemäß Anspruch 13, wobei der Prozess oder die Krankheit aus der Gruppe bestehend aus Hämangiom, festen Tumoren, Tumoren im Blut, Leukämie, Metastase, Telangiectasia, Psoriasis, Sklerodermie, Pyogenem Granulom, myokardialer Angiogenese, Crohn'scher Krankheit, Plaque Neovaskularisierung, koronaren Kollateralen, zerebralen Kollateralen, arteriovenösen Missbildungen, ischämischer Angiogenese der Extremitäten, kornealen Krankheiten, Rubeose, neovaskulärem Glaukom, diabetischer Retinopathie, retrolentaler Fibroplasie, Arthritis, diabetischer Neovaskularisierung, Macula Degeneration, Wundheilung, Magengeschwür, Heliobacter-zugehörigen Krankheiten, Frakturen, Keloiden, Vaskulogenese, Hämatopoese, Ovulation, Menstruation, Plazentation, und Katzenkratzfieber ausgewählt ist.

15. Zusammensetzung gemäß einem der Ansprüche 1 bis 14, wobei die Zusammensetzung zur Verwendung in einer Methode zur Behandlung oder zur Unterdrückung des Wachstums eines Tumors in einem Menschen oder einem Tier ist.

## Revendications

1. Composition comprenant un vecteur comprenant une séquence de nucléotides codant pour une protéine appelée angiostatine, la protéine ayant une masse molaire, déterminée par électrophorèse en gel de polyacrylamide en conditions réductrices, d'entre environ 38 kilodaltons et 45 kilodaltons, et ayant une séquence d'aminoacides commençant à peu près au niveau de l'aminoacide 98 de la molécule de plasminogène, ou ayant une séquence d'aminoacides des domaines kringle 1 à 3 et d'une partie du domaine kringle 4 du plasminogène, la protéine ayant une activité antiangiogénique, où le vecteur est un vecteur viral ou un vecteur non viral et est capable d'exprimer l'angiostatine lorsqu'il est présent dans une cellule.

2. Composition selon la revendication 1, dans laquelle l'angiostatine codée ayant une activité antiangiogénique a une homologie d'au moins 70 % avec la séquence de SEQ ID NO: 2.

3. Composition selon la revendication 1 ou 2, dans laquelle le vecteur est un plasmide.

4. Composition selon l'une quelconque des revendications 1 à 3, dans laquelle la protéine est un fragment de plasminogène humain, de plasminogène murin, de plasminogène bovin, de plasminogène de Rhésus ou de plasminogène porcin.

5. Composition selon l'une quelconque des revendications 1 à 4, dans laquelle les 339 premiers aminoacides de la séquence d'aminoacides de la protéine sont choisis dans le groupe constitué par SEQ ID N° 2, SEQ ID N° 3, SEQ ID N° 4, SEQ ID N° 5 et SEQ ID N° 6.

6. Composition comprenant un vecteur viral ou un vecteur non viral comprenant une séquence de nucléotides codant pour un fragment de plasminogène ayant une activité antiangiogénique, qui contient au moins les domaines kringle 1 à 3 du plasminogène, le fragment de plasminogène étant un fragment de plasminogène humain pouvant être obtenu par digestion du plasminogène avec une élastase.

7. Composition selon l'une quelconque des revendications 1 ou 2 ou des revendications 4 à 6, dans laquelle le vecteur est un vecteur viral choisi dans le groupe constitué par un vecteur de rétrovirus, un vecteur de poliovirus, un vecteur de virus Sindbis, un vecteur d'adénovirus, un vecteur de virus adéno-associé, un vecteur de virus de l'herpès, un vecteur de SV40 ou un vecteur du virus de la vaccine.

8. Composition selon la revendication 7, dans laquelle le vecteur est un vecteur de rétrovirus ou un vecteur d'adénovirus.

9. Composition selon l'une quelconque des revendications 1 à 8, la composition étant destinée à une utilisation thérapeutique.

10. Composition selon la revendication 6, destinée à une utilisation thérapeutique.

11. Composition selon la revendication 6 avec un excipient pharmaceutiquement acceptable.

12. Composition selon l'une quelconque des revendications 1 à 11, la composition étant destinée à une utilisation dans un procédé d'inhibition de l'angiogenèse chez un être humain ou un animal.

13. Composition selon l'une quelconque des revendications 1 à 12, la composition étant destinée à une utilisation dans un procédé de traitement d'un processus ou d'une maladie médiés par l'angiogenèse.

14. Composition selon la revendication 13, où le processus ou la maladie sont choisis dans le groupe constitué par l'hémangiome, les tumeurs solides, les tumeurs diffusées par voie sanguine, la leucémie, les métastases, les télangiectasies, le psoriasis, la sclérodermie, le granulome pyogénique, l'angiogenèse myocardique, la maladie de Crohn, le néovascularisation de plaques, les collatérales coronariennes, les collatérales cérébrales, les malformations artérioveineuses, l'angiogenèse ischémique des membres, les maladies de la cornée, la rubéose, le glaucome néovasculaire, la rétinopathie diabétique, la fibroplasie rétrolentale, l'arthrite, la néovascularisation diabétique, la dégénérescence maculaire, la cicatrisation, l'ulcère peptique, les maladies associées à *Helicobacter,* les fractures, les chéloïdes, la vasculogenèse, l'hématopoïèse, l'ovulation, la menstruation, la placentation, et la maladie des griffes du chat.

**15.** Composition selon l'une quelconque des revendications 1 à 14, la composition étant destinée à une utilisation dans un procédé de traitement ou de répression de la croissance de tumeurs chez un être humain ou un animal.

# FIGURE 1

Mouse Plasminogen Sequence:

met asp his lys glu val ile leu leu phe leu leu leu leu lys
pro gly gln gly asp ser leu asp gly tyr ile ser thr gln gly
ala ser leu phe ser leu thr lys lys gln leu ala ala gly gly
val ser asp cys leu ala lys cys glu gly glu thr asp phe val
cys arg ser phe gln tyr his ser lys glu gln gln cys val ile
met ala glu asn ser lys thr ser ser ile ile arg met arg asp
val ile leu phe glu lys arg val tyr leu ser glu cys lys thr
gly ile gly asn gly tyr arg gly thr met ser arg thr lys ser
gly val ala cys gln lys trp gly ala thr phe pro his val pro
asn tyr ser pro ser thr his pro asn glu gly leu glu glu asn
tyr cys arg asn pro asp asn asp glu gln gly pro trp cys tyr
thr thr asp pro asp lys arg tyr asp tyr cys asn ile pro glu
cys glu glu glu cys met tyr cys ser gly glu lys tyr glu gly
lys ile ser lys thr met ser gly leu asp cys gln ala trp asp
ser gln ser pro his ala his gly tyr ile pro ala lys phe pro
ser lys asn leu lys met asn tyr cys his asn pro asp gly glu
pro arg pro trp cys phe thr thr asp pro thr lys arg trp glu
tyr cys asp ile pro arg cys thr thr pro pro pro pro pro ser
pro thr tyr gln cys leu lys gly arg gly glu asn tyr arg gly
thr val ser val thr val ser gly lys thr cys gln arg trp ser
glu gln thr pro his arg his asn arg thr pro glu asn phe pro
cys lys asn leu glu glu asn tyr cys arg asn pro asp gly glu
thr ala pro trp cys tyr thr thr asp ser gln leu arg trp glu
tyr cys glu ile pro ser cys glu ser ser ala ser pro asp gln
ser asp ser ser val pro pro glu glu gln thr pro val val gln
glu cys tyr gln ser asp gly gln ser tyr arg gly thr ser ser
thr thr ile thr gly lys lys cys gln ser trp ala ala met phe
pro his arg his ser lys thr pro glu asn phe pro asp ala gly
leu glu met asn tyr cys arg asn pro asp gly asp lys gly pro
trp cys tyr thr thr asp pro ser val arg trp glu tyr cys asn
leu lys arg cys ser glu thr gly gly ser val val glu leu pro
thr val ser gln glu pro ser gly pro ser asp ser glu thr asp
cys met tyr gly asn gly lys asp tyr arg gly lys thr ala val
thr ala ala gly thr pro cys gln gly trp ala ala gln glu pro
his arg his ser ile phe thr pro gln thr asn pro arg ala asp
leu glu lys asn tyr cys arg asn pro asp gly asp val asn gly
pro trp cys tyr thr thr asn pro arg lys leu tyr asp tyr cys
asp ile pro leu cys ala ser ala ser ser phe glu cys gly lys
pro gln val glu pro lys lys cys pro gly arg val val gly gly
cys val ala asn pro his ser trp pro trp gln ile ser leu arg
thr arg phe thr gly gln his phe cys gly gly thr leu ile ala
pro glu trp val leu thr ala ala his cys leu glu lys ser ser
arg pro glu phe tyr lys val ile leu gly ala his glu glu tyr
ile arg gly leu asp val gln glu ile ser val ala lys leu ile
leu glu pro asn asn arg asp ile ala leu leu lys leu ser arg
pro ala thr ile thr asp lys val ile pro ala cys leu pro ser

pro asn tyr met val ala asp arg thr ile cys tyr ile thr gly
trp gly glu thr gln gly thr phe gly ala gly arg leu lys glu
ala gln leu pro val ile glu asn lys val cys asn arg val glu
tyr leu asn asn arg val lys ser thr glu leu cys ala gly gln
leu ala gly gly val asp ser cys gln gly asp ser gly gly pro
leu val cys phe glu lys asp lys tyr ile leu gln gly val thr
ser trp gly leu gly cys ala arg pro asn lys pro gly val tyr
val arg val ser arg phe val asp trp ile glu arg glu met arg
asn asn

# FIGURE 2

MOUSE
HUMAN
RHESUS MONKEY
PORCINE
BOVINE

val tyr leu ser glu cys lys thr gly ile gly asn gly tyr arg gly
val tyr leu ser glu cys lys thr gly asn gly lys asn tyr arg gly
val tyr leu ser glu cys lys thr gly asn gly lys asn tyr arg gly
ile tyr leu ser glu cys lys thr gly asn gly lys asn tyr arg gly
ile tyr leu leu glu cys lys thr gly asn gly gln thr tyr arg gly

thr met ser arg thr lys ser gly val ala cys gln lys trp gly ala
thr met ser lys thr lys asn gly ile thr cys gln lys trp ser ser
thr met ser lys thr arg thr gly ile thr cys gln lys trp ser ser
thr thr ser lys thr lys ser gly val ile cys gln lys trp ser val
thr thr ala glu thr lys ser gly val thr cys gln lys trp ser ala

thr phe pro his val pro asn tyr ser pro ser thr his pro asn glu
thr ser pro his arg pro arg phe ser pro ala thr his pro ser glu
thr ser pro his arg pro thr phe ser pro ala thr his pro ser glu
ser ser pro his ile pro lys tyr ser pro glu lys phe pro leu ala
thr ser pro his val pro lys phe ser pro glu lys phe pro leu ala

gly leu glu glu asn tyr cys arg asn pro asp asn asp glu gln gly
gly leu glu glu asn tyr cys arg asn pro asp asn asp pro gln gly
gly leu glu glu asn tyr cys arg asn pro asp asn asp gly gln gly
gly leu glu glu asn tyr cys arg asn pro asp asn asp glu lys gly
gly leu glu glu asn tyr cys arg asn pro asp asn asp glu asn gly

pro trp cys tyr thr thr asp pro asp lys arg tyr asp tyr cys asn
pro trp cys tyr thr thr asp pro glu lys arg tyr asp tyr cys asp
pro trp cys tyr thr thr asp pro glu glu arg phe asp tyr cys asp
pro trp cys tyr thr thr asp pro glu thr arg phe asp tyr cys asp
pro trp cys tyr thr thr asp pro asp lys arg tyr asp tyr cys asp

ile pro glu cys glu glu glu cys met tyr cys ser gly glu lys tyr
ile leu glu cys glu glu glu cys met his cys ser gly glu asn tyr
ile pro glu cys glu asp glu cys met his cys ser gly glu asn tyr
ile pro glu cys glu asp glu cys met his cys ser gly glu his tyr
ile pro glu cys glu asp lys cys met his cys ser gly glu asn tyr

glu gly lys ile ser lys thr met ser gly leu asp cys gln ala trp
asp gly lys ile ser lys thr met ser gly leu glu cys gln ala trp
asp gly lys ile ser lys thr met ser gly leu glu cys gln ala trp
glu gly lys ile ser lys thr met ser gly ile glu cys gln ser trp
glu gly lys ile ala lys thr met ser gly arg asp cys gln ala trp

asp ser gln ser pro his ala his gly tyr ile pro ala lys phe pro
asp ser gln ser pro his ala his gly tyr ile pro ser lys phe pro
asp ser gln ser pro his ala his gly tyr ile pro ser lys phe pro
gly ser gln ser pro his ala his gly tyr leu pro ser lys phe pro
asp ser gln ser pro his ala his gly tyr ile pro ser lys phe pro

ser lys asn leu lys met asn tyr cys his asn pro asp gly glu pro
asn lys asn leu lys lys asn tyr cys arg asn pro asp arg glu leu
asn lys asn leu lys lys asn tyr cys arg asn pro asp gly glu pro
asn lys asn leu lys met asn tyr cys arg asn pro asp gly glu pro
asn lys asn leu lys met asn tyr cys arg asn pro asp gly glu pro

arg pro trp cys phe thr thr asp pro thr lys arg trp glu tyr cys
arg pro trp cys phe thr thr asp pro asn lys arg trp glu leu cys
arg pro trp cys phe thr thr asp pro asn lys arg trp glu leu cys
arg pro trp cys phe thr thr asp pro asn lys arg trp glu phe cys
arg pro trp cys phe thr thr asp pro gln lys arg trp glu phe cys

asp ile pro arg cys thr thr pro pro pro pro pro ser pro thr tyr
asp ile pro arg cys thr thr pro pro pro ser ser gly pro thr tyr
asp ile pro arg cys thr thr pro pro pro ser ser gly pro thr tyr
asp ile pro arg cys thr thr pro pro pro thr ser gly pro thr tyr
asp ile pro arg cys thr thr pro pro pro ser ser gly pro lys tyr

gln cys leu lys gly arg gly glu asn tyr arg gly thr val ser val
gln cys leu lys gly thr gly glu asn tyr arg gly asn val ala val
gln cys leu lys gly thr gly glu asn tyr arg gly asp val ala val
gln cys leu lys gly arg gly glu asn tyr arg gly thr val ser val
gln cys leu lys gly thr gly lys asn tyr gly gly thr val ala val

thr val ser gly lys thr cys gln arg trp ser glu gln thr pro his
thr val ser gly his thr cys gln his trp ser ala gln thr pro his
thr val ser gly his thr cys his gly trp ser ala gln thr pro his
thr ala ser gly his thr cys gln arg trp ser ala gln ser pro his
thr glu ser gly his thr cys gln arg trp ser glu gln thr pro his

arg his asn arg thr pro glu asn phe pro cys lys asn leu glu glu
thr his asn arg thr pro glu asn phe pro cys lys asn leu asp glu
thr his asn arg thr pro glu asn phe pro cys lys asn leu asp glu
lys his asn arg thr pro glu asn phe pro cys lys asn leu glu glu
lys his asn arg thr pro glu asn phe pro cys lys asn leu glu glu

asn tyr cys arg asn pro asp gly glu thr ala pro trp cys tyr thr
asn tyr cys arg asn pro asp gly lys arg ala pro trp cys his thr
asn tyr cys arg asn pro asp gly glu lys ala pro trp cys tyr thr
asn tyr cys arg asn pro asp gly glu thr ala pro trp cys tyr thr
asn tyr cys arg asn pro asp gly glu lys ala pro trp cys tyr thr

thr asp ser gln leu arg trp glu tyr cys glu ile pro ser cys glu
thr asn ser gln val arg trp glu tyr cys lys ile pro ser cys asp
thr asn ser gln val arg trp glu tyr cys lys ile pro ser cys glu
thr asp ser glu val arg trp asp tyr cys lys ile pro ser cys gly
thr asn ser glu val arg trp glu tyr cys thr ile pro ser cys glu

ser ser ala ser pro asp gln ser asp ser ser val pro pro glu glu
ser ser pro val ser thr glu gln leu ala pro thr ala pro pro glu
ser ser pro val ser thr glu pro leu asp pro thr ala pro pro glu
ser ser thr thr ser thr glu his leu asp ala pro val pro pro glu
ser ser pro leu ser thr glu arg met asp val pro val pro pro glu

gln thr pro val val gln glu cys tyr gln ser asp gly gln ser tyr
leu thr pro val val gln asp cys tyr his gly asp gly gln ser tyr
leu thr pro val val gln glu cys tyr his gly asp gly gln ser tyr
gln thr pro val ala gln asp cys tyr arg gly asn gly glu ser tyr
gln thr pro val pro gln asp cys tyr his gly asn gly gln ser tyr

arg gly thr ser ser thr thr ile thr gly lys lys cys gln ser trp
arg gly thr ser ser thr thr thr thr gly lys lys cys gln ser trp
arg gly thr ser ser thr thr thr thr gly lys lys cys gln ser trp
arg gly thr ser ser thr thr ile thr gly arg lys cys gln ser trp
arg gly thr ser ser thr thr ile thr gly arg lys cys gln ser trp

ala ala met phe pro his arg his ser lys thr pro glu asn phe pro
ser ser met thr pro his arg his gln lys thr pro glu asn tyr pro
ser ser met thr pro his trp his glu lys thr pro glu asn phe pro
val ser met thr pro his arg his glu lys thr pro gly asn phe pro
ser ser met thr pro his arg his leu lys thr pro glu asn tyr pro

asp ala gly leu glu met asn tyr cys arg asn pro asp gly asp lys
asn ala gly leu thr met asn tyr cys arg asn pro asp ala asp lys
asn ala gly leu thr met asn tyr cys arg asn pro asp ala asp lys
asn ala gly leu thr met asn tyr cys arg asn pro asp ala asp lys
asn ala gly leu thr met asn tyr cys arg asn pro asp ala asp lys

gly pro trp
gly pro trp
gly pro trp
ser pro trp
ser pro trp

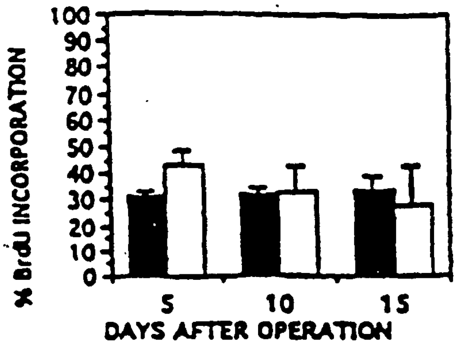


FIGURE 3A

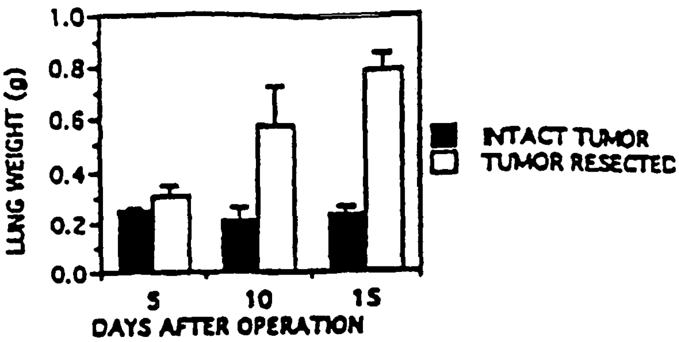


FIGURE 3B

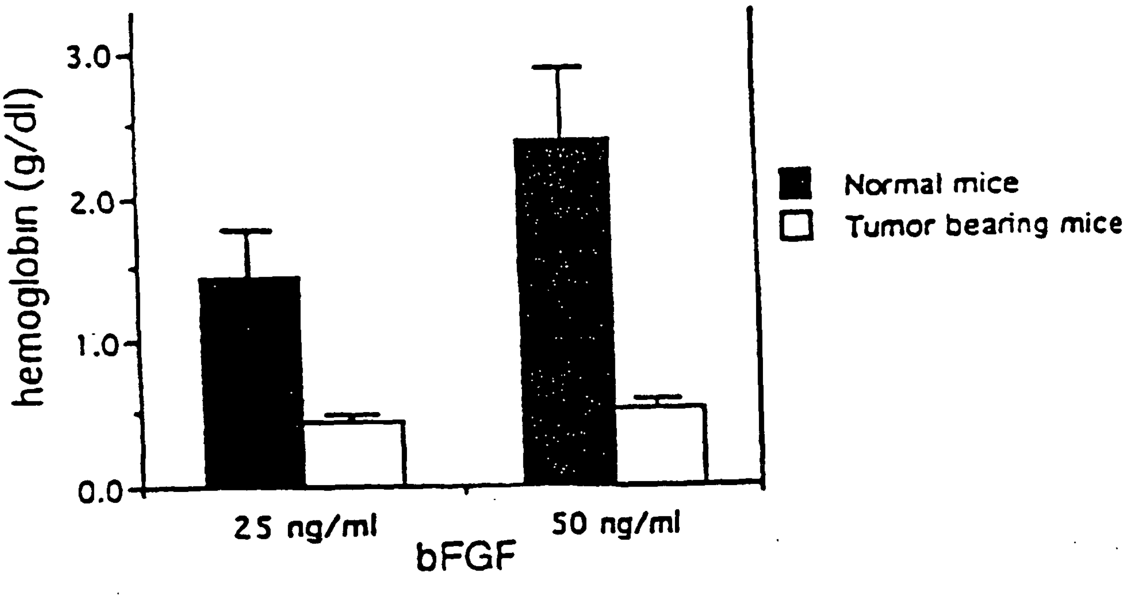


FIGURE 4

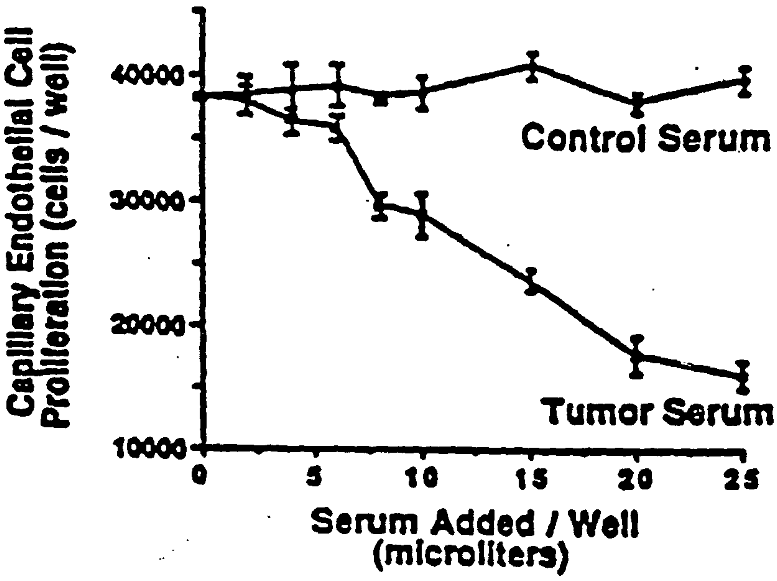


FIGURE 5

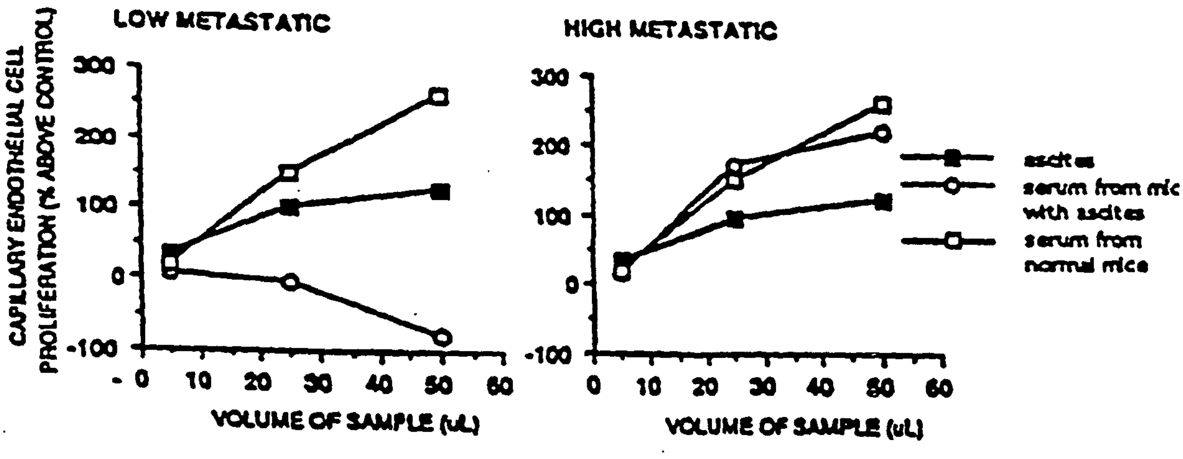


FIGURE 6A

FIGURE 6B

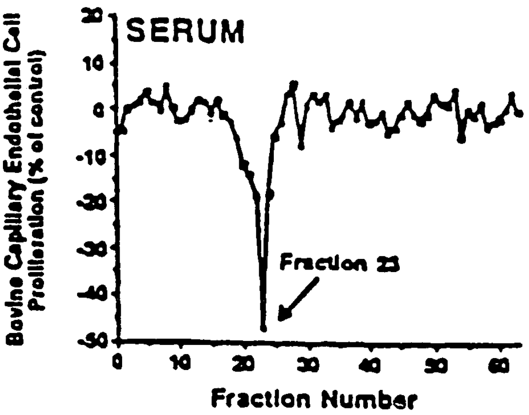


FIGURE 7A

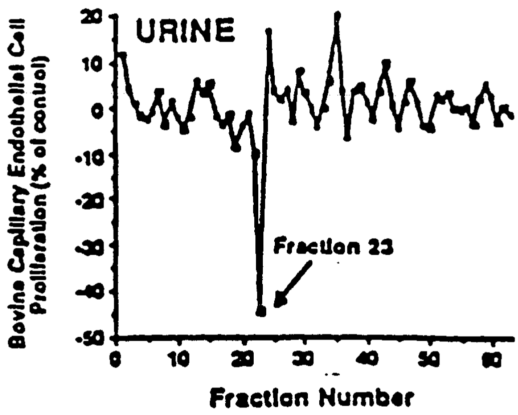


FIGURE 7B

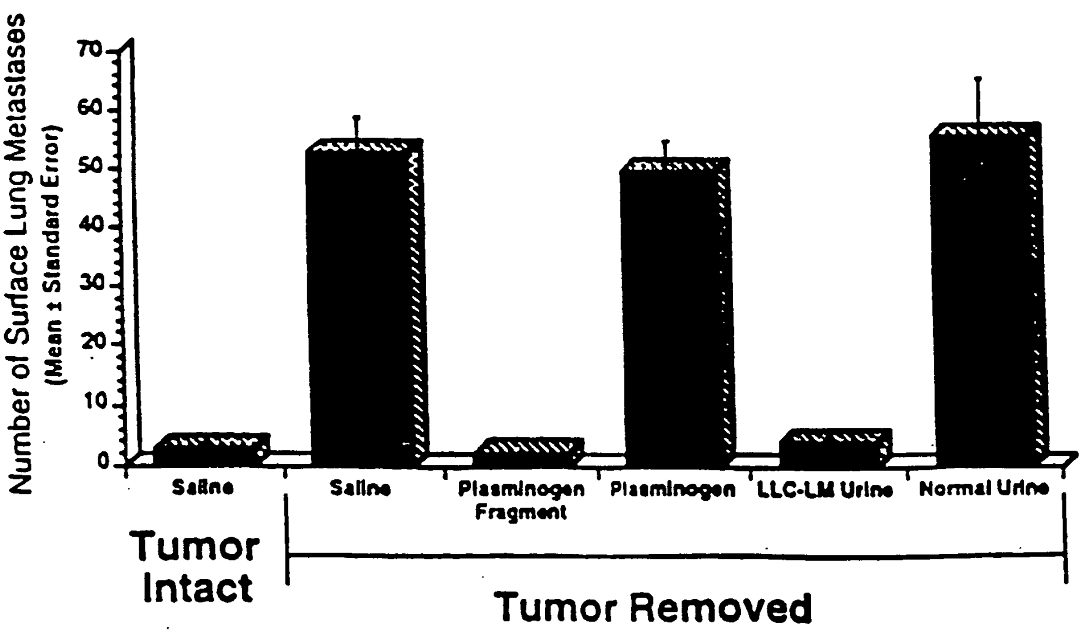


FIGURE 8

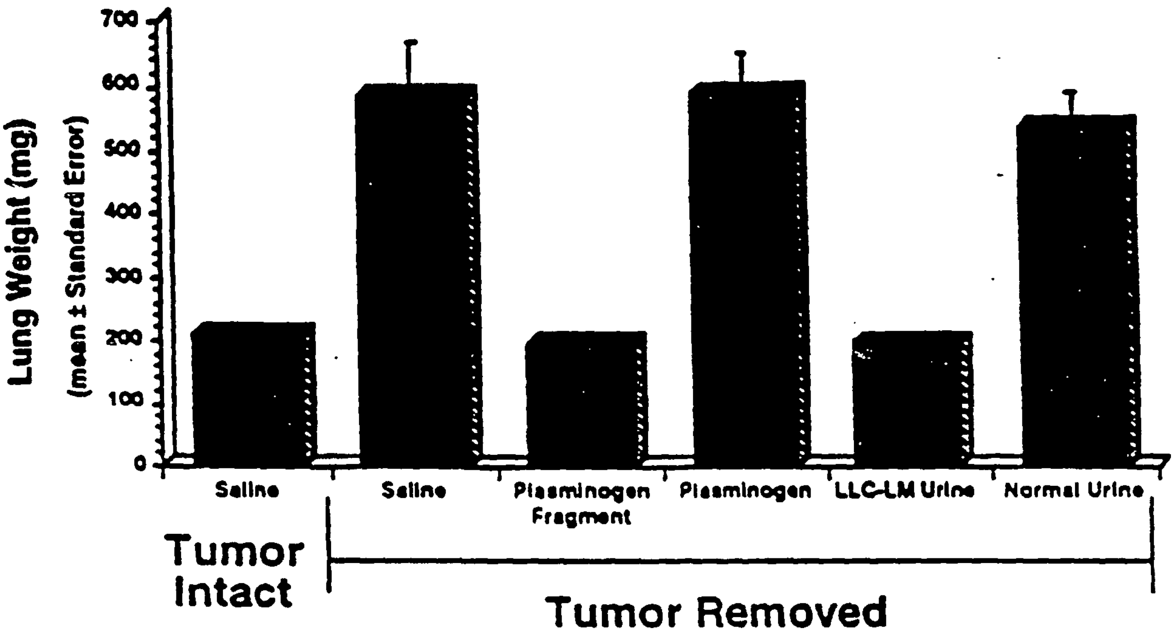
Lung Weight (mg)
(mean ± Standard Error)
700
600
500
400
300
200
100
0
Saline
Saline
Plasminogen Fragment
Plasminogen
LLC-LM Urine
Normal Urine
Tumor Intact
Tumor Removed

FIGURE 9

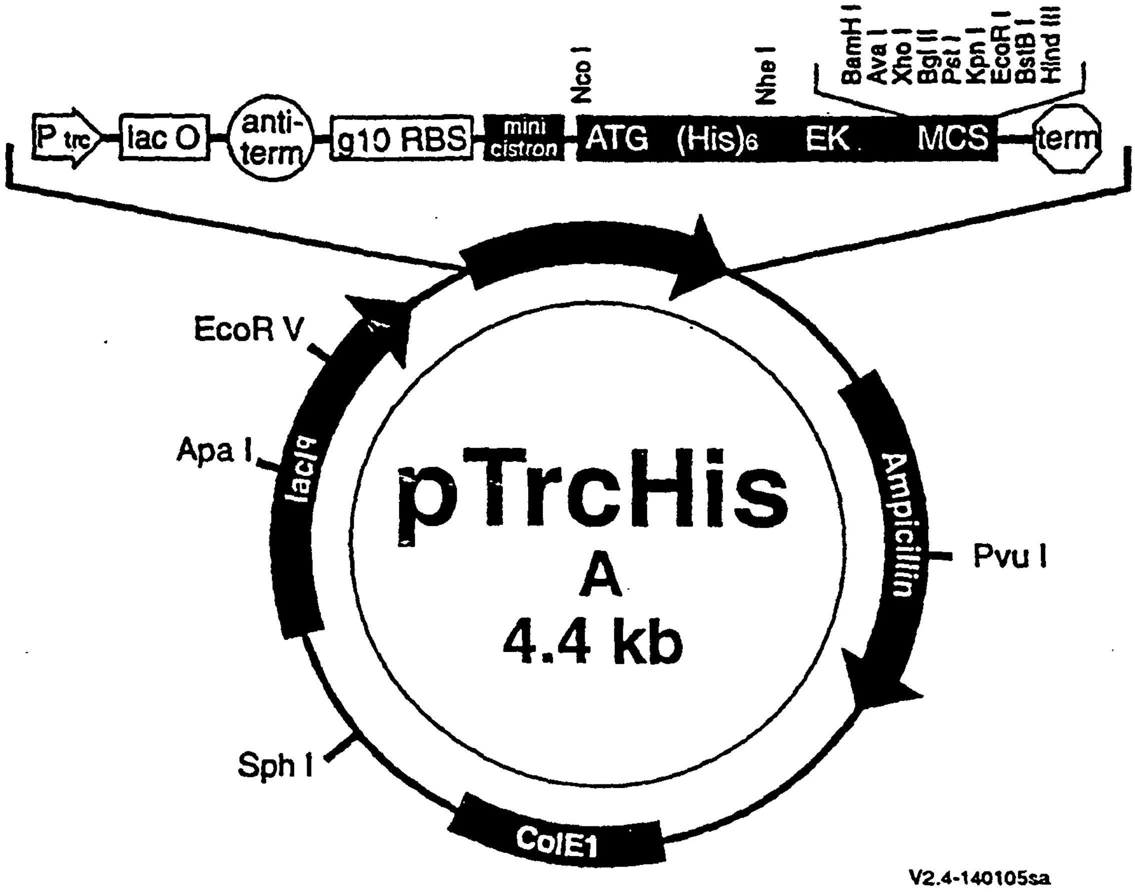
BamH I
Ava I
Xho I
Bgl II
Pst I
Kpn I
EcoR I
BstB I
Hind III
Nco I
Nhe I
P trc
lac O
anti-term
g10 RBS
mini cistron
ATG (His)6 EK MCS
term
EcoR V
Apa I
lacIq
pTrcHis A 4.4 kb
Ampicillin
Pvu I
Sph I
ColE1
V2.4-140105sa

FIGURE 10

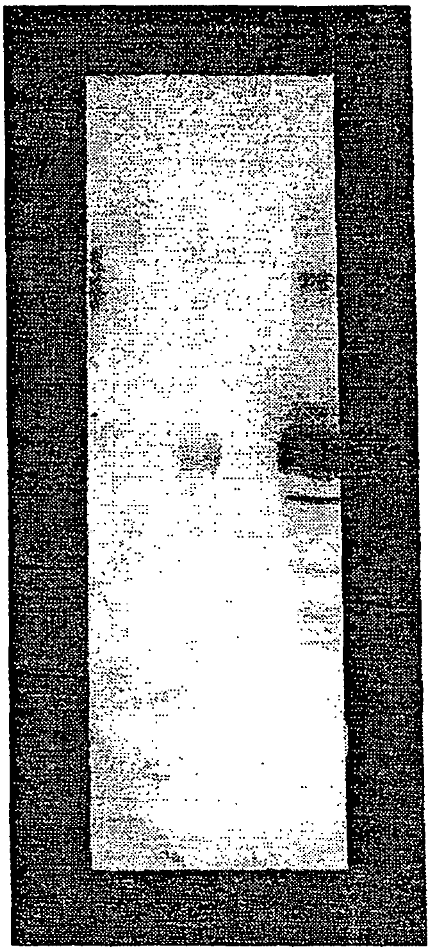

FIGURE 11

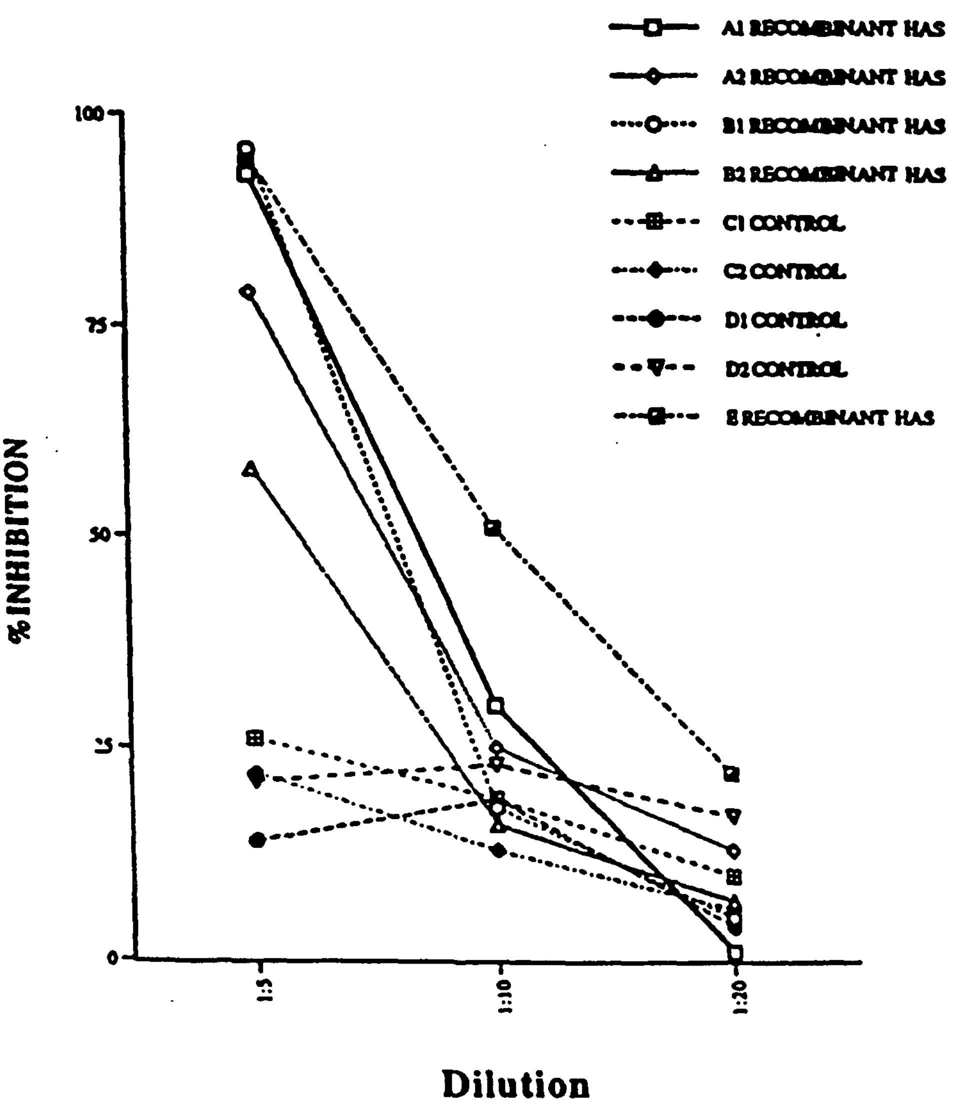
A1 RECOMBINANT HAS
A2 RECOMBINANT HAS
B1 RECOMBINANT HAS
B2 RECOMBINANT HAS
C1 CONTROL
C2 CONTROL
D1 CONTROL
D2 CONTROL
E RECOMBINANT HAS
100
75
50
25
0
% INHIBITION
1:5
1:10
1:20
Dilution

FIGURE 12

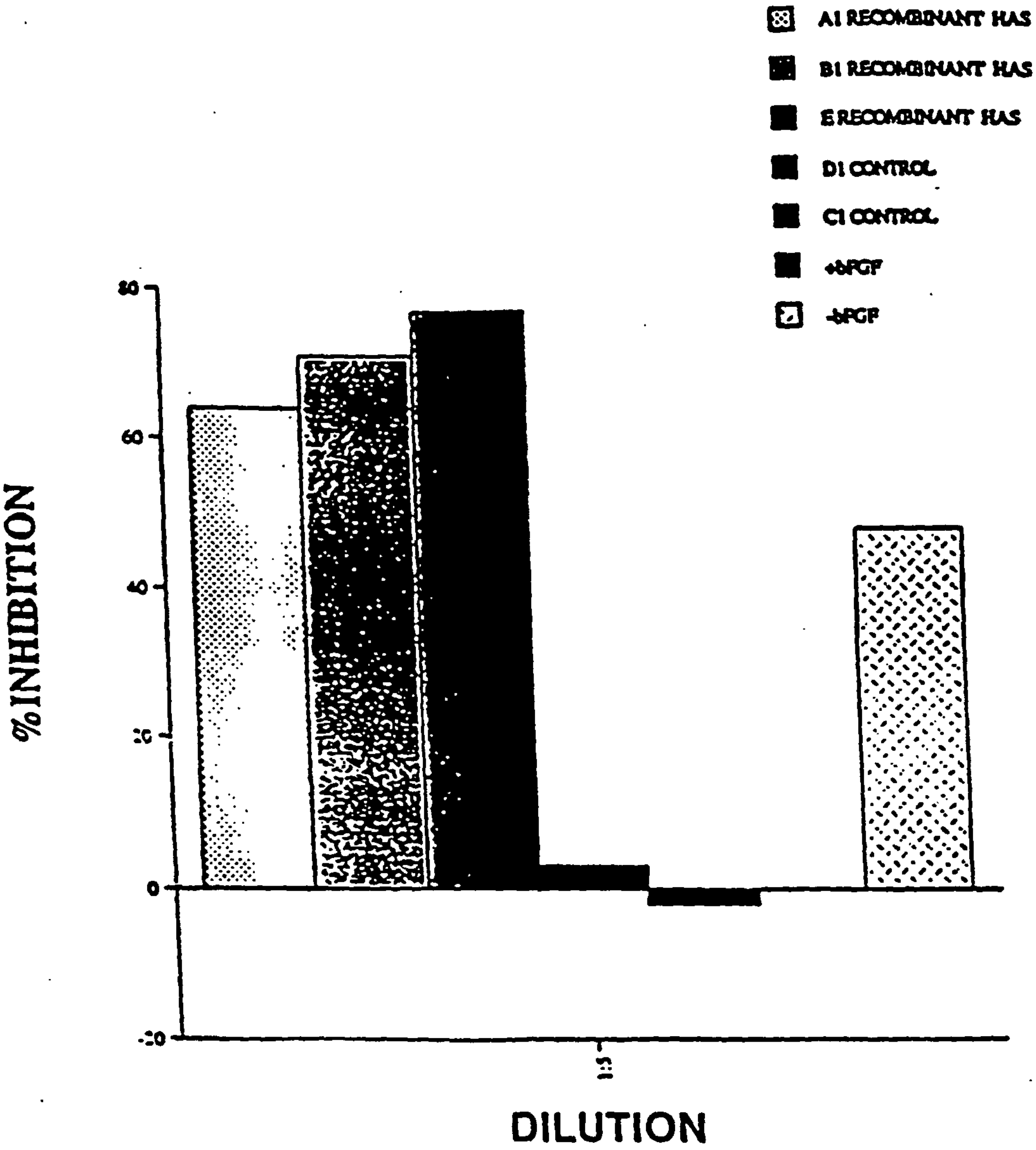
A1 RECOMBINANT HAS
B1 RECOMBINANT HAS
E RECOMBINANT HAS
D1 CONTROL
C1 CONTROL
+bFGF
-bFGF
80
60
40
20
0
-20
%INHIBITION
DILUTION

FIGURE 13

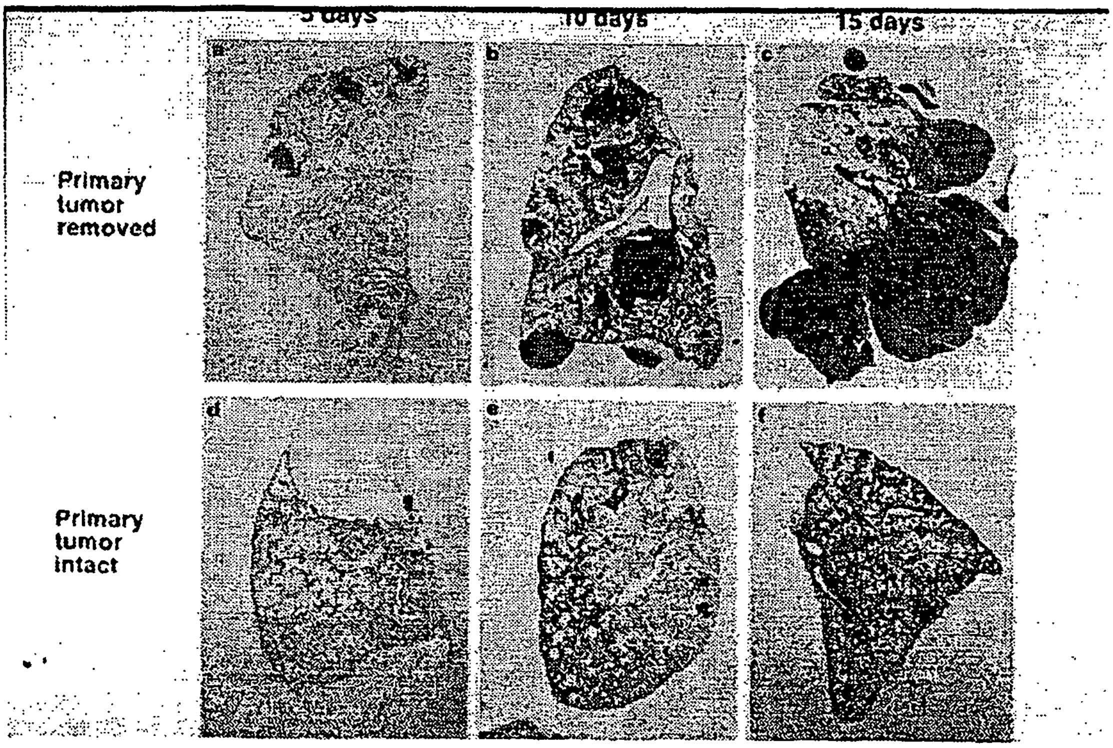
5 days
10 days
15 days
a
b
c
d
e
f
Primary tumor removed
Primary tumor intact

FIGURE 14

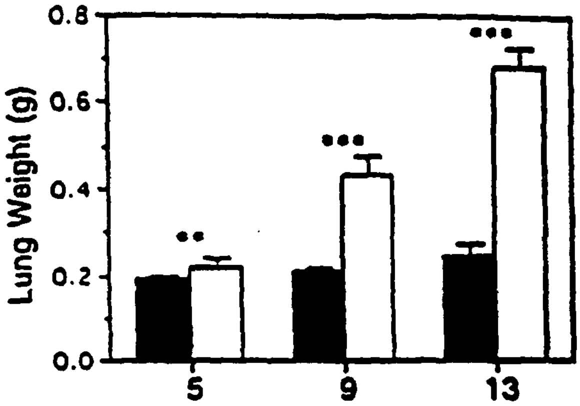


FIGURE 15A

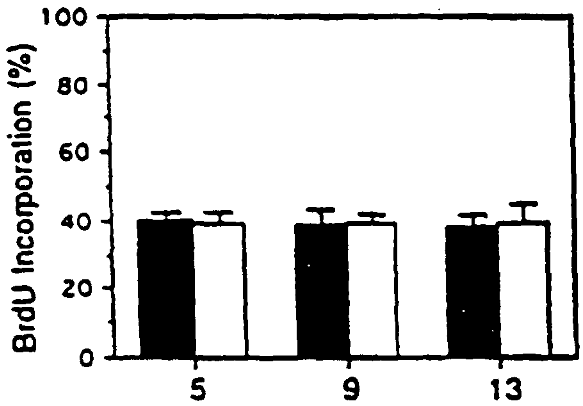


FIGURE 15B

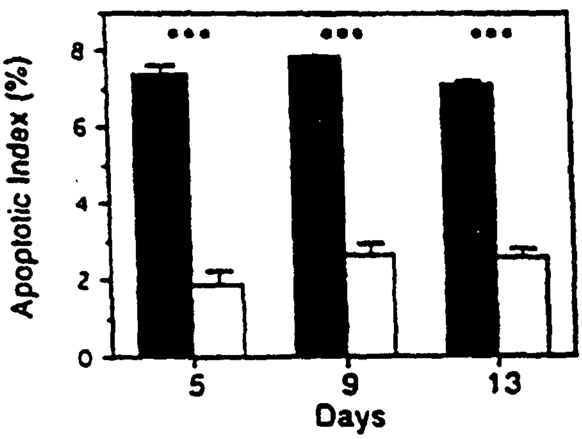


FIGURE 15C

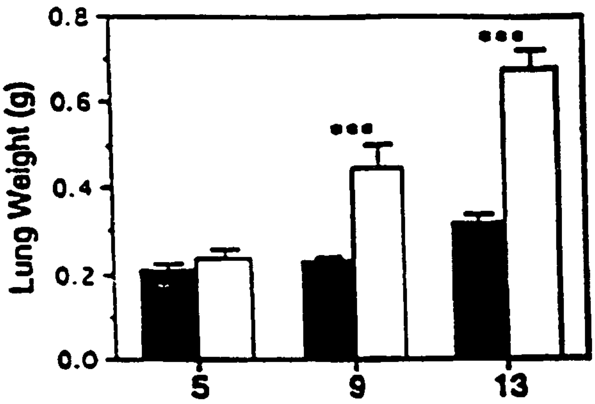


FIGURE 16A

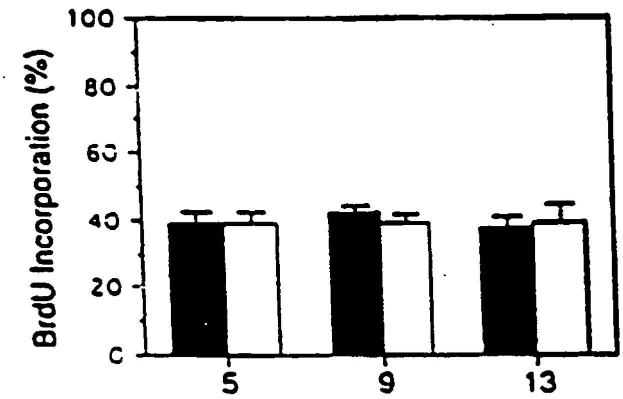


FIGURE 16B

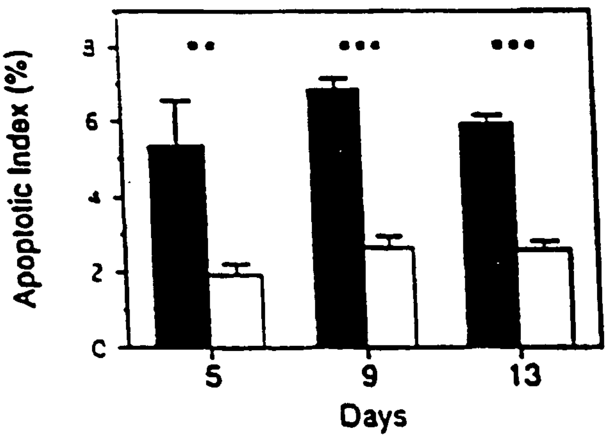


FIGURE 16C

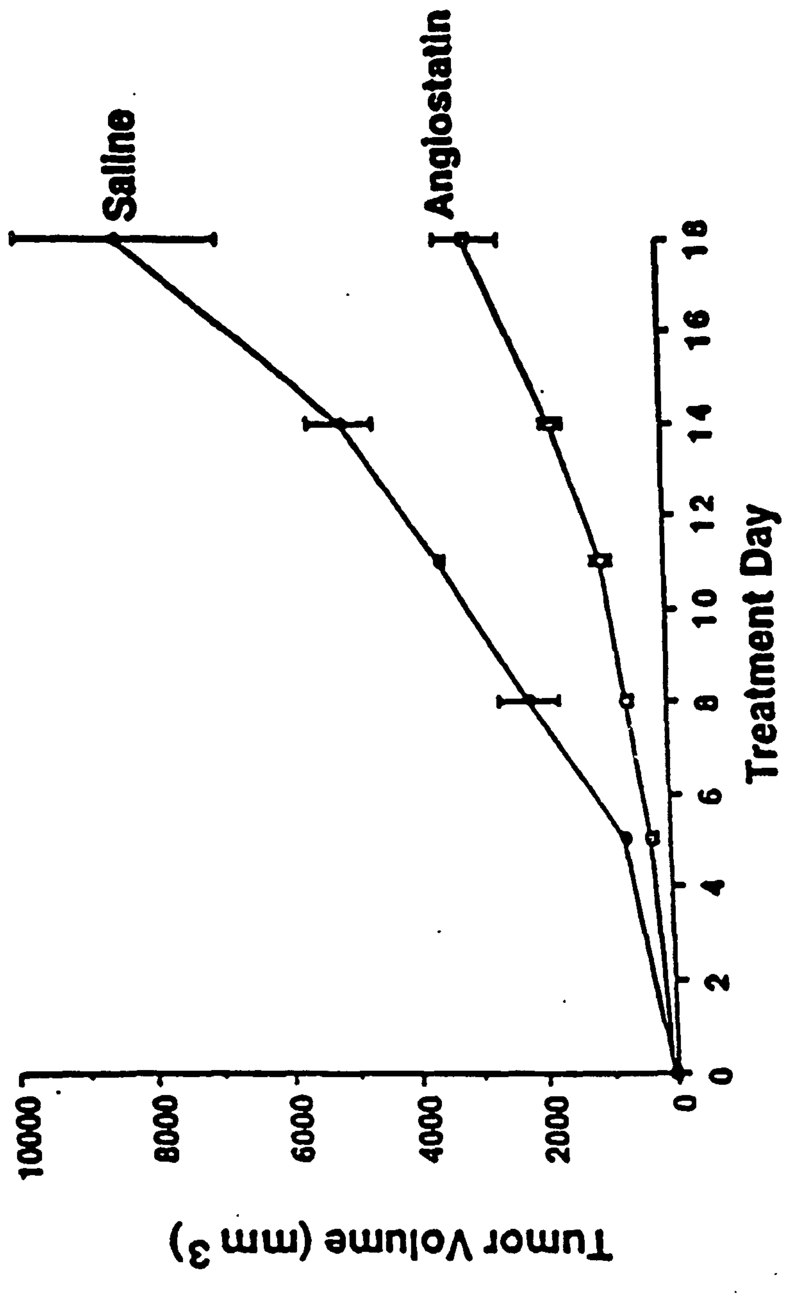
Saline
Angiostatin
Tumor Volume (mm 3)
10000
8000
6000
4000
2000
0
0
2
4
6
8
10
12
14
16
18
Treatment Day

FIGURE 17

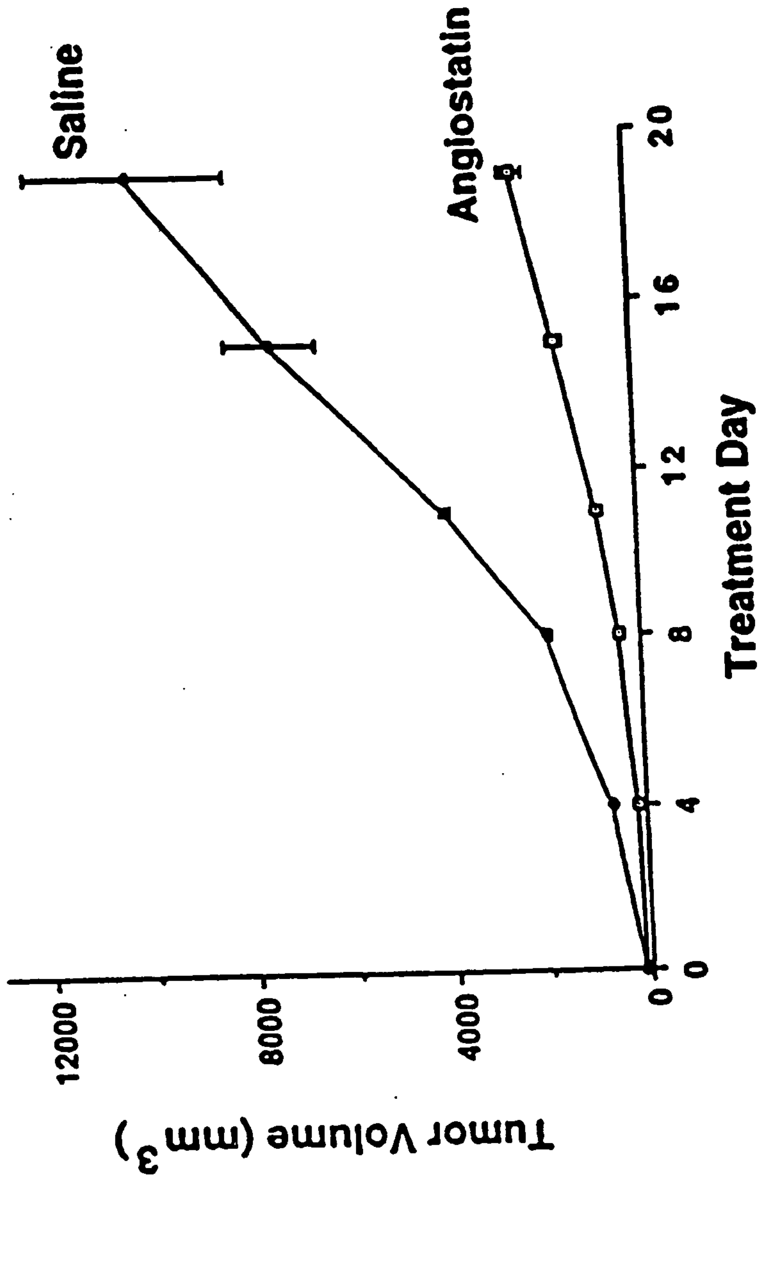
Saline
Angiostatin
Tumor Volume (mm3)
12000
8000
4000
0
0
4
8
12
16
20
Treatment Day

FIGURE 18

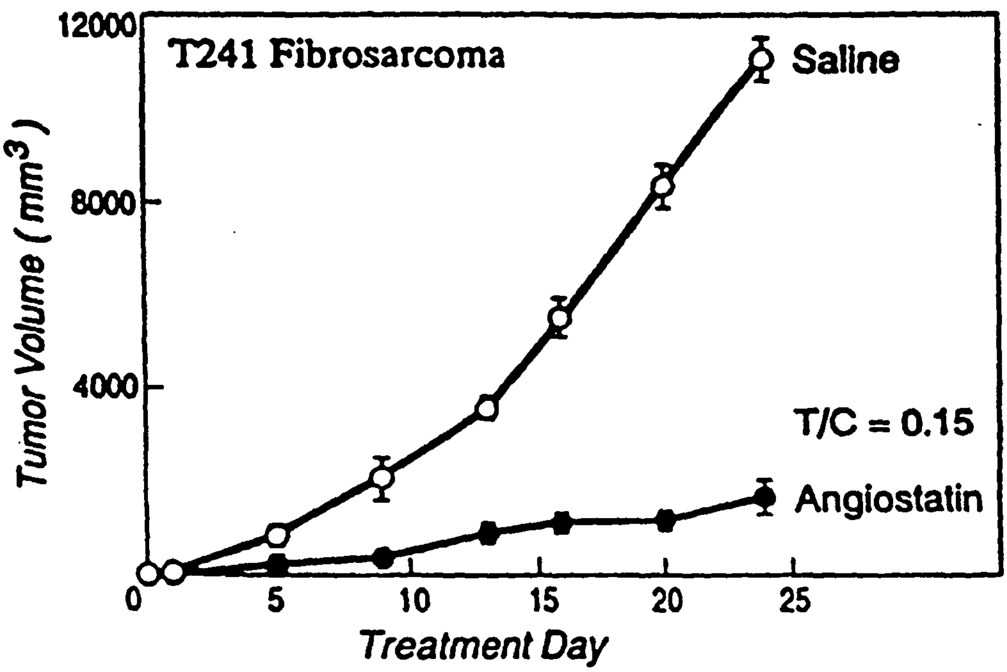
T241 Fibrosarcoma
Saline
T/C = 0.15
Angiostatin
12000
8000
4000
0
5
10
15
20
25
Tumor Volume ( mm3 )
Treatment Day

FIGURE 19

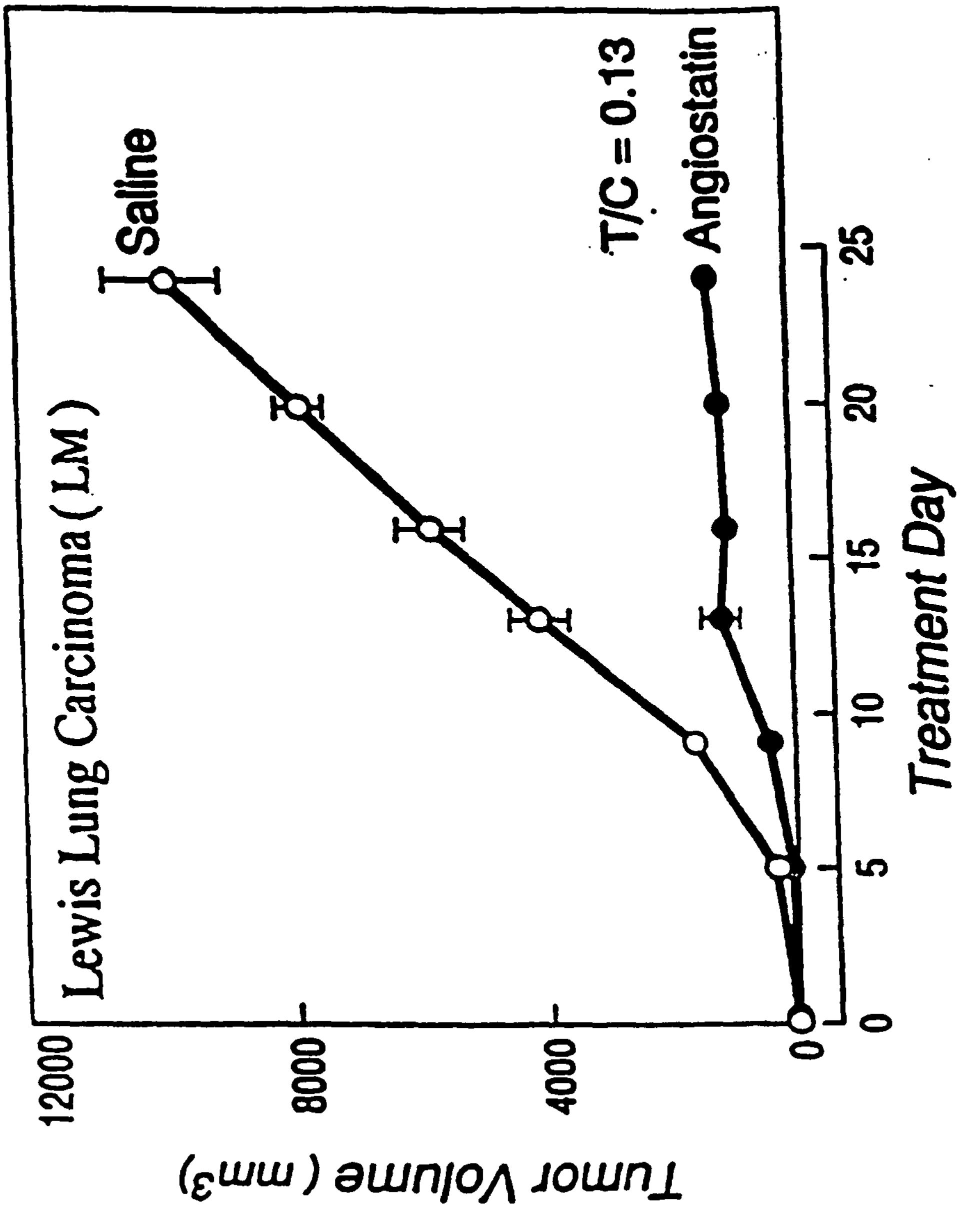
Lewis Lung Carcinoma ( LM )
Saline
T/C = 0.13
Angiostatin
Tumor Volume ( mm3)
12000
8000
4000
0
Treatment Day
0
5
10
15
20
25

FIGURE 20

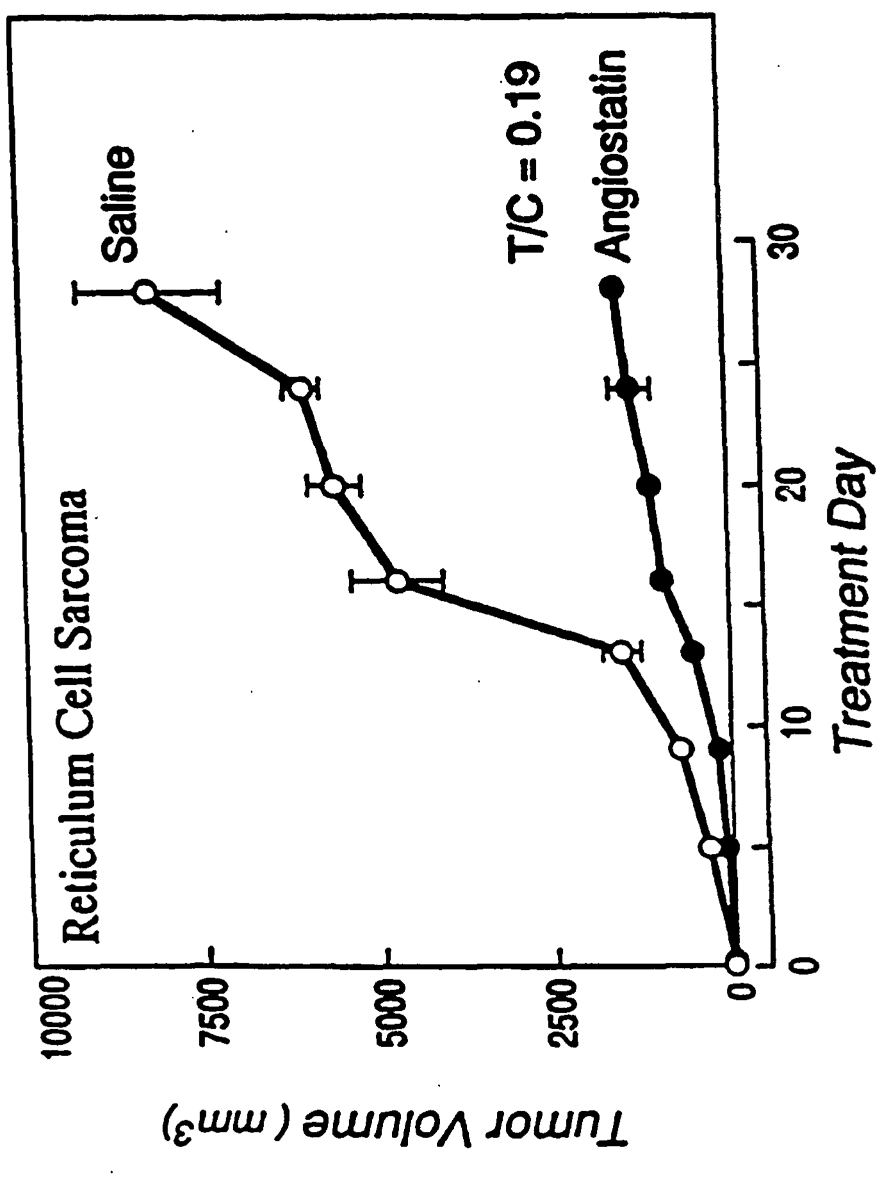
Reticulum Cell Sarcoma
Saline
T/C = 0.19
Angiostatin
Tumor Volume ( mm3)
10000
7500
5000
2500
0
0
10
20
30
Treatment Day

FIGURE 21

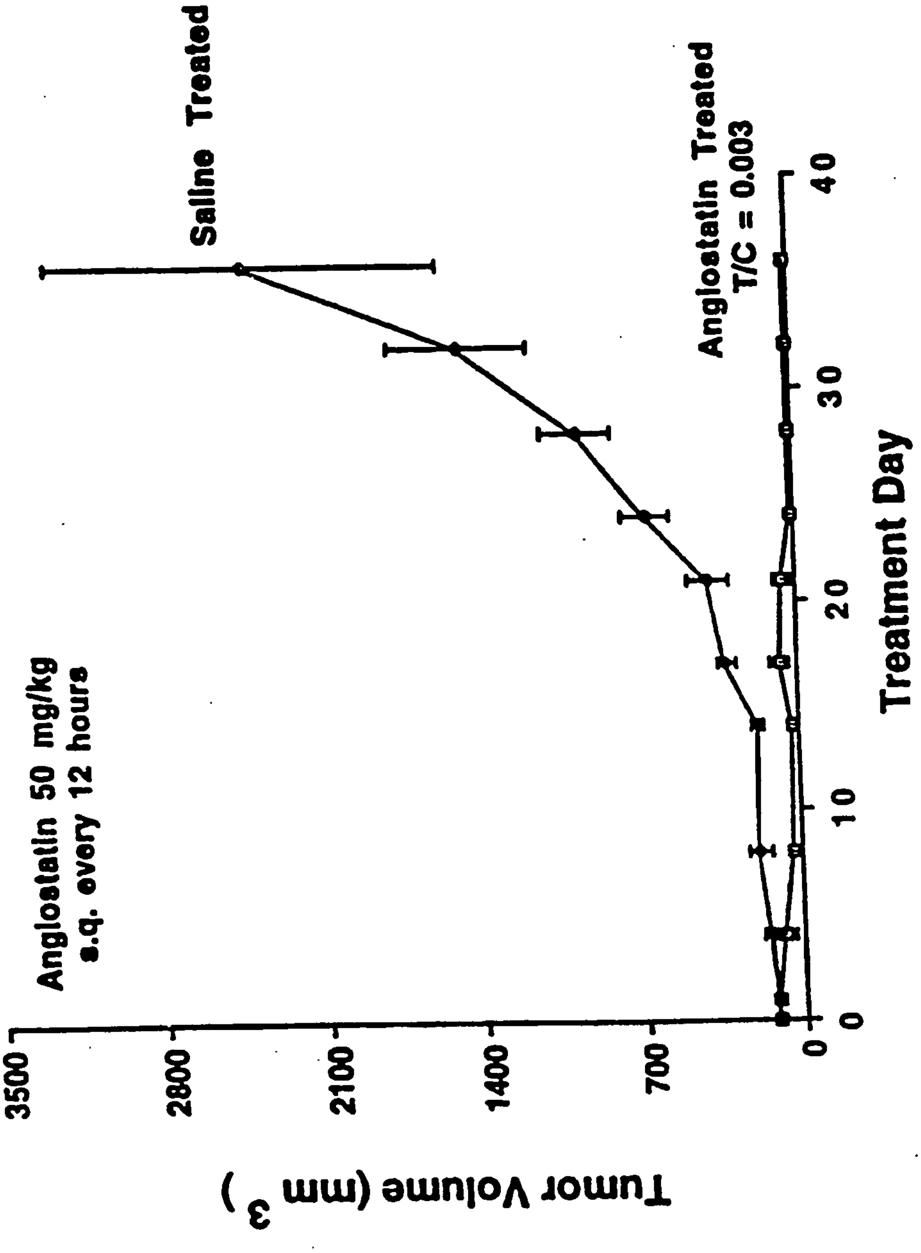
Angiostatin 50 mg/kg
s.q. every 12 hours
Saline Treated
Angiostatin Treated
T/C = 0.003
Tumor Volume (mm 3 )
3500
2800
2100
1400
700
0
0
10
20
30
40
Treatment Day

FIGURE 22

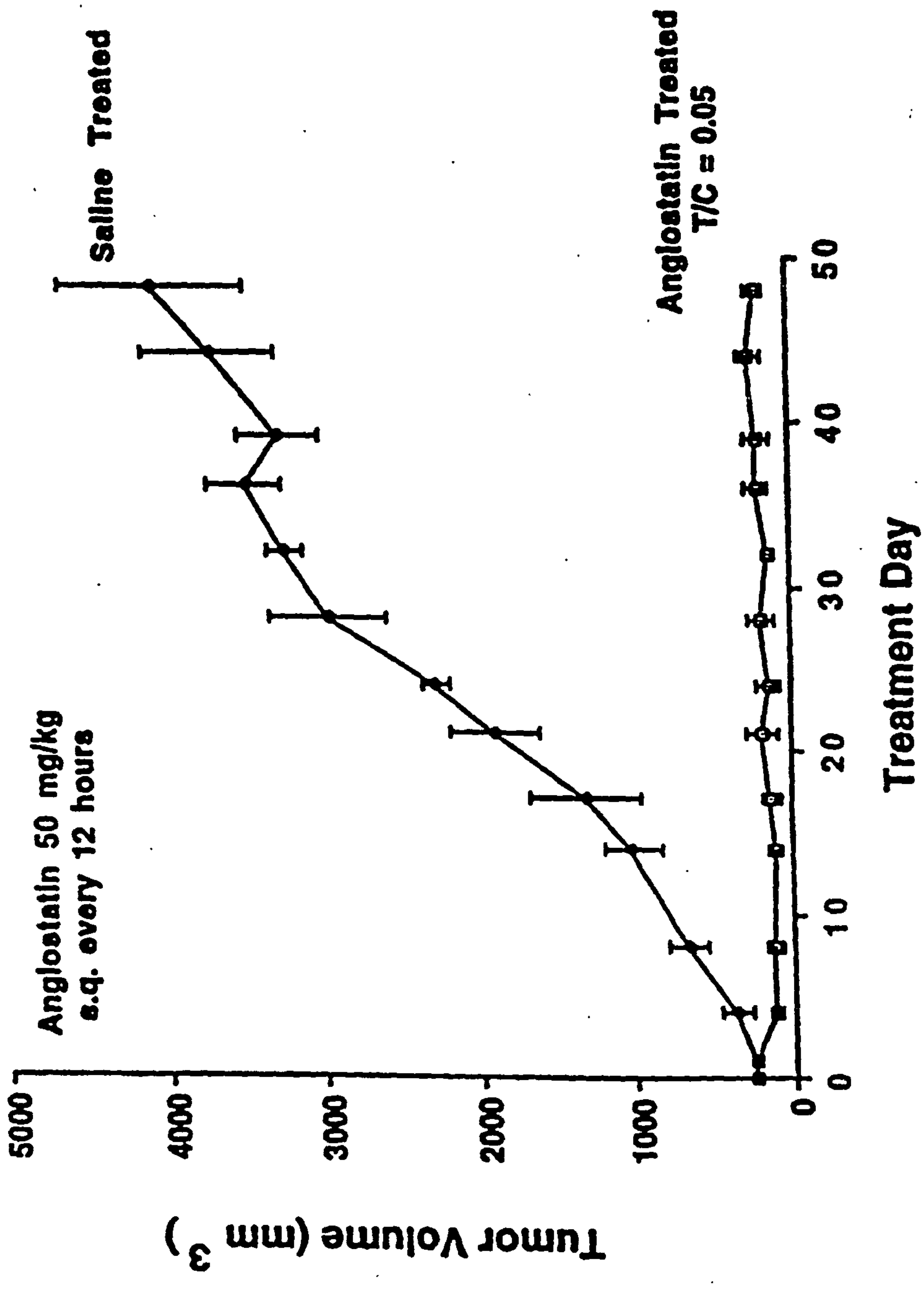
Angiostatin 50 mg/kg
s.q. every 12 hours
Saline Treated
Angiostatin Treated
T/C = 0.05
Tumor Volume (mm 3)
5000
4000
3000
2000
1000
0
0
10
20
30
40
50
Treatment Day

FIGURE 23

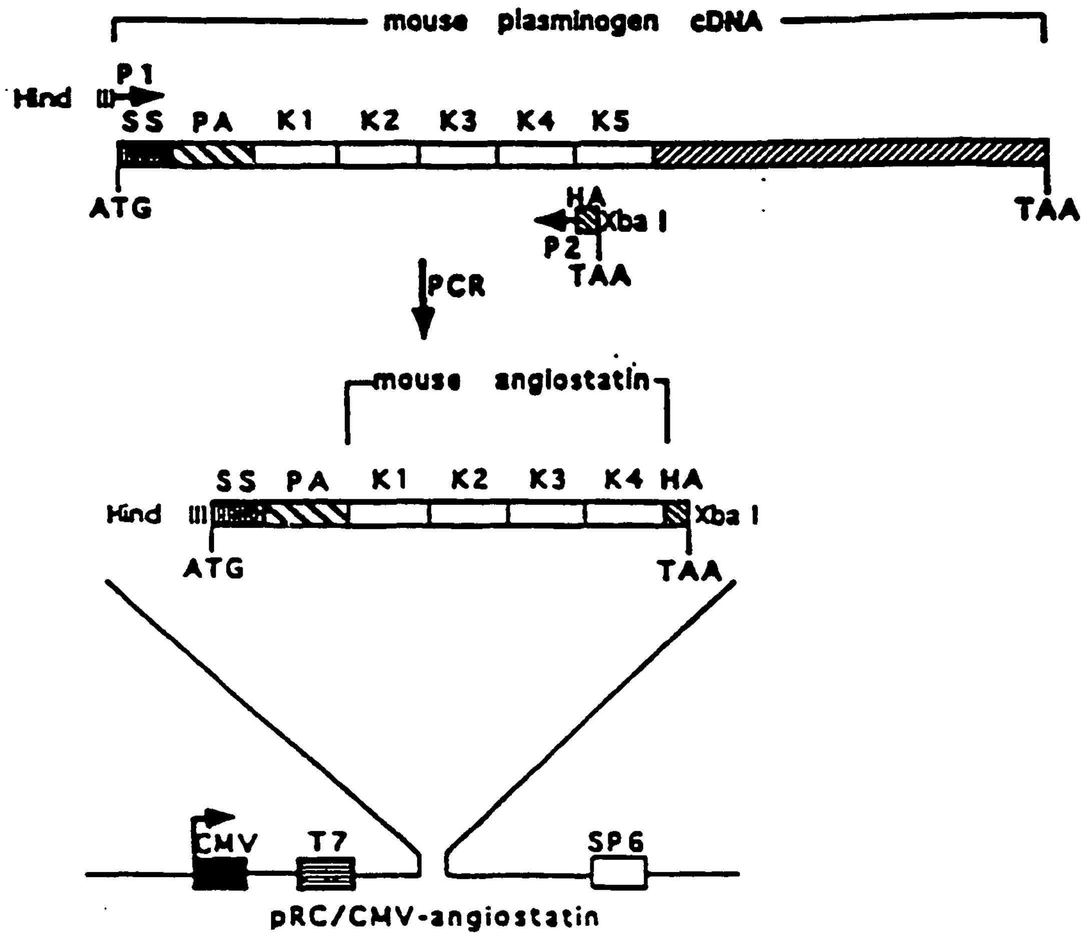
mouse plasminogen cDNA
P1
Hind III
SS PA K1 K2 K3 K4 K5
ATG
HA
Xba I
P2
TAA
TAA
PCR
mouse angiostatin
SS PA K1 K2 K3 K4 HA
Hind III
Xba I
ATG
TAA
CMV
T7
SP6
pRC/CMV-angiostatin

FIGURE 24

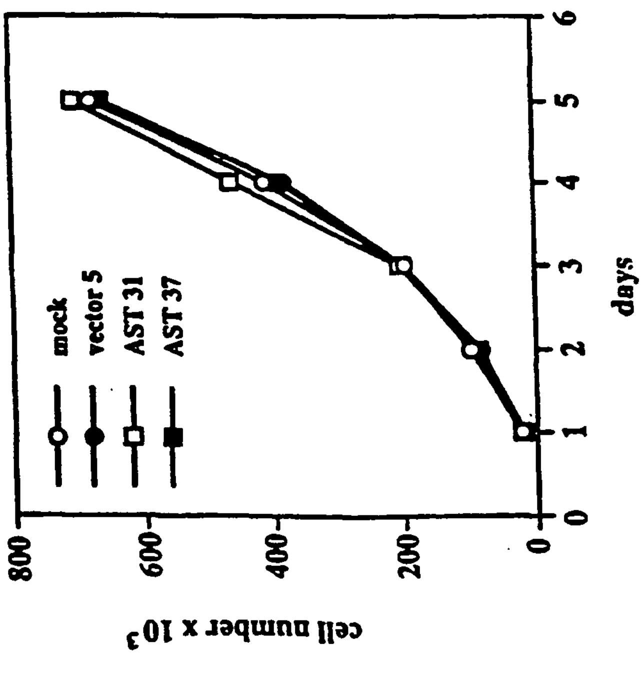


FIGURE 25A

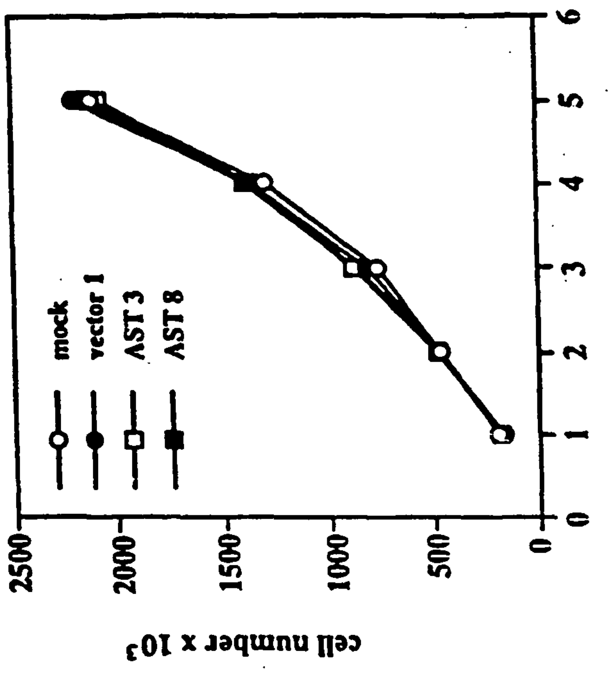


FIGURE 25B

FIGURE 26A

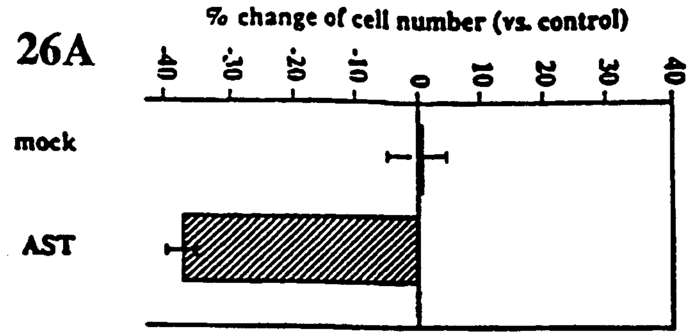


FIGURE 26B

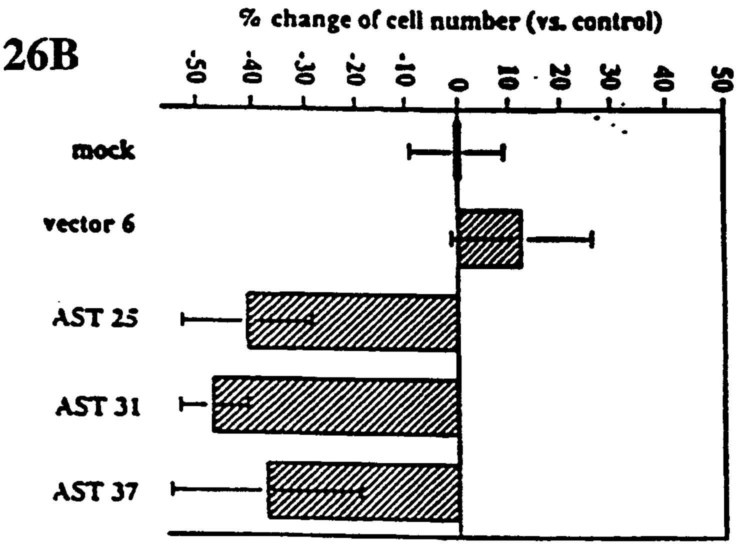


FIGURE 26C

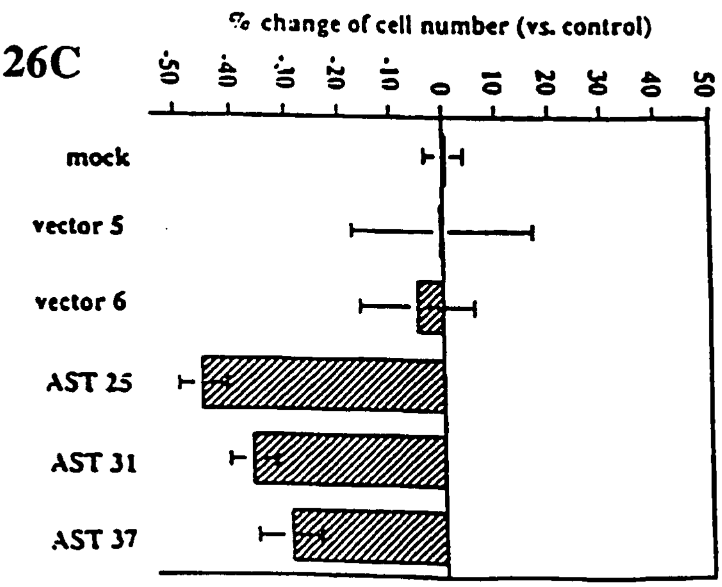

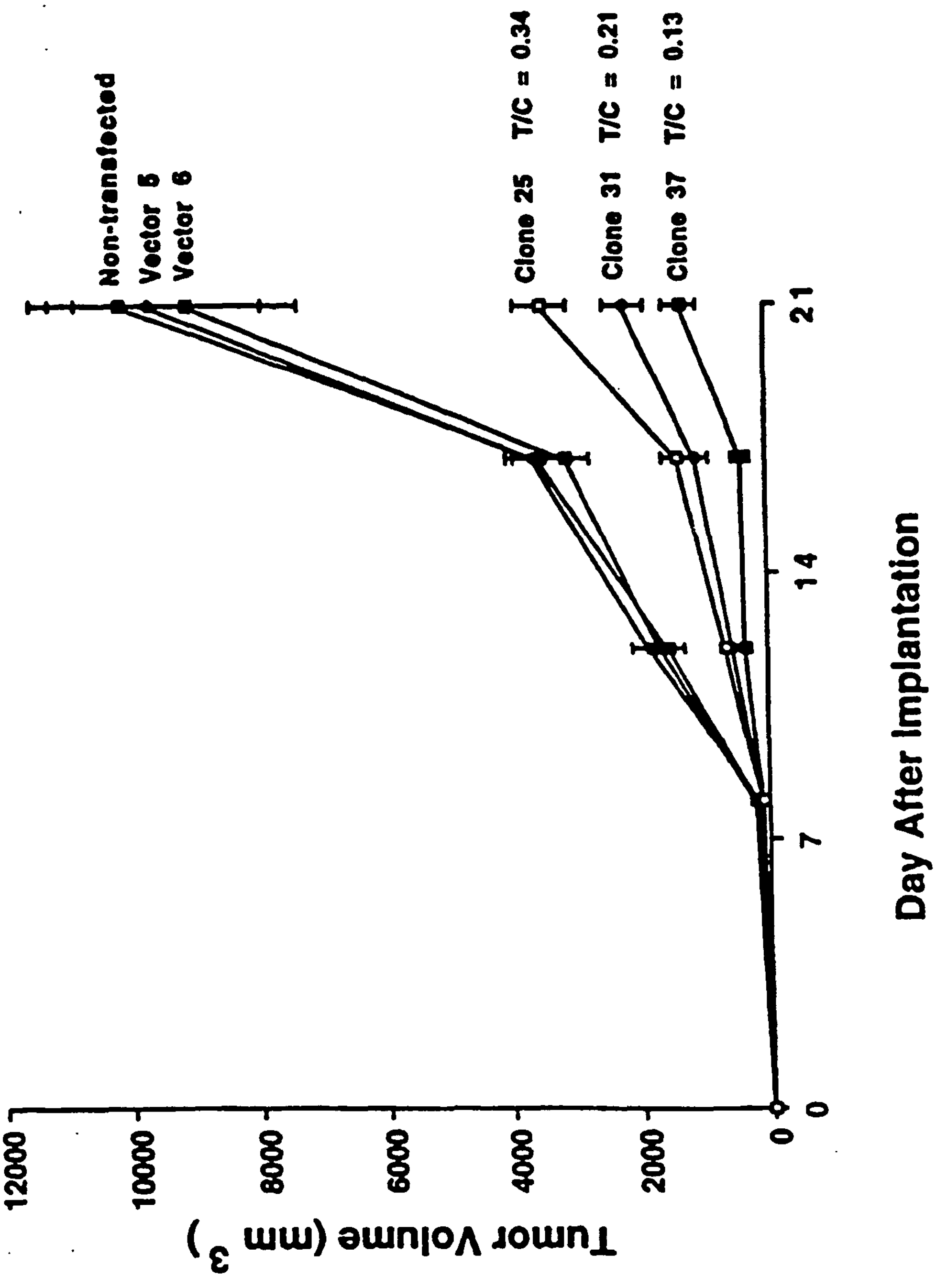
Tumor Volume (mm 3)
0
2000
4000
6000
8000
10000
12000
0
7
14
21
Day After Implantation
Non-transfected
Vector 5
Vector 6
Clone 25 T/C = 0.34
Clone 31 T/C = 0.21
Clone 37 T/C = 0.13

FIGURE 27

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **Folkman J.** Tumor angiogenesis: Therapeutic implications. *N. Engl. Jour. Med.,* 1971, vol. 285, 1182
- **Algire GH et al.** Vascular reactions of normal and malignant tumors in vivo. I. Vascular reactions of mice to wounds and to normal and neoplastic transplants. *J. Natl. Cancer Inst.,* 1945, vol. 6, 73-85
- Tumor behavior in isolated perfused organs: In vitro growth and metastasis of biopsy material in rabbit thyroid and canine intestinal segments. **Folkman J et al.** Annals of Surgery. 1966, vol. 164, 491-502
- **Gimbrone, M.A., Jr. et al.** Tumor growth and neovascularization: An experimental model using the rabbit cornea. *J. Natl. Cancer Institute,* 1974, vol. 52, 41-427
- **Gimbrone MA Jr. et al.** Tumor dormancy in vivo by prevention of neovascularization. *J. Exp. Med.,* vol. 136, 261-276
- **Knighton D.** Avascular and vascular phases of tumor growth in the chick embryo. *British J. Cancer,* 1977, vol. 35, 347-356
- **Lien W. et al.** The blood supply of experimental liver metastases. II. A microcirculatory study of normal and tumor vessels of the liver with the use of perfused silicone rubber. *Surgery,* 1970, vol. 68, 334-340
- **Folkman J et al.** Induction of angiogenesis during the transition from hyperplasia to neoplasia. *Nature,* 1989, vol. 339, 58-61
- **Kim K J et al.** Inhibition of vascular endothelial growth factor-induced angiogenesis suppresses tumor growth in vivo. *Nature,* 1993, vol. 362, 841-844
- **Hori A et al.** Suppression of solid tumor growth by immunoneutralizing monoclonal antibody against human basic fibroblast growth factor. *Cancer Research,* 1991, vol. 51, 6180-6184
- **Gross JL et al.** Modulation of solid tumor growth in vivo by bFGF. *Proc. Amer. Assoc. Canc. Res.,* 1990, vol. 31, 79
- **Ingber D et al.** Angioinhibins: Synthetic analogues of fumagillin which inhibit angiogenesis and suppress tumor growth. *Nature,* 1990, vol. 48, 555-557
- **Weidner N et al.** Tumor angiogenesis correlates with metastasis in invasive breast carcinoma. *N. Engl. J. Med.,* 1991, vol. 324, 1-8
- **Weidner N et al.** Tumor angiogenesis: A new significant and independent prognostic indicator in early-stage breast carcinoma. *J Natl. Cancer Inst.,* 1992, vol. 84, 1875-1887
- **Weidner N ; Carroll PR ; Flax J ; Blumenfeld W ; Folkman J.** Tumor angiogenesis correlates with metastasis in invasive prostate carcinoma. *American Journal of Pathology,* 1993, vol. 143 (2), 401-409
- **Srivastava A et al.** The prognostic significance of tumor vascularity in intermediate thickness (0.76-4.0 mm thick) skin melanoma. *Amer. J. Pathol.,* 1988, vol. 133, 419-423
- **Nguyen M et al.** Elevated levels of an angiogenic peptide, basic fibroblast growth factor, in urine of bladder cancer patients. *J. Natl. Cancer Inst.,* 1993, vol. 85, 241-242
- The plasminogen-plasmin enzyme system. **Robbins, K.C. et al.** Hemostasis and Thrombosis. Basic Principles and Practice. J.B. Lippincott Company, 1987, 340-357
- **Yoshimura, T et al.** Cloning, sequencing, and expression of human macrophage stimulating protein (MSP, MST1) confirms MSP as a member of the family of kringle proteins and locates the MSP gene on Chromosome 3. *J. Biol. Chem.,* 1993, vol. 268 (21), 15461-15468
- **N. Yang.** *Crit. Rev. Biotechn.,* 1992, vol. 12 (4), 335-356
- *Genetic Engineering News,* 15 April 1994
- Molecular Cloning: A Laboratory Manual. Cold Spring Harbor Press, 1989
- **Browne, M.J. et al.** Expression of recombinant human plasminogen and aglycoplasminogen in HeLa cells. *Fibrinolysis,* 1991, vol. 5 (4), 257-2601991
- **E. Atherton ; R.C. Sheppard.** *Solid Phase Peptide Synthesis: A Practical Approach*
- **Muthukkaruppan Vr. et al.** Angiogenesis in the mouse cornea. *Science,* 1979, vol. 205, 1416-1418
- **Passaniti A et al.** A simple, quantitative method for assessing angiogenesis and anti-angiogenic agents using reconstituted basement membrane, heparin and fibroblast growth factor. *Lab. Invest.,* 1992, vol. 67, 519
- Angiogenesis and its inhibitors. **Folkman J.** Important Advances in Oncology. J.B. Lippincott, 1985

- **Obeso et al.** Methods in Laboratory Investigation, A Hemangioendothelioma-derived cell line; Its use as a Model for the Study of Endothelial Cell Biology. *Lab Invest.,* 1990, vol. 63 (2), 259-269
- **Sotrrup-Jensen, L. et al.** Progress in Chemical Fibrinolysis and Thrombolysis. Raven Press, 1978, vol. 3
- **Wiman, B. et al.** *Biochemica et Biophysica Acta,* 1979, vol. 579, 142
- **Sreekrishna, K. et al.** High level expression of heterologous proteins in methylotropic yeast Pichia pastoris. *J. Basic Microbiol.,* 1988, vol. 29 (4), 265-278
- **Clare, J.J. et al.** Production of epidermal growth factor in yeast: High-level secretion using Pichia pastoris strains containing multiple gene copies. *Gene,* 1991, vol. 105, 205-212
- **Smith H.** Phytochrome transgenics: functional, ecological and biotechnical applications. *Semin. Cell. Biol.,* 1994, vol. 5 (5), 315-325

SEQUENCE LISTING

(1) GENERAL INFORMATION:

(i) APPLICANT: The Children's Medical Center Corporation
(ii) TITLE OF INVENTION: Angiostatin and Method of Use
(iii) NUMBER OF SEQUENCES: 6

(2) INFORMATION FOR SEQ ID NO:1:

(i) SEQUENCE CHARACTERISTICS:

(A) LENGTH: 812
(B) TYPE: peptide
(D) TOPOLOGY: linear

(xi) SEQUENCE DESCRIPTION: SEQ ID NO:1:

| | | | | | | | | | | | | | | |
|---|---|---|---|---|---|---|---|---|---|---|---|---|---|---|
| Met | Asp | His | Lys | Glu | Val | Ile | Leu | Leu | Phe | Leu | Leu | Leu | Leu | Lys |
| 1 | | | | 5 | | | | | 10 | | | | | 15 |
| Pro | Gly | Gln | Gly | Asp | Ser | Leu | Asp | Gly | Tyr | Ile | Ser | Thr | Gln | Gly |
| | | | | 20 | | | | | 25 | | | | | 30 |
| Ala | Ser | Leu | Phe | Ser | Leu | Thr | Lys | Lys | Gln | Leu | Ala | Ala | Gly | Gly |
| | | | | 35 | | | | | 40 | | | | | 45 |
| Val | Ser | Asp | Cys | Leu | Ala | Lys | Cys | Glu | Gly | Glu | Thr | Asp | Phe | Val |
| | | | | 50 | | | | | 55 | | | | | 60 |
| Cys | Arg | Ser | Phe | Gln | Tyr | His | Ser | Lys | Glu | Gln | Gln | Cys | Val | Ile |
| | | | | 65 | | | | | 70 | | | | | 75 |
| Met | Ala | Glu | Asn | Ser | Lys | Thr | Ser | Ser | Ile | Ile | Arg | Met | Arg | Asp |
| | | | | 80 | | | | | 85 | | | | | 90 |
| Val | Ile | Leu | Phe | Glu | Lys | Arg | Val | Tyr | Leu | Ser | Glu | Cys | Lys | Thr |
| | | | | 95 | | | | | 100 | | | | | 105 |
| Gly | Ile | Gly | Asn | Gly | Tyr | Arg | Gly | Thr | Met | Ser | Arg | Thr | Lys | Ser |
| | | | | 110 | | | | | 115 | | | | | 120 |
| Gly | Val | Ala | Cys | Gln | Lys | Trp | Gly | Ala | Thr | Phe | Pro | His | Val | Pro |
| | | | | 125 | | | | | 130 | | | | | 135 |
| Asn | Tyr | Ser | Pro | Ser | Thr | His | Pro | Asn | Glu | Gly | Leu | Glu | Glu | Asn |
| | | | | 140 | | | | | 145 | | | | | 150 |
| Tyr | Cys | Arg | Asn | Pro | Asp | Asn | Asp | Glu | Gln | Gly | Pro | Trp | Cys | Tyr |
| | | | | 155 | | | | | 160 | | | | | 165 |
| Thr | Thr | Asp | Pro | Asp | Lys | Arg | Tyr | Asp | Tyr | Cys | Asn | Ile | Pro | Glu |
| | | | | 170 | | | | | 175 | | | | | 180 |
| Cys | Glu | Glu | Glu | Cys | Met | Tyr | Cys | Ser | Gly | Glu | Lys | Tyr | Glu | Gly |
| | | | | 185 | | | | | 190 | | | | | 195 |
| Lys | Ile | Ser | Lys | Thr | Met | Ser | Gly | Leu | Asp | Cys | Gln | Ala | Trp | Asp |
| | | | | 200 | | | | | 205 | | | | | 210 |
| Ser | Gln | Ser | Pro | His | Ala | His | Gly | Tyr | Ile | Pro | Ala | Lys | Phe | Pro |

```
                    215                     220                     225
Ser Lys Asn Leu Lys Met Asn Tyr Cys His Asn Pro Asp Gly Glu
                    230                     235                     240
Pro Arg Pro Trp Cys Phe Thr Thr Asp Pro Thr Lys Arg Trp Glu
                    245                     250                     255
Tyr Cys Asp Ile Pro Arg Cys Thr Thr Pro Pro Pro Pro Pro Ser
                    260                     265                     270
Pro Thr Tyr Gln Cys Leu Lys Gly Arg Gly Glu Asn Tyr Arg Gly
                    275                     280                     285
Thr Val Ser Val Thr Val Ser Gly Lys Thr Cys Gln Arg Trp Ser
                    290                     295                     300
Glu Gln Thr Pro His Arg His Asn Arg Thr Pro Glu Asn Phe Pro
                    305                     310                     315
Cys Lys Asn Leu Glu Glu Asn Tyr Cys Arg Asn Pro Asp Gly Glu
                    320                     325                     330
Thr Ala Pro Trp Cys Tyr Thr Thr Asp Ser Gln Leu Arg Trp Glu
                    335                     340                     345
Tyr Cys Glu Ile Pro Ser Cys Glu Ser Ser Ala Ser Pro Asp Gln
                    350                     355                     360
Ser Asp Ser Ser Val Pro Pro Glu Glu Gln Thr Pro Val Val Gln
                    365                     370                     375
Glu Cys Tyr Gln Ser Asp Gly Gln Ser Tyr Arg Gly Thr Ser Ser
                    380                     385                     390
Thr Thr Ile Thr Gly Lys Lys Cys Gln Ser Trp Ala Ala Met Phe
                    395                     400                     405
Pro His Arg His Ser Lys Thr Pro Glu Asn Phe Pro Asp Ala Gly
                    410                     415                     420
Leu Glu Met Asn Tyr Cys Arg Asn Pro Asp Gly Asp Lys Gly Pro
                    425                     430                     435
Trp Cys Tyr Thr Thr Asp Pro Ser Val Arg Trp Glu Tyr Cys Asn
                    440                     445                     450
Leu Lys Arg Cys Ser Glu Thr Gly Gly Ser Val Val Glu Leu Pro
                    455                     460                     465
Thr Val Ser Gln Glu Pro Ser Gly Pro Ser Asp Ser Glu Thr Asp
                    470                     475                     480
Cys Met Tyr Gly Asn Gly Lys Asp Tyr Arg Gly Lys Thr Ala Val
                    485                     490                     495
Thr Ala Ala Gly Thr Pro Cys Gln Gly Trp Ala Ala Gln Glu Pro
```

500 505 510
His Arg His Ser Ile Phe Thr Pro Gln Thr Asn Pro Arg Ala Asp
515 520 525
Leu Glu Lys Asn Tyr Cys Arg Asn Pro Asp Gly Asp Val Asn Gly
530 535 540
Pro Trp Cys Tyr Thr Thr Asn Pro Arg Lys Leu Tyr Asp Tyr Cys
545 550 555
Asp Ile Pro Leu Cys Ala Ser Ala Ser Ser Phe Glu Cys Gly Lys
560 565 570
Pro Gln Val Glu Pro Lys Lys Cys Pro Gly Arg Val Val Gly Gly
575 580 585
Cys Val Ala Asn Pro His Ser Trp Pro Trp Gln Ile Ser Leu Arg
590 595 600
Thr Arg Phe Thr Gly Gln His Phe Cys Gly Gly Thr Leu Ile Ala
605 610 615
Pro Glu Trp Val Leu Thr Ala Ala His Cys Leu Glu Lys Ser Ser
620 625 630
Arg Pro Glu Phe Tyr Lys Val Ile Leu Gly Ala His Glu Glu Tyr
635 640 645
Ile Arg Gly Leu Asp Val Gln Glu Ile Ser Val Ala Lys Leu Ile
650 655 660
Leu Glu Pro Asn Asn Arg Asp Ile Ala Leu Leu Lys Leu Ser Arg
665 670 675
Pro Ala Thr Ile Thr Asp Lys Val Ile Pro Ala Cys Leu Pro Ser
680 685 690
Pro Asn Tyr Met Val Ala Asp Arg Thr Ile Cys Tyr Ile Thr Gly
695 700 705
Trp Gly Glu Thr Gln Gly Thr Phe Gly Ala Gly Arg Leu Lys Glu
710 715 720
Ala Gln Leu Pro Val Ile Glu Asn Lys Val Cys Asn Arg Val Glu
725 730 735
Tyr Leu Asn Asn Arg Val Lys Ser Thr Glu Leu Cys Ala Gly Gln
740 745 750
Leu Ala Gly Gly Val Asp Ser Cys Gln Gly Asp Ser Gly Gly Pro
755 760 765
Leu Val Cys Phe Glu Lys Asp Lys Tyr Ile Leu Gln Gly Val Thr
770 775 780
Ser Trp Gly Leu Gly Cys Ala Arg Pro Asn Lys Pro Gly Val Tyr

```
                    785                     790                     795
Val Arg Val Ser Arg Phe Val Asp Trp Ile Glu Arg Glu Met Arg
                    800                     805                     810
Asn Asn
```

(3) INFORMATION FOR SEQ ID NO:2:

(i) SEQUENCE CHARACTERISTICS:

(A) LENGTH: 339
(B) TYPE: peptide
(D) TOPOLOGY: linear

(xi) SEQUENCE DESCRIPTION: SEQ ID NO:2:

Val Tyr Leu Ser Glu Cys Lys Thr Gly Ile Gly Asn Gly Tyr Arg
1 5 10 15
Gly Thr Met Ser Arg Thr Lys Ser Gly Val Ala Cys Gln Lys Trp
20 25 30
Gly Ala Thr Phe Pro His Val Pro Asn Tyr Ser Pro Ser Thr His
35 40 45
Pro Asn Glu Gly Leu Glu Glu Asn Tyr Cys Arg Asn Pro Asp Asn
50 55 60
Asp Glu Gln Gly Pro Trp Cys Tyr Thr Thr Asp Pro Asp Lys Arg
65 70 75
Tyr Asp Tyr Cys Asn Ile Pro Glu Cys Glu Glu Glu Cys Met Tyr
80 85 90
Cys Ser Gly Glu Lys Tyr Glu Gly Lys Ile Ser Lys Thr Met Ser
95 100 105
Gly Leu Asp Cys Gln Ala Trp Asp Ser Gln Ser Pro His Ala His
110 115 120
Gly Tyr Ile Pro Ala Lys Phe Pro Ser Lys Asn Leu Lys Met Asn
125 130 135
Tyr Cys His Asn Pro Asp Gly Glu Pro Arg Pro Trp Cys Phe Thr
140 145 150
Thr Asp Pro Thr Lys Arg Trp Glu Tyr Cys Asp Ile Pro Arg Cys
155 160 165
Thr Thr Pro Pro Pro Pro Pro Ser Pro Thr Tyr Gln Cys Leu Lys
170 175 180
Gly Arg Gly Glu Asn Tyr Arg Gly Thr Val Ser Val Thr Val Ser
185 190 195
Gly Lys Thr Cys Gln Arg Trp Ser Glu Gln Thr Pro His Arg His

```
                200                 205                 210
Asn Arg Thr Pro Glu Asn Phe Pro Cys Lys Asn Leu Glu Glu Asn
                215                 220                 225
Tyr Cys Arg Asn Pro Asp Gly Glu Thr Ala Pro Trp Cys Tyr Thr
                230                 235                 240
Thr Asp Ser Gln Leu Arg Trp Glu Tyr Cys Glu Ile Pro Ser Cys
                245                 250                 255
Glu Ser Ser Ala Ser Pro Asp Gln Ser Asp Ser Ser Val Pro Pro
                260                 265                 270
Glu Glu Gln Thr Pro Val Val Gln Glu Cys Tyr Gln Ser Asp Gly
                275                 280                 285
Gln Ser Tyr Arg Gly Thr Ser Ser Thr Thr Ile Thr Gly Lys Lys
                290                 295                 300
Cys Gln Ser Trp Ala Ala Met Phe Pro His Arg His Ser Lys Thr
                305                 310                 315
Pro Glu Asn Phe Pro Asp Ala Gly Leu Glu Met Asn Tyr Cys Arg
                320                 325                 330
Asn Pro Asp Gly Asp Lys Gly Pro Trp
                335
```

(4) INFORMATION FOR SEQ ID NO:3:

(i) SEQUENCE CHARACTERISTICS:

(A) LENGTH: 339
(B) TYPE: peptide
(D) TOPOLOGY: linear

(xi) SEQUENCE DESCRIPTION: SEQ ID NO:3:

Val Tyr Leu Ser Glu Cys Lys Thr Gly Asn Gly Lys Asn Tyr Arg
1 5 10 15
Gly Thr Met Ser Lys Thr Lys Asn Gly Ile Thr Cys Gln Lys Trp
20 25 30
Ser Ser Thr Ser Pro His Arg Pro Arg Phe Ser Pro Ala Thr His
35 40 45
Pro Ser Glu Gly Leu Glu Glu Asn Tyr Cys Arg Asn Pro Asp Asn
50 55 60
Asp Pro Gln Gly Pro Trp Cys Tyr Thr Thr Asp Pro Glu Lys Arg
65 70 75
Tyr Asp Tyr Cys Asp Ile Leu Glu Cys Glu Glu Glu Cys Met His
80 85 90
Cys Ser Gly Glu Asn Tyr Asp Gly Lys Ile Ser Lys Thr Met Ser

```
                95                      100                     105
Gly Leu Glu Cys Gln Ala Trp Asp Ser Gln Ser Pro His Ala His
                110                     115                     120
Gly Tyr Ile Pro Ser Lys Phe Pro Asn Lys Asn Leu Lys Lys Asn
                125                     130                     135
Tyr Cys Arg Asn Pro Asp Arg Glu Leu Arg Pro Trp Cys Phe Thr
                140                     145                     150
Thr Asp Pro Asn Lys Arg Trp Glu Leu Cys Asp Ile Pro Arg Cys
                155                     160                     165
Thr Thr Pro Pro Pro Ser Ser Gly Pro Thr Tyr Gln Cys Leu Lys
                170                     175                     180
Gly Thr Gly Glu Asn Tyr Arg Gly Asn Val Ala Val Thr Val Ser
                185                     190                     195
Gly His Thr Cys Gln His Trp Ser Ala Gln Thr Pro His Thr His
                200                     205                     210
Asn Arg Thr Pro Glu Asn Phe Pro Cys Lys Asn Leu Asp Glu Asn
                215                     220                     225
Tyr Cys Arg Asn Pro Asp Gly Lys Arg Ala Pro Trp Cys His Thr
                230                     235                     240
Thr Asn Ser Gln Val Arg Trp Glu Tyr Cys Lys Ile Pro Ser Cys
                245                     250                     255
Asp Ser Ser Pro Val Ser Thr Glu Gln Leu Ala Pro Thr Ala Pro
                260                     265                     270
Pro Glu Leu Thr Pro Val Val Gln Asp Cys Tyr His Gly Asp Gly
                275                     280                     285
Gln Ser Tyr Arg Gly Thr Ser Ser Thr Thr Thr Thr Gly Lys Lys
                290                     295                     300
Cys Gln Ser Trp Ser Ser Met Thr Pro His Arg His Gln Lys Thr
                305                     310                     315
Pro Glu Asn Tyr Pro Asn Ala Gly Leu Thr Met Asn Tyr Cys Arg
                320                     325                     330
Asn Pro Asp Ala Asp Lys Gly Pro Trp
                335
```

(5) INFORMATION FOR SEQ ID NO:4:

(i) SEQUENCE CHARACTERISTICS:

(A) LENGTH: 339

(B) TYPE: peptide

(D) TOPOLOGY: linear

(xi) SEQUENCE DESCRIPTION: SEQ ID NO:4:

Val Tyr Leu Ser Glu Cys Lys Thr Gly Asn Gly Lys Asn Tyr Arg
1 5 10 15
Gly Thr Met Ser Lys Thr Arg Thr Gly Ile Thr Cys Gln Lys Trp
20 25 30
Ser Ser Thr Ser Pro His Arg Pro Thr Phe Ser Pro Ala Thr His
35 40 45
Pro Ser Glu Gly Leu Glu Glu Asn Tyr Cys Arg Asn Pro Asp Asn
50 55 60
Asp Gly Gln Gly Pro Trp Cys Tyr Thr Thr Asp Pro Glu Glu Arg
65 70 75
Phe Asp Tyr Cys Asp Ile Pro Glu Cys Glu Asp Glu Cys Met His
80 85 90
Cys Ser Gly Glu Asn Tyr Asp Gly Lys Ile Ser Lys Thr Met Ser
95 100 105
Gly Leu Glu Cys Gln Ala Trp Asp Ser Gln Ser Pro His Ala His
110 115 120
Gly Tyr Ile Pro Ser Lys Phe Pro Asn Lys Asn Leu Lys Lys Asn
125 130 135
Tyr Cys Arg Asn Pro Asp Gly Glu Pro Arg Pro Trp Cys Phe Thr
140 145 150
Thr Asp Pro Asn Lys Arg Trp Glu Leu Cys Asp Ile Pro Arg Cys
155 160 165
Thr Thr Pro Pro Pro Ser Ser Gly Pro Thr Tyr Gln Cys Leu Lys
170 175 180
Gly Thr Gly Glu Asn Tyr Arg Gly Asp Val Ala Val Thr Val Ser
185 190 195
Gly His Thr Cys His Gly Trp Ser Ala Gln Thr Pro His Thr His
200 205 210
Asn Arg Thr Pro Glu Asn Phe Pro Cys Lys Asn Leu Asp Glu Asn
215 220 225
Tyr Cys Arg Asn Pro Asp Gly Glu Lys Ala Pro Trp Cys Tyr Thr
230 235 240
Thr Asn Ser Gln Val Arg Trp Glu Tyr Cys Lys Ile Pro Ser Cys
245 250 255
Glu Ser Ser Pro Val Ser Thr Glu Pro Leu Asp Pro Thr Ala Pro
260 265 270
Pro Glu Leu Thr Pro Val Val Gln Glu Cys Tyr His Gly Asp Gly

```
                275                     280                     285
Gln Ser Tyr Arg Gly Thr Ser Ser Thr Thr Thr Thr Gly Lys Lys
                290                     295                     300
Cys Gln Ser Trp Ser Ser Met Thr Pro His Trp His Glu Lys Thr
                305                     310                     315
Pro Glu Asn Phe Pro Asn Ala Gly Leu Thr Met Asn Tyr Cys Arg
                320                     325                     330
Asn Pro Asp Ala Asp Lys Gly Pro Trp
                335
```

(6) INFORMATION FOR SEQ ID NO:5:

(i) SEQUENCE CHARACTERISTICS:

(A) LENGTH: 339
(B) TYPE: peptide
(D) TOPOLOGY: linear

(xi) SEQUENCE DESCRIPTION: SEQ ID NO:5:

Ile Tyr Leu Ser Glu Cys Lys Thr Gly Asn Gly Lys Asn Tyr Arg
1 5 10 15
Gly Thr Thr Ser Lys Thr Lys Ser Gly Val Ile Cys Gln Lys Trp
20 25 30
Ser Val Ser Ser Pro His Ile Pro Lys Tyr Ser Pro Glu Lys Phe
35 40 45
Pro Leu Ala Gly Leu Glu Glu Asn Tyr Cys Arg Asn Pro Asp Asn
50 55 60
Asp Glu Lys Gly Pro Trp Cys Tyr Thr Thr Asp Pro Glu Thr Arg
65 70 75
Phe Asp Tyr Cys Asp Ile Pro Glu Cys Glu Asp Glu Cys Met His
80 85 90
Cys Ser Gly Glu His Tyr Glu Gly Lys Ile Ser Lys Thr Met Ser
95 100 105
Gly Ile Glu Cys Gln Ser Trp Gly Ser Gln Ser Pro His Ala His
110 115 120
Gly Tyr Leu Pro Ser Lys Phe Pro Asn Lys Asn Leu Lys Met Asn
125 130 135
Tyr Cys Arg Asn Pro Asp Gly Glu Pro Arg Pro Trp Cys Phe Thr
140 145 150
Thr Asp Pro Asn Lys Arg Trp Glu Phe Cys Asp Ile Pro Arg Cys
155 160 165
Thr Thr Pro Pro Pro Thr Ser Gly Pro Thr Tyr Gln Cys Leu Lys

170 175 180

Gly Arg Gly Glu Asn Tyr Arg Gly Thr Val Ser Val Thr Ala Ser

185 190 195

Gly His Thr Cys Gln Arg Trp Ser Ala Gln Ser Pro His Lys His

200 205 210

Asn Arg Thr Pro Glu Asn Phe Pro Cys Lys Asn Leu Glu Glu Asn

215 220 225

Tyr Cys Arg Asn Pro Asp Gly Glu Thr Ala Pro Trp Cys Tyr Thr

230 235 240

Thr Asp Ser Glu Val Arg Trp Asp Tyr Cys Lys Ile Pro Ser Cys

245 250 255

Gly Ser Ser Thr Thr Ser Thr Glu His Leu Asp Ala Pro Val Pro

260 265 270

Pro Glu Gln Thr Pro Val Ala Gln Asp Cys Tyr Arg Gly Asn Gly

275 280 285

Glu Ser Tyr Arg Gly Thr Ser Ser Thr Thr Ile Thr Gly Arg Lys

290 295 300

Cys Gln Ser Trp Val Ser Met Thr Pro His Arg His Glu Lys Thr

305 310 315

Pro Gly Asn Phe Pro Asn Ala Gly Leu Thr Met Asn Tyr Cys Arg

320 325 330

Asn Pro Asp Ala Asp Lys Ser Pro Trp

335

(7) INFORMATION FOR SEQ ID NO:6:

(i) SEQUENCE CHARACTERISTICS:

(A) LENGTH: 339
(B) TYPE: peptide
(D) TOPOLOGY: linear

(xi) SEQUENCE DESCRIPTION: SEQ ID NO:6:

Ile Tyr Leu Leu Glu Cys Lys Thr Gly Asn Gly Gln Thr Tyr Arg
1 5 10 15
Gly Thr Thr Ala Glu Thr Lys Ser Gly Val Thr Cys Gln Lys Trp
20 25 30
Ser Ala Thr Ser Pro His Val Pro Lys Phe Ser Pro Glu Lys Phe
35 40 45
Pro Leu Ala Gly Leu Glu Glu Asn Tyr Cys Arg Asn Pro Asp Asn
50 55 60
Asp Glu Asn Gly Pro Trp Cys Tyr Thr Thr Asp Pro Asp Lys Arg

65 70 75

Tyr Asp Tyr Cys Asp Ile Pro Glu Cys Glu Asp Lys Cys Met His

80 85 90

Cys Ser Gly Glu Asn Tyr Glu Gly Lys Ile Ala Lys Thr Met Ser

95 100 105

Gly Arg Asp Cys Gln Ala Trp Asp Ser Gln Ser Pro His Ala His

110 115 120

Gly Tyr Ile Pro Ser Lys Phe Pro Asn Lys Asn Leu Lys Met Asn

125 130 135

Tyr Cys Arg Asn Pro Asp Gly Glu Pro Arg Pro Trp Cys Phe Thr

140 145 150

Thr Asp Pro Gln Lys Arg Trp Glu Phe Cys Asp Ile Pro Arg Cys

155 160 165

Thr Thr Pro Pro Pro Ser Ser Gly Pro Lys Tyr Gln Cys Leu Lys

170 175 180

Gly Thr Gly Lys Asn Tyr Gly Gly Thr Val Ala Val Thr Glu Ser

185 190 195

Gly His Thr Cys Gln Arg Trp Ser Glu Gln Thr Pro His Lys His

200 205 210

Asn Arg Thr Pro Glu Asn Phe Pro Cys Lys Asn Leu Glu Glu Asn

215 220 225

Tyr Cys Arg Asn Pro Asp Gly Glu Lys Ala Pro Trp Cys Tyr Thr

230 235 240

Thr Asn Ser Glu Val Arg Trp Glu Tyr Cys Thr Ile Pro Ser Cys

245 250 255

Glu Ser Ser Pro Leu Ser Thr Glu Arg Met Asp Val Pro Val Pro

260 265 270

Pro Glu Gln Thr Pro Val Pro Gln Asp Cys Tyr His Gly Asn Gly

275 280 285

Gln Ser Tyr Arg Gly Thr Ser Ser Thr Thr Ile Thr Gly Arg Lys

290 295 300

Cys Gln Ser Trp Ser Ser Met Thr Pro His Arg His Leu Lys Thr

305 310 315

Pro Glu Asn Tyr Pro Asn Ala Gly Leu Thr Met Asn Tyr Cys Arg

320 325 330

Asn Pro Asp Ala Asp Lys Ser Pro Trp

335